# EUROPÄISCHE PATENTSCHRIFT

(11) **EP 1 532 150 B1**
(45) Veröffentlichungstag und Bekanntmachung des Hinweises auf die Patenterteilung: **11.06.2008**
(21) Anmeldenummer: 03792343.0
(22) Anmeldetag: 16.08.2003
(51) Int. Cl.: C07D 473/30, C07D 473/38, A61K 31/52, A61K 31/522, A61P 3/10, A61P 29/00, A61P 19/10

(54) **NEUE PURINDERIVATE, DEREN HERSTELLLUNG UND DEREN VERWENDUNG ALS ARZNEIMITTEL**
NOVEL PURINE DERIVATIVES, PRODUCTION AND USE THEREOF AS MEDICAMENTS
NOUVEAUX DERIVES DE PURINE, LEUR PROCEDE DE PRODUCTION ET LEUR UTILISATION EN TANT QUE MEDICAMENT

(30) Priorität: 22.08.2002 DE 10238477
(43) Veröffentlichungstag der Anmeldung: 25.05.2005
(73) Patentinhaber: Boehringer Ingelheim Pharma GmbH & Co. KG, 55216 Ingelheim am Rhein (DE)
(72) Erfinder: HIMMELSBACH, Frank, 88441 Mittelbiberach (DE); ECKHARDT, Matthias, 88400 Biberach an der Riss (DE); LANGKOPF, Elke, 88447 Warthausen (DE); LOTZ, Ralf, R., H., 88433 Schemmerhofen (DE); MAIER, Roland, 88400 Biberach an der Riss (DE); MARK, Michael, 88400 Biberach an der Riss (DE)
(86) Internationale Anmeldenummer: PCT/EP2003/009100
(87) Internationale Veröffentlichungsnummer: WO 2004/018469

(56) Entgegenhaltungen:
- EP-A- 1 054 012
- US-A- 5 041 448
- AUGUSTYNS K ET AL: "THE UNIQUE PROPERTIES OF DIPEPTIDYL-PEPTIDASE IV (DPP IV/CD26) AND THE THERAPEUTIC POTENTIAL OF DPP IV INHIBITORS" CURRENT MEDICINAL CHEMISTRY, BENTHAM SCIENCE PUBLISHERS BV, BE, Bd. 6, Nr. 4, 1999, Seiten 311-327, XP000870290 ISSN: 0929-8673

## Beschreibung

Gegenstand der vorliegenden Erfindung sind substituierte Purine der allgemeinen Formel 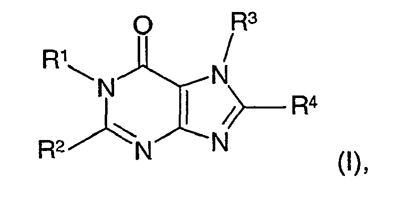 deren Tautomere, deren Stereoisomere, deren Gemische, deren Prodrugs und deren Salze, insbesonders deren physiologisch verträgliche Salze mit anorganischen oder organischen Säuren oder Basen, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

In der obigen Formel I bedeuten
R¹ ein Wasserstoffatom,
eine C₁₋₈-Alkylgruppe,
eine C₃₋₈-Alkenylgruppe,
eine C₃₋₄-Alkenylgruppe, die durch eine C₁₋₂-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl- oder Morpholin-4-ylcarbonyl-gruppe substituiert ist,
eine C₃₋₈-Alkinylgruppe,
eine durch eine Gruppe Rₐ substituierte C₁₋₆-Alkylgruppe, wobei
Rₐ eine C₃₋₇-Cycloalkyl-, Heteroaryl-, Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonylgruppe bedeutet,
eine durch eine Phenylgruppe substituierte C₁₋₆-Alkylgruppe, wobei der Phenylring durch die Gruppen R¹⁰ bis R¹⁴ substituiert ist und
R¹⁰ ein Wasserstoffatom,
ein Fluor-, Chlor-, Brom- oder lodatom,
eine C₁₋₄-Alkyl-, Hydroxy- oder C₁₋₄-Alkyloxygruppe,
eine Nitro-, Amino-, C₁₋₃-Alkylamino-, Di-(C₁₋₃-alkyl)-amino-, Cyan-C₁₋₃-alkylamino-, *N*-(Cyan-C₁₋₃-alkyl)-*N*-(C₁₋₃-alkyl)-amino-, C₁₋₃-Alkyloxy-carbonyl-C₁₋₃-alkylamino-, Pyrrolidin-1-yl-, Piperidin-1-yl-, Morpholin-4-yl-, Piperazin-1-yl- oder 4-(C₁₋₃-Alkyl)-piperazin-1-yl-Gruppe,
eine Formylamino-, C₁₋₃-Alkyl-carbonylamino-, C₃₋₆-Cycloalkyl-carbonylamino-, C₃₋₆-Cycloalkyl-C₁₋₃-alkyl-carbonylamino-, -Arylcarbonylamino-, Aryl-C₁₋₃-alkyl-carbonylamino-, C₁₋₃-Alkyloxy-carbonylamino-, Aminocarbonylamino-, C₁₋₃-Alkylaminocarbonylamino-, Di-(C₁₋₃-alkyl)-aminocarbonylamino-, Pyrrolidin-1-yl-carbonylamino-, Piperidin-1-yl-carbonylamino-, Morpholin-4-yl-carbonylamino-, Piperazin-1-yl-carbonylamino- oder 4-(C₁₋₃-Alkyl)-piperazin-1-yl-carbonylamino-, C₁₋₃-Alkyl-sulfonylamino-, Bis-(C₁₋₃-alkylsulfonyl)-amino-, Aminosulfonylamino-, C₁₋₃-Alkylamino-sulfonylamino-, Di-(C₁₋₃-alkyl)-amino-sulfonylamino-, Pyrrolidin-1-yl-sulfonylamino-, Piperidin-1-yl-sulfonylamino-, Morpholin-4-yl-sulfonylamino-, Piperazin-1-yl-sulfonylamino- oder 4-(C₁₋₃-Alkyl)-piperazin-1-yl-sulfonylamino-, (C₁₋₃-Alkylamino)-thiocarbonylamino-, (C₁₋₃-Alkyloxy-carbonylamino)-carbonylamino-, Arylsulfonylamino- oder Aryl-C₁₋₃-alkyl-sulfonylaminogruppe,
eine *N*-(C₁₋₃-Alkyl)-formylamino-, *N*-(C₁₋₃-Alkyl)-*N*-(C₁₋₆-alkyl-carbonyl)-amino-, *N-*(C₁₋₃-Alkyl)-*N*-(C₃₋₆-cycloalkyl-carbonyl)-amino-, *N*-(C₁₋₃-Alkyl)-*N*-(C₃₋₆-cycloalkyl-C₁₋₃-alkyl-carbonyl)-amino-, *N*-(C₁₋₃-Alkyl)-*N*-(arylcarbonyl)-amino-, *N*-(C₁₋₃-Alkyl)-*N*-(aryl-C₁₋₃-alkyl-carbonyl)-amino-; *N*-(C₁₋₃-Alkyl)-*N*-(C₁₋₃-alkyloxycarbonyl)-amino-, *N*-(Aminocarbonyl)-*N*-(C₁₋₃-alkyl)-amino-, *N*-(C₁₋₃-Alkyl-aminocarbonyl)-*N*-(C₁₋₃-alkyl)-amino-, *N*-[Di-(C₁₋₃-alkyl)aminocarbonyl]-*N*-(C₁₋₃-alkyl)-amino-, *N*-(C₁₋₃-Alkyl)-*N*-(C₁₋₃-alkyl-sulfonyl)-amino-, *N*-(C₁₋₃-Alkyl)-*N*-(arylsulfonyl)-amino- oder *N*-(C₁₋₃-Alkyl)-*N*-(aryl-C₁₋₃-alkyl-sulfonyl)-aminogruppe,
eine 2-Oxo-imidazolidin-1-yl-, 2,4-Dioxo-imidazofidin-1-yl-, 2,5-Dioxo-imidazolidin-1-yl- oder 2-Oxo-hexahydropyrimidin-1-ylgruppe, in der das Stickstoffatom in 3-Stellung jeweils durch eine Methyl- oder Ethylgruppe substituiert sein kann,
eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-, Piperazin-1-yl-carbonyl- oder 4-(C₁₋₃-Alkyl)-piperazin-1-yl-carbonylgruppe,
eine C₁₋₃-Alkyl-carbonyl- oder eine Arylcarbonylgruppe,
eine Carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-, Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1-yl-carbonyl-C₁₋₃-alkyl- oder 4-(C₁₋₃-Alkyl)-piperazin-1-yl-carbonyl-C₁₋₃-alkylgruppe,
eine Carboxy-C₁₋₃-alkyloxy-, C₁₋₃-Alkyloxy-carbonyl-C₁₋₃-alkyloxy-, Cyan-C₁₋₃-alkyloxy-, Aminocarbonyl-C₁₋₃-alkyloxy-, C₁₋₃-Alkyl-aminocarbonyl-C₁₋₃-alkyloxy-, Di-(C₁₋₃-alkyl)-aminocarbonyl-C₁₋₃-alkyloxy-, Pyrrolidin- i -yl-carbonyl-C₁₋₃-alkyloxy-, Piperidin-1-yl-carbonyl-C₁₋₃-alkyloxy-, Morpholin-4-yl-carbonyl-C₁₋₃-alkyloxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
eine Hydroxy-C₁₋₃-alkyl-, C₁₋₃-Alkyl-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1-yl-C₁₋₃-alkyl-, 4-(C₁₋₃-Alkyl)-piperazin-1-yl-C₁₋₃-alkylgruppe,
eine Hydroxy-C₁₋₃-alkyloxy-, C₁₋₃-Alkyloxy-C₁₋₃-alkyloxy-, C₁₋₃-Alkylsulfanyl-C₁₋₃-alkyloxy-, C₁₋₃-Alkylsulfinyl-C₁₋₃-alkyloxy-, C₁₋₃-Alkylsulfonyl-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1-yl-C₁₋₃-alkyloxy-, 4-(C₁₋₃-Alkyl)-piperazin-1-yl-C₁₋₃-alkyloxygruppe,
eine Mercapto-, C₁₋₃-Alkylsulfanyl-, C₁₋₃-Alkysulfinyl-, C₁₋₃-Alkylsulfonyl-, C₁₋₃-Alkylsulfonyloxy-, Arylsulfonyloxy-, Trifluormethylsulfanyl-, Trifluormethylsulfinyl- oder Trifluormethylsulfonylgruppe,
eine Sulfo-, Aminosulfonyl-, C₁₋₃-Alkyl-aminosulfonyl-, Di-(C₁₋₃-Alkyl)-aminosulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1-yl-sulfonylgruppe,
eine durch 1 bis 3 Fluoratome substituierte Methyl- oder Methoxygruppe,
eine durch 1 bis 5 Fluoratome substituierte Ethyl- oder Ethyloxygruppe,
eine C₂₋₄-Alkenyl- oder C₂₋₄-Alkinylgruppe,
eine C₃₋₄-Alkenyloxy- oder C₃₋₄-Alkinyloxygruppe,
eine C₃₋₆-Cycloalkyl- oder C₃₋₆-Cycloalkyloxygruppe,
eine C₃₋₆-Cycloalkyl-C₁₋₃-alkyl- oder C₃₋₆-Cycloalkyl-C₁₋₃-alkyloxygruppe oder
eine Aryl-, Aryloxy-, Aryl-C₁₋₃-alkyl- oder Aryl-C₁₋₃-alkyloxygruppe,
R¹¹ und R¹², die gleich oder verschieden sein können, jeweils ein Wasserstoffatom, ein Fluor-, Chlor-, Brom- oder lodatom, eine C₁₋₃-Alkyl-, Trifluormethyl-, Hydroxy-, C₁₋₃-Alkyloxy- oder Cyangruppe, oder
R¹¹ zusammen mit R¹², sofern diese an benachbarte Kohlenstoffatome gebunden sind, auch eine Methylendioxy-, Difluormethylendioxy- oder eine geradkettige C₃₋₅-Alkylengruppe und
R¹³ und R¹⁴, die gleich oder verschieden sein können, jeweils ein Wasserstoffatom, ein Fluor-, Chlor- oder Bromatom, eine Trifluormethyl-, C₁₋₃-Alkyl- oder C₁₋₃-Alkyloxygruppe bedeuten,
eine Phenyl-C₁₋₄-alkylgruppe, in der der Alkylteil durch eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-Gruppe substituiert ist und der Phenylteil durch die Gruppen R¹⁰ bis R¹⁴ substituiert ist, wobei R¹⁰ bis R¹⁴ wie vorstehend erwähnt definiert sind;
eine durch die Gruppen R¹⁰ bis R¹⁴ substituierte Phenylgruppe, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyl-(CH₂)ₘ-A-(CH₂)ₙ-Gruppe, in der der Phenylteil durch R¹⁰ bis R¹⁴ substituiert ist, wobei R¹⁰ bis R¹⁴ wie vorstehend erwähnt definiert sind und
A eine Carbonylgruppe, m die Zahl 0, 1 oder 2 und n die Zahl 1, 2 oder 3 bedeuten,
eine Phenylcarbonylmethylgruppe, in der der Phenylteil durch R¹⁰ bis R¹⁴ substituiert ist, wobei R¹⁰ bis R¹⁴ wie vorstehend erwähnt definiert sind und der Methylteil durch eine C₁₋₃-Alkylgruppe substituiert ist,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, m und n wie vorstehend erwähnt definiert sind und
B eine Methylengruppe, die durch eine Hydroxy-, C₁₋₃-Alkyloxy-, Amino-, C₁₋₃-Alkylamino-, Di-(C₁₋₃-alkyl)-amino-, Mercapto-, C₁₋₃-Alkylsulfanyl-, C₁₋₃-Alkylsulfinyl- oder C₁₋₃-Alkylsulfonylgruppe substituiert ist und gegebenenfalls zusätzlich durch eine Methyl- oder Ethylgruppe substituiert ist, bedeutet,
eine Naphthyl-C₁₋₃-alkylgruppe, in der der Naphthylteil durch die Gruppen R¹⁰ bis R¹⁴ substituiert ist, wobei R¹⁰ bis R¹⁴ wie vorstehend erwähnt definiert sind,
eine Naphthyl-(CH₂)ₘ-A-(CH₂)ₙ-Gruppe, in der der Naphthylteil durch R¹⁰ bis R¹⁴ substituiert ist, wobei R¹⁰ bis R¹⁴, A, m und n wie vorstehend erwähnt definiert sind,
eine Naphthyl-(CH₂)ₘ-B-(CH₂)ₙ-Gruppe, in der der Naphthylteil durch R¹⁰ bis R¹⁴ substituiert ist, wobei R¹⁰ bis R¹⁴, B, in und n wie vorstehend erwähnt definiert sind, eine [1,4]Naphthochinon-2-yl-, Chromen-4-on-3-yl-, 1-Oxoindan-2-yl-, 1,3-Dioxoindan-2-yl- oder 2,3-Dihydro-3-oxo-benzofuran-2-ylgruppe
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₃-Alkyloxycarbonyl-, Aminocarbonyl-, C₁₋₃-Alkylaminocarbonyl-, Di-(C₁₋₃-alkyl)aminocarbonyl-, Pyrrolidin-1-yl-carbonyl-, Piperidin-1-yl-carbonyl- oder Morpholin-4-yl-carbonyl-, Piperazin-1-yl-carbonyl-, 4-Methylpiperazin-1-yl-carbonyl- oder 4-Ethylpiperazin-1-yl-carbonyl-Gruppe bedeutet und A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴ und m wie vorstehend erwähnt sind und D eine Sauerstoff- oder Schwefelatom, eine Imino-, C₁₋₃-Alkylimino-, Sulfinyl- oder Sulfonylgruppe bedeutet,
eine Naphthyl-(CH₂)ₘ-D-C₁₋₃-alkylgruppe, in der der Naphthylteil durch die Gruppen R¹⁰ bis R¹⁴ substituiert ist, wobei R¹⁰ bis R¹⁴, D und m wie vorstehend erwähnt sind, eine durch eine Gruppe R_{b} substituierte C₂₋₆-Alkylgruppe, wobei
R_{b} durch mindestens zwei Kohlenstoffatome vom Ringstickstoffatom in 1-Stellung des Puringerüstes isoliert ist und
R_{b} eine Hydroxy-, C₁₋₃-Alkyloxy-, Mercapto-, C₁₋₃-Alkylsulfanyl-, C₁₋₃-Alkylsulfinyl, C₁₋₃-Alkylsulfonyl-, Amino-, C₁₋₃-Alkyl-carbonylamino-, C₃₋₆-Cycloalkyl-carbonylamino-, Arylcarbonylamino-, C₁₋₃-Alkylamino-, Di-(C₁₋₃-alkyl)-amino-, Pyrrolidin-1-yl-, Piperidin-1-yl-, Morpholin-4-yl-, Piperazin-1-yl- oder 4-(C₁₋₃-Alkyl)-piperazin-1-ylgruppe bedeutet,
eine C₃₋₆-Cycloalkylgruppe,
oder eine Amino- oder Arylcarbonylaminogruppe,
R² ein Wasserstoffatom,
eine C₁₋₈-Alkylgruppe,
eine C₃₋₈-Alkenylgruppe,
eine C₃₋₄-Alkenylgruppe, die durch eine C₁₋₂-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl- oder Morpholin-4-ylcarbonyl-gruppe substituiert ist,
eine C₃₋₈-Alkinylgruppe,
eine C₃₋₆-Cycloalkyl-Gruppe,
eine durch eine Gruppe Rₐ substituierte C₁₋₆-Alkylgruppe, wobei Rₐ wie vorstehend erwähnt definiert ist,
eine Phenylgruppe, die durch R¹⁰ bis R¹⁴ substituiert ist, wobei R¹⁰ bis R¹⁴ wie vorstehend erwähnt definiert sind,
eine durch eine Phenylgruppe substituierte C₁₋₆-Alkylgruppe, wobei der Phenylring durch die Gruppen R¹⁰ bis R¹⁴ substituiert ist und R¹⁰ bis R¹⁴ wie vorstehend erwähnt definiert sind,
eine Phenyl-C₁₋₄-alkylgruppe, in der der Alkylteil durch eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-Gruppe substituiert ist und der Phenylteil durch die Gruppen R¹⁰ bis R¹⁴ substituiert ist, wobei R¹⁰ bis R¹⁴ wie vorstehend erwähnt definiert sind,
eine Phenyl-C₂₋₃-alkenyl-Gruppe, in der der Phenylteil durch R¹⁰ bis R¹⁴ substituiert ist, wobei R¹⁰ bis R¹⁴ wie vorstehend erwähnt definiert sind,
eine Heteroaryl-Gruppe,
eine Phenyl-(CH₂)ₘ-A- oder Phenyl-(CH₂)ₘ-A-(CH₂)ₙ-Gruppe, in der der Phenylteil jeweils durch R¹⁰ bis R¹⁴ substituiert ist, wobei A, R¹⁰ bis R¹⁴, m und n wie vorstehend erwähnt definiert sind,
eine Phenylcarbonylmethylgruppe, in der der Phenylteil durch R¹⁰ bis R¹⁴ substituiert ist, wobei R¹⁰ bis R¹⁴ wie vorstehend erwähnt definiert sind und der Methylteil durch eine C₁₋₃-Alkylgruppe substituiert ist,
eine Phenyl-(CH₂)ₘ-B- oder Phenyl-(CH₂)ₘ-B-(CH₂)ₙ-Gruppe, in der der Phenylteil jeweils durch R¹⁰ bis R¹⁴ substituiert ist, wobei B, R¹⁰ bis R¹⁴, m und n wie vorstehend erwähnt definiert sind,
eine Naphthyl-C₁₋₃-alkylgruppe, in der der Naphthylteil durch die Gruppen R¹⁰ bis R¹⁴ substituiert ist, wobei R¹⁰ bis R¹⁴ wie vorstehend erwähnt definiert sind,
eine Naphthyl-(CH₂)ₘ-A- oder Naphthyl-(CH₂)ₘ-A-(CH₂)ₙ-Gruppe, in der der Naphthylteil jeweils durch R¹⁰ bis R¹⁴ substituiert ist, wobei R¹⁰ bis R¹⁴, A, m und n wie vorstehend erwähnt definiert sind,
eine Naphthyl-(CH₂)ₘ-B- oder Naphthyl-(CH₂)ₘ-B-(CH₂)ₙ-Gruppe, in der der Naphthylteil jeweils durch R¹⁰ bis R¹⁴ substituiert ist, wobei R¹⁰ bis R¹⁴, B, m und n wie vorstehend erwähnt definiert sind,
eine Heteroaryl-(CH₂)ₘ-A- oder Heteroaryl-(CH₂)ₘ-A-(CH₂)ₙ-Gruppe, wobei A, m und n wie vorstehend erwähnt definiert sind,
eine Heteroaryl-(CH₂)ₘ-B- oder Heteroaryl-(CH₂)ₘ-B-(CH₂)ₙ-Gruppe, wobei B, m und n wie vorstehend erwähnt definiert sind,
eine C₁₋₆-Alkyl-A- oder C₁₋₆-Alkyl-A-(CH₂)ₙ-Gruppe, wobei A und n wie vorstehend erwähnt definiert sind,
eine C₃₋₇-Cycloalkyl-(CH₂)ₘ-A- oder C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B- oder C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹, A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴, D und m wie vorstehend erwähnt sind,
eine Naphthyl-(CH₂),-D-C₁₋₃-alkylgruppe, in der der Naphthylteil durch die Gruppen R¹⁰ bis R¹⁴ substituiert ist, wobei R¹⁰ bis R¹⁴, D und m wie vorstehend erwähnt sind,
eine durch eine Gruppe R_{b} substituierte C₁₋₆-Alkylgruppe, wobei R_{b} wie vorstehend erwähnt definiert ist,
eine Cyano-, Carboxy-, C₁₋₃-Alkyloxy-carbonyl-, Aminocarbonyl-, C₁₋₃-Alkylaminocarbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonyl-Gruppe,
eine Amino-, C₁₋₆-Alkylamino- oder Di-(C₁₋₆-alkyl)-aminogruppe,
eine durch die Reste R¹⁵ und R¹⁶ substituierte Aminogruppe, in der
R¹⁵ ein Wasserstoffatom oder eine C₁₋₆-Alkylgruppe und
R¹⁶ eine C₁₋₆-Alkylgruppe, die durch Rₐ substituiert ist, bedeuten, wobei Rₐ wie vorstehend erwähnt definiert ist,
eine durch die Reste R¹⁵ und R¹⁷ substituierte Aminogruppe, in der
R¹⁵ wie vorstehend erwähnt definiert ist und
R¹⁷ eine C₂₋₆-Alkylgruppe, die durch eine Hydroxy-, C₁₋₃-Alkyloxy-, Aryloxy-, Mercapto-, C₁₋₃-Alkylsulfanyl-, C₁₋₃-Alkylsulfinyl-, C₁₋₃-Alkylsulfonyl-, C₁₋₃-Alkylsulfonylamino-, Arylsulfanyl-, Arylsulfinyl-, Arylsulfonyl-, Arylsulfonylamino-, C₁₋₃-Alkyl-carbonylamino-, C₃₋₆-Cycloalkyl-carbonylamino-, Arylcarbonylamino-, C₁₋₃-Alkyl-oxycarbonylamino-, Aminocarbonylamino-, C₁₋₃-Alkylaminocarbonylamino-, Di-(C₁₋₃-alkyl)-aminocarbonylamino-, Amino-, C₁₋₃-Alkylamino-, Di-(C₁₋₃-alkyl)-amino-, Pyrrolidin-1-yl-, Piperidin-1-yl-, Morpholin-4-yl-, Piperazin-1-yl- oder 4-(C₁₋₃-Alkyl)-piperazin-1-yl-Gruppe substituiert ist, bedeuten,
eine C₃₋₆-Cycloalkylamino- oder *N*-(C₃₋₆-Cycloalkyl)-*N*-(C₁₋₃-alkyl)-amino-Gruppe,
eine Phenylamino- oder *N*-(Phenyl)-*N*-(C₁₋₃-alkyl)-amino-Gruppe, in der der Phenylteil jeweils durch R¹⁰ bis R¹⁴ substituiert ist, wobei R¹⁰ bis R¹⁴ wie vorstehend erwähnt definiert sind,
eine Phenyl-C₁₋₆-alkylamino- oder *N*-(Phenyl-C₁₋₆-alkyl)-N-(C₁₋₃-alkyl)-amino-Gruppe, in der der Phenylteil jeweils durch R¹⁰ bis R¹⁴ substituiert ist, wobei R¹⁰ bis R¹⁴ wie vorstehend erwähnt definiert sind,
eine Naphthylamino- oder *N*-(Naphthyl)-*N*-(C₁₋₃-alkyl)-amino-Gruppe,
eine Naphthyl-C₁₋₆-alkylamino- oder *N*-(Naphthyl-C₁₋₆-alkyl)-N-(C₁₋₃-alkyl)-amino-Gruppe,
eine Heteroarylamino- oder *N*-(Heteroaryl)-*N*-(C₁₋₃-alkyl)-amino-Gruppe,
eine Pyrrolidin-1-yl-, Piperidin-1-yl-, Homopiperidin-1-yl-, Morpholin-4-yl-, Homomorpholin-4-yl-, Piperazin-1-yl-, 4-(C₁₋₃-Alkyl)-piperazin-1-yl-, Homopiperazin-1-yl- oder 4-(C₁₋₃-Alkyl)-homopiperazin-1-yl-Gruppe, oder
eine C₁₋₆-Al kyloxy-, C₃₋₆-Cycloalkyloxy- oder C₃₋₆-Cycloalkyl-C₁₋₆-alkyloxy-Gruppe,
eine C₁₋₆-Alkylsulfanyl-, C₃₋₆-Cycloalkylsulfanyl- oder C₃₋₆-Cycloalkyl-C₁₋₆-alkylsulfanyl-Gruppe,
eine Phenyloxy- oder Phenylsulfanylgruppe, in der der Phenylteil jeweils durch R¹⁰ bis R¹⁴ substituiert ist, wobei R¹⁰ bis R¹⁴ wie vorstehend erwähnt definiert sind,
eine Phenyl-C₁₋₆-alkyloxy- oder Phenyl-C₁₋₆-alkylsulfanylgruppe, in der der Phenylteil jeweils durch R¹⁰ bis R¹⁴ substituiert ist, wobei R¹⁰ bis R¹⁴ wie vorstehend erwähnt definiert sind,
eine Naphthyloxy- oder eine Naphthylsulfanyl-Gruppe, in der der Naphthylteil jeweils durch R¹⁰ bis R¹⁴ substituiert ist, wobei R¹⁰ bis R¹⁴ wie vorstehend erwähnt definiert sind,
eine Naphthyl-C₁₋₆-alkyloxy- oder Naphthyl-C₁₋₆-alkylsulfanyl-Gruppe, in der der Naphthylteil jeweils durch R¹⁰ bis R¹⁴ substituiert ist, wobei R¹⁰ bis R¹⁴ wie vorstehend erwähnt definiert sind,
eine Heteroaryloxy- oder Heteroarylsulfanyl-Gruppe oder
eine Heteroaryl-C₁₋₆-alkyloxy- oder Heteroaryl-C₁₋₆-alkylsulfanyl-Gruppe,
R³ eine C₁₋₈-Alkylgruppe,
eine durch die Gruppe R_{c} substituierte C₁₋₄-Alkylgruppe, wobei
R_{c} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇-Cycloalkenylgruppe,
eine Arylgruppe oder
eine Furanyl-, Thienyl-, Oxazolyl-, Isoxazolyl-, Thiazolyl-, Isothiazolyl-, Pyridyl-, Pyridazinyl-, Pyrimidyl-oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder lodatom oder durch eine Trifluormethyl-, Cyan- oder C₁₋₃-Alkyloxygruppe substituiert sein können,
eine C₃₋₈-Alkenylgruppe,
eine durch ein Fluor-, Chlor- oder Bromatom oder eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine Amino-, C₁₋₃-Alkylamino- oder Di-(C₁₋₃-alkyl)-amino-Gruppe substituiert ist und zusätzlich durch eine oder zwei C₁₋₃-Alkylgruppen substituiert sein kann,
eine Piperidin-1-yl- oder Hexahydroazepin-1-ylgruppe, die in 3-Stellung oder in 4-Stellung durch eine Amino-, C₁₋₃-Alkylamino- oder Di-(C₁₋₃-alkyl)amino-Gruppe substituiert ist und zusätzlich durch eine oder zwei C₁₋₃-Alkylgruppen substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)-aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist;
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1 yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine gegebenenfalls am Kohlenstoffgerüst durch eine oder zwei C₁₋₃-Alkylgruppen substituierte Piperazin-1-yl- oder [1,4]Diazepan-1-ylgruppe,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine-oder zwei-C₁₋₃-Alkylgruppen substituierte [1,4]Diazepan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine *N*-(C₃₋₇-Cycloalkyl)-*N*-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine N-(C₃₋₇-Cycloalkyl)-N-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine *N*-(C₃₋₇-Cycloalkyl-C₁₋₂-alkyl)-*N*-(C₁₋₂-alkyl)-aminogruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine *N*-(C₃₋₇-Cycloalkyl-C₁₋₂-alkyl)-*N*-(C₁₋₂-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine R¹⁹-C₂₋₄-Alkylamino-Gruppe, in der R¹⁹ durch mindestens zwei Kohlenstoffatome vom Stickstoffatom des C₂₋₄-Alkylamino-Teils getrennt ist und
R¹⁹ eine Amino-, C₁₋₃-Alkylamino- oder Di-(C₁₋₃-alkyl)-aminogruppe darstellt,
eine R¹⁹-C₂₋₄-Alkylamino-Gruppe, in der das Stickstoffatom des C₂₋₄-Alkylamino-Teils durch eine C₁₋₃-Alkylgruppe substituiert ist und R¹⁹ durch mindestens zwei Kohlenstoffatome vom Stickstoffatom des C₂₋₄-Alkylamino-Teils getrennt ist, wobei R¹⁹ wie vorstehend erwähnt definiert ist,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃-Alkylgruppen substituiert sein können,
eine durch den Rest R²⁰ und eine C₁₋₃-Alkylgruppe substituierte Aminogruppe, in der R²⁰ wie vorstehend erwähnt definiert ist, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und zusätzlich durch eine oder zwei C₁₋₃-Alkylgruppen substituiert sein kann, wobei R¹⁹ wie vorstehend erwähnt definiert ist,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino- oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Berizofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimidinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1*H*-benzimidazolyl-, 2,3-Dihydro-2-oxo-benzoxazolyl-, 1,2-Dihydro-2-oxo-chinolinyl-, 1,4-Dihydro-4-oxochinolinyl-, 1,2-Dihydro-1-oxo-isochinolinyl-, 1,4-Dihydro-4-oxo-cinnolinyl-, 1,2-Dihydro-2-oxo-chinazolinyl-, 3,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxochinoxalinyl-, 1,2-Dihydro-1-oxo-phthalazinyl-, 1,2,3,4-Tetrahydro-1,4-dioxophthalazinyl-, Chromanyl-, Cumarinyl-, 2,3-Dihydro-benzo[1,4]dioxinyl- oder 3,4-Dihydro-3-oxo-2*H*-benzo[1,4]oxazinyl-Gruppe zu verstehen ist,
wobei die vorstehend erwähnten Heteroarylgruppen durch R¹⁰ bis R¹⁴ substituiert sein können, wobei R¹⁰ bis R¹⁴ wie vorstehend erwähnt definiert sind,
wobei, soweit nichts anderes erwähnt wurde, die vorstehend erwähnten Alkyl-, Alkenyl- und Alkinylgruppen geradkettig oder verzweigt sein können,
sowie die am Ringstickstoffatom in 3-Stellung oder in 9-Stellung des Hypoxanthingerüstes N-oxidierten oder methylierten oder ethylierten Derivate,
sowie die Derivate, in denen die 6-Oxogruppe des Hypoxanthingerüstes durch eine Thioxogruppe ersetzt ist,
mit der Maßgabe, daß die Verbindungen
8-(Piperidin-4-ylmethyl)-7-(4-fluorbenzyl)-1,7-dihydro-purin-6-on und
8-(1-Methyl-piperidin-4-ylmethyl)-7-(4-fluorbenzyl)-1,7-dihydro-purin-6-on
ausgeschlossen sind,
deren Tautomere, Enantiomere, Diastereomere, deren Gemische, deren Prodrugs und deren Salze.

Verbindungen, die eine in-vivo abspaltbare Gruppe enthalten, sind Prodrugs der entsprechenden Verbindungen, bei denen diese in-vivo abspaltbare Gruppe abgespalten ist.

Die bei der Definition der vorstehend erwähnten Reste erwähnten Carboxygruppen können durch eine in-vivo in eine Carboxygruppe überführbare Gruppe oder durch eine unter physiologischen Bedingungen negativ geladene Gruppe ersetzt sein,
desweiteren können die bei der Definition der vorstehend erwähnten Reste erwähnten Amino- und Iminogruppen durch einen in-vivo abspaltbaren Rest substituiert sein. Derartige Gruppen werden beispielsweise in der WO 98/46576 und von N.M. Nielsen et al. in International Journal of Pharmaceutics 39, 75-85 (1987) beschrieben.

Unter einer in-vivo in eine Carboxygruppe überführbare Gruppe ist beispielsweise eine Hydroxymethylgruppe, eine mit einem Alkohol veresterte Carboxygruppe, in der der alkoholische Teil vorzugsweise ein C₁₋₆-Alkanol, ein Phenyl-C₁₋₃-alkanol, ein C₃₋₉-Cycloalkanol, wobei ein C₅₋₈-Cycloalkanol zusätzlich durch ein oder zwei C₁₋₃-Alkylgruppen substituiert sein kann, ein C₅₋₈-Cycloalkanol, in dem eine Methylengruppe in 3- oder 4-Stellung durch ein Sauerstoffatom oder durch eine gegebenenfalls durch eine C₁₋₃-Alkyl-, Phenyl-C₁₋₃-alkyl-, Phenyl-C₁₋₃-alkyloxycarbonyl- oder C₂₋₆-Alkanoylgruppe substituierte Iminogruppe ersetzt ist und der Cycloalkanolteil zusätzlich durch ein oder zwei C₁₋₃-Alkylgruppen substituiert sein kann, ein C₄₋₇-Cycloalkenol, ein C₃₋₅-Alkenol, ein Phenyl-C₃₋₅-alkenol, ein C₃₋₅-Alkinol oder Phenyl-C₃₋₅-alkinol mit der Maßgabe, daß keine Bindung an das Sauerstoffatom von einem Kohlenstoffatom ausgeht, welches eine Doppel- oder Dreifachbindung trägt, ein C₃₋₈-Cycloalkyl-C₁₋₃-alkanol, ein Bicycloalkanol mit insgesamt 8 bis 10 Kohlenstoffatomen, das im Bicycloalkylteil zusätzlich durch eine oder zwei C₁₋₃-Alkylgruppen substituiert sein kann, ein 1,3-Dihydro-3-oxo-1-isobenzfuranol oder ein Alkohol der Formel

Rₚ-CO-O-(R_{q}CRᵣ)-OH,

in dem
Rₚ eine C₁₋₈-Alkyl-, C₅₋₇-Cycloalkyl-, C₁₋₈-Alkyloxy-, C₅₋₇-Cycloalkyloxy-, Phenyl- oder Phenyl- C₁₋₃-alkylgruppe,
R_{q} ein Wasserstoffatom, eine C₁₋₃-Alkyl-, C₅₋₇-Cycloalkyl- oder Phenylgruppe und
Rᵣ ein Wasserstoffatom oder eine C₁₋₃-Alkylgruppe darstellen,
unter einer unter physiologischen Bedingungen negativ geladenen Gruppe wie eine Tetrazol-5-yl-, Phenylcarbonylaminocarbonyl-, Trifluormethylcarbonylaminocarbonyl-, C₁₋₆-Alkylsulfonylamino-, Phenylsulfonylamino-, Benzylsulfonylamino-, Trifluormethylsulfonylamino-, C₁₋₆-Alkylsulfonylaminocarbonyl-, Phenylsulfonylaminocarbonyl-, Benzylsulfonylaminocarbonyl- oder Perfluor-C₁₋₆-alkylsulfonylaminocarbonylgruppe
und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Brom- oder Jodatome, durch C₁₋₃-Alkyl- oder C₁₋₃-Alkyloxygruppen mono- oder disubstituierte Phenylcarbonylgruppe, wobei die Substituenten gleich oder verschieden sein können, eine Pyridinoylgruppe oder eine C₁₋₁₆-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine C₁₋₁₆-Alkyloxycarbonyl- oder C₁₋₁₆-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert.-Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy- oder Hexadecylcarbonyloxygruppe, eine Phenyl-C₁₋₆-alkyloxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch C₁₋₆-Alkyl- oder C₃₋₇-Cycloalkylgruppen mono- oder disubstituiert und die Substituenten gleich oder verschieden sein können, eine C₁₋₃-Alkylsulfonyl-C₂₋₄-alkyloxycarbonyl-, C₁₋₃-Alkyloxy-C₂₋₄-alkyloxy-C₂₋₄-alkyloxycarbonyl-,Rₚ-CO-O-(R_{g}CRᵣ)-O-CO-, C₁₋₆-Alkyl-CO-NH-(RₛCRₜ)-O-CO- oder C₁₋₆-Alkyl-CO-O-(RₛCRₜ)-(RₛCRₜ)-O-CO-Gruppe, in denen Rₚ bis Rᵣ wie vorstehend erwähnt definiert sind,
Rₛ und Rₜ, die gleich oder verschieden sein können, Wasserstoffatome oder C₁₋₃-Alkylgruppen darstellen,
zu verstehen.

Desweiteren schließen die in den vor- und nachstehenden Definitionen erwähnten gesättigten Alkyl- und Alkyloxyteile, die mehr als 2 Kohlenstoffatome enthalten, soweit nichts Anderes erwähnt wurde, auch deren verzweigte Isomere wie beispielsweise die Isopropyl-, tert.Butyl-, Isobutylgruppe etc. ein.

Eine besonders zu erwähnende Untergruppe betrifft diejenigen Verbindungen der allgemeinen Formel I, in denen R¹, R² und R³ wie vorstehend erwähnt definiert sind und
R⁴ eine Pyrrolidin-1-ylgruppe, die in 3-Stellung durch eine Aminogruppe substituiert ist,
eine Piperidin-1-ylgruppe, die in 3-Stellung durch eine Aminogruppe substituiert ist,
eine Piperidin-3-yl- oder Piperidin-4-ylgruppe,
eine Hexahydroazepin-1-yl-gruppe, die in 3-Stellung oder in 4-Stellung durch eine Aminogruppe substituiert ist,
eine Piperazin-1-yl- oder [1,4]Diazepan-1-ylgruppe,
eine (2-Aminocyclohexyl)amino-Gruppe,
eine Cyclohexylgruppe, die in 3-Stellung durch eine Aminogruppe substituiert ist,
oder eine N-(2-Aminoethyl)-methylamino- oder eine N-(2-Aminoethyl)-ethylamino-Gruppe bedeutet,
deren Tautomere, Enantiomere, Diastereomere, deren Gemische, deren Prodrugs und deren Salze.

Bevorzugte Verbindungen der obigen allgemeinen Formel I sind diejenigen, in denen
R¹ ein Wasserstoffatom,
eine C₁₋₆-Alkylgruppe,
eine C₃₋₆-Alkenylgruppe,
eine C₃₋₄-Alkinylgruppe,
eine C₃₋₆-Cycloalkylmethyl-Gruppe,
eine Phenyl-C₁₋₃-alkyl-Gruppe, in der der Phenylteil durch R¹⁰ und R¹¹ substituiert ist,
wobei
R¹⁰ ein Wasserstoffatom, ein Fluor- Chlor-oder Bromatom,
eine Methyl- oder Trifluormethyl-Gruppe,
eine Cyan-, Aminocarbonyl-, Dimethylaminocarbonyl- oder Methylsulfonyl-Gruppe,
eine Amino-, Acetylamino- oder Methylsulfonylamino-Gruppe,
eine Hydroxy-, Methoxy-, Difluormethoxy-, Trifluormethoxy-, Carboxymethoxy-, Methoxycarbonylmethoxy-, Ethyloxycarbonylmethoxy-, Aminocarbonylmethoxy-, Methylaminocarbonylmethoxy-, Ethylaminocarbonylmethoxy- oder Dimethylaminocarbonylmethoxy-Gruppe und
R¹¹ ein Wasserstoffatom, ein Fluor- oder Chloratom,
oder eine Methyl- oder Methoxy-Gruppe bedeuten,
eine Naphthylmethylgruppe, in der der Naphthylteil durch R¹⁰ und R¹¹ substituiert ist, wobei R¹⁰ und R¹¹ wie vorstehend erwähnt definiert sind,
eine Heteroarylmethyl-Gruppe, wobei unter dem Begriff
Heteroaryl eine Furanyl-, Thienyl-, Oxazolyl-, Isoxazolyl-, Thiazolyl-, Pyridyl-, Pyrimidinyl-, Pyrazinyl-, Chinolinyl-, Isochinolinyl- oder Chinazolinylgruppe zu verstehen ist und die vorstehend erwähnten Heteroarylgruppen durch R¹⁰ und R¹¹ substituiert sind, wobei R¹⁰ und R¹¹ wie vorstehend erwähnt definiert sind,
eine Phenylcarbonylmethylgruppe, in der der Phenylteil durch R¹⁰ und R¹¹ substituiert ist, wobei R¹⁰ und R¹¹ wie vorstehend erwähnt definiert sind,
eine Furanylcarbonylmethyl-, Thienylcarbonylmethyl- oder Pyridylcarbonylmethylgruppe,
oder eine 2-Oxo-propyl- oder Cyclohexylcarbonylmethylgruppe,
R² ein Wasserstoffatom,
eine C₁₋₆-Alkylgruppe,
eine C₃₋₆-Alkenylgruppe,
eine C₃₋₄-Alkinylgruppe,
eine C₃₋₆-Cycloalkyl- oder C₃₋₆-Cycloalkyl-C₁₋₃-alkyl-Gruppe,
eine Phenylgruppe, die durch H¹⁰ und R¹¹ substituiert ist, wobei R¹⁰ und R¹¹ wie vorstehend erwähnt definiert sind,
eine Phenyl-C₁₋₃-alkyl-Gruppe, in der der Phenylteil durch R¹⁰ und R¹¹ substituiert ist, wobei R¹⁰ und R¹¹ wie vorstehend erwähnt definiert sind,
eine Phenyl-C₂₋₃-alkenyl-Gruppe, in der der Phenylteil durch R¹⁰ und R¹¹ substituiert ist, wobei R¹⁰ und R¹¹ wie vorstehend erwähnt definiert sind,
eine Phenylcarbonylmethylgruppe, in der der Phenylteil durch R¹⁰ und R¹¹ substituiert ist, wobei R¹⁰ und R¹¹ wie vorstehend erwähnt definiert sind,
eine Furanyl-, Thienyl- oder Pyridylgruppe,
eine Furanyl-C₁₋₃-alkyl-, Thienyl-C₁₋₃-alkyl- oder Pyridyl-C₁₋₃-alkyl-Gruppe,
eine Cyanogruppe,
eine Amino-, C₁₋₄-Alkylamino- oder Di-(C₁₋₄-alkyl)-aminogruppe,
eine durch die Reste R¹⁵ und R¹⁶ substituierte Aminogruppe, in der
R¹⁵ ein Wasserstoffatom oder eine Methyl- oder Ethylgruppe und
R¹⁶ eine C₁₋₄-Alkylgruppe, die durch eine Cyan-, Carboxy-, Methoxycarbonyl-, Ethyloxycarbonyl-, Aminocarbonyl-, Methylaminocarbonyl-, Dimethylaminocarbonyl-, Ethylaminocarbonyl-, Diethylaminocarbonyl-, Pyrrolidin-1-yl-carbonyl- oder Morpholin-4-ylcarbonyl-Gruppe substituiert ist, bedeuten,
eine durch die Reste R¹⁵ und R¹⁷ substituierte Aminogruppe, in der
R¹⁵ wie vorstehend erwähnt definiert ist und
R¹⁷ eine geradkettige C₂₋₄-Alkylgruppe, die jeweils endständig durch eine Amino-, Methylamino-, Dimethylamino-, Acetylamino-, Ethyloxycarbonylamino-, Phenylcarbonylamino-, Methylsulfonylamino-, Phenylsulfonylamino-, Hydroxy-, Methoxy-, Phenyloxy-, Methylsulfanyl- oder Phenylsulfanyl-Gruppe substituiert ist, bedeutet,
eine Pyrrolidin-1yl-, Piperidin-1-yl-, Morpholin-4-yl-, Piperazin-1-yl- oder 4-Methylpiperazin-1-yl-Gruppe,
eine C₃₋₆-Cycloalkylamino- oder C₃₋₆-Cycloalkyl-C₁₋₃-alkylamino-Gruppe,
eine Phenylaminogruppe,
eine Phenyl-C₁₋₃-alkylaminogruppe, in der der Phenylteil durch R¹⁰ und R¹¹ substituiert ist, wobei R¹⁰ und R¹¹ wie vorstehend erwähnt definiert sind,
eine Naphthylmethylaminogruppe,
eine Heteroaryl-C₁₋₂-alkylaminogruppe, wobei der Begriff Heteroaryl wie vorstehend erwähnt definiert ist, oder
eine Methylsulfanyl-, Benzylsulfanyl- oder (2-Phenylethyl)sulfanyl-gruppe,
R³ eine C₄₋₆-Alkenylgruppe,
eine C₃₋₄-Alkenylgruppe, die durch ein Fluor- Chlor- oder Bromatom oder eine Trifluormethylgruppe substituiert ist,
eine 2-Butin-1-ylgruppe oder
eine durch die Gruppe R_{c} substituierte Methylgruppe, wobei
R_{c} eine 1-Cyclopenten-1-yl-oder 1-Cyclohexen-1-yl-Gruppe,
eine gegebenenfalls durch ein Fluor-, Chlor-, Brom- oder lodatom, durch eine Methyl-, Trifluormethyl-, Cyan-, Methoxy-, Difluormethoxy- oder Trifluormethoxygruppe substituierte Phenylgruppe,
eine Phenylgruppe, die durch zwei Fluoratome substituiert ist,
eine Naphthylgruppe oder
eine Furanyl-, Thienyl-, oder Pyridylgruppe bedeutet,
und
R⁴ eine Piperidin-1-ylgruppe, die in 3-Stellung durch eine Aminogruppe substituiert ist,
eine Hexahydroazepin-1-yl-gruppe, die in 3-Stellung oder in 4-Stellung durch eine Aminogruppe substituiert ist,
eine (2-Aminocyclohexyl)amino-Gruppe,
eine Cyclohexylgruppe, die in 3-Stellung durch eine Aminogruppe substituiert ist, oder
eine N-(2-Aminoethyl)-methylamino- oder eine N-(2-Aminoethyl)-ethylamino-Gruppe bedeuten,
wobei soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische und deren Salze.

Besonders bevorzugte Verbindungen der obigen allgemeinen Formel I sind diejenigen, in denen
R¹ ein Wasserstoffatom,
eine Methyl-, Benzyl- oder 2-Phenylethylgruppe,
eine Naphthylmethyl- oder Methoxynaphthylmethylgruppe oder
eine Phenylcarbonylmethylgruppe,
R² ein Wasserstoffatom,
eine Methyl- oder 2-Phenylethylgruppe,
eine Phenylcarbonylmethyl-Gruppe,
eine Cyanogruppe,
eine Amino-, Methylamino-, Dimethylamino-, Isopropylamino-, Cyclohexylamino- oder (Cyclohexylmethyl)amino-Gruppe,
eine Benzylamino-, Fluorbenzylamino- oder (2-Phenylethyl)amino-Gruppe oder
eine Piperidin-1-ylgruppe,
R³ eine Benzyl- oder 3-Methyl-but-2-en-1-yl-Gruppe
und
R⁴ eine (3-Amino-piperidin-1-yl)-Gruppe bedeuten,
deren Tautomere, Enantiomere, Diastereomere, deren Gemische und deren Salze,
insbesondere jedoch die Verbindungen
(1) 8-(3-Amino-piperidin-1-yl)-7-benzyl-2-benzylamino-1-methyl-1,7-dihydropurin-6-on,
(2) 8-(3-Amino-piperidin-1-yl)-7-benzyl-2-(4-fluor-benzylamino)-1-methyl-1,7-dihydro-purin-6-on,
(3) 8-(3-Amino-piperidin-1-yl)-7-benzyl-1-methyl-2-[(2-phenylethyl)amino]-1,7-dihydro-purin-6-on,
(4) 8-(3-Amino-piperidin-1-yl)-7-benzyl-2-isopropylamino-1-methyl-1,7-dihydropurin-6-on,
(5) 8-(3-Amino-piperidin-1-yl)-7-benzyl-1-methyl-2-methylamino-1,7-dihydropurin-6-on,
(6) 8-(3-Amino-piperidin-1-yl)-7-benzyl-2-cyclohexylamino-1-methyl-1,7-dihydropurin-6-on,
(7) 8-(3-Amino-piperidin-1-yl)-7-benzyl-2-[(cyclohexylmethyl)amino]-1-methyl-1,7-dihydro-purin-6-on,
(8) 8-(3-Amino-piperidin-1-yl)-7-benzyl-1-methyl-2-(piperidin-1-yl)-1,7-dihydropurin-6-on,
(9) 8-(3-Amino-piperidin-1-yl)-7-benzyl-2-dimethylamino-1-methyl-1,7-dihydropurin-6-on,
(10) 2-Amino-8-(3-amino-piperidin-1-yl)-7-benzyl-1-methyl-1,7-dihydro-purin-6-on,
(11) 8-(3-Amino-piperidin-1-yl)-2-benzylamino-1-methyl-7-(3-methyl-but-2-en-1-yl)-1,7-dihydro-purin-6-on,
(12) 8-(3-Amino-piperidin-1-yl)-7-benzyl-1-methyl-2-methyl-1,7-dihydro-purin-6-on,
(13) 8-(3-Amino-piperidin-1-yl)-1-methyl-7-(3-methyl-but-2-en-1-yl)-2-(2-phenylethyl)-1,7-dihydro-purin-6-on,
(14) 8-(3-Amino-piperidin-1-yl)-7-benzyl-1-methyl-1,7-dihydro-purin-6-on,
(15) 8-(3-Amino-piperidin-1-yl)-7-benzyl-1-(2-oxo-2-phenyl-ethyl)-1,7-dihydropurin-6-on ,
(16) 8-(3-Amino-piperidin-1-yl)-2-methyl-7-(3-methyl-but-2-en-1-yl)-1-[(naphthalin-1-yl)methyl]-1,7-dihydro-purin-6-on,
(17) 8-(3-Amino-piperidin-1-yl)-7-(3-methyl-but-2-en-1-yl)-1-[(naphthalin-1-yl)methyl]-1,7-dihydro-purin-6-on und
(18) 8-(3-Amino-piperidin-1-yl)-7-(3-methyl-but-2-en-1-yl)-1-[(4-methoxynaphthalin-1 -yl)methyl]-1,7-dihydro-purin-6-on
sowie deren Tautomere, Enantiomere, Diastereomere, deren Gemische und deren Salze.

Die Verbindungen der allgemeinen Formel I lassen sich herstellen durch Entschützung einer Verbindung der allgemeinen Formel 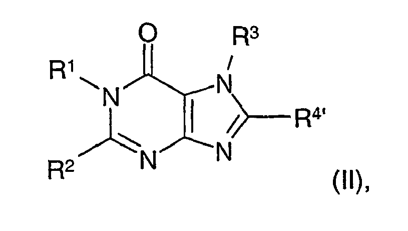 in der R¹, R² und R³ wie eingangs erwähnt definiert sind und
R⁴ eine der eingangs für R⁴ erwähnten Gruppen bedeutet, die eine Imino-, Amino- oder Alkylaminogruppe enthalten, wobei die Imino-, Amino- bzw. Alkylaminogruppe durch eine Schutzgruppe substituiert ist, gegebenenfalls gefolgt von einer nachträglichen Alkylierung der Imino-, Amino- bzw. C₁₋₃-Alkylaminogruppe.

Die Freisetzung einer Aminogruppe aus einer geschützten Vorstufe ist eine Standardreaktion in der synthetischen organischen Chemie. Als Schutzgruppen kommen eine Vielzahl von Gruppen in Frage. Eine Übersicht über die Chemie der Schutzgruppen findet sich in Theodora W.Greene und Peter G.M.Wuts, Protective Groups in Organic Synthesis, Second Edition, 1991, Verlag John Wiley and Sons sowie in Philip J. Kocienski, Protecting Groups, Georg Thieme Verlag, 1994.

Als Beispiele für Schutzgruppen seien genannt:
die tert.-Butyloxycarbonylgruppe, die sich durch Behandeln mit einer Säure wie beispielsweise Trifluoressigsäure oder Chlorwasserstoff in Gegenwart eines Lösungsmittels wie beispielsweise Methylenchlorid, Essigester oder Dioxan bei Temperaturen zwischen 0°C und der Siedetemperatur des verwendeten Lösungsmittels abspalten lässt,
die 2.2.2-Trichlorethoxycarbonylgruppe, die sich abspalten lässt durch Behandeln mit Metallen wie beispielsweise Zink oder Cadmium in einem Lösungsmittel wie Essigsäure oder einem Gemisch aus Tetrahydrofuran und einer schwachen wässrigen Säure bei Temperaturen zwischen 0°C und der Siedetemperatur des verwendeten Lösungsmittels und
die Carbobenzyloxycarbonylgruppe, die sich beispielsweise abspalten lässt durch Hydrogenolyse in Gegenwart eines Edelmetallkatalysators wie beispielsweise Palladium-Kohle und einem Lösungsmittel wie beispielsweise Alkohole, Essigester, Dioxan, Tetrahydrofuran oder Gemische dieser Lösungsmittel bei Temperaturen zwischen 0°C und dem Siedepunkt des Lösungsmittels, durch Behandeln mit Bortribromid in Methylenchlorid bei Temperaturen zwischen -20°C und Raumtemperatur, oder durch Behandeln mit Aluminiumchlorid/Anisol bei Temperaturen zwischen 0°C und Raumtemperatur.

Die gegebenenfalls nachträgliche Einführung eines C₁₋₃-Alkylrests kann mittels Alkylierung oder reduktiver Alkylierung erfolgen.

Die nachträgliche Alkylierung wird gegebenenfalls in einem Lösungsmittel oder Lösungsmittelgemisch wie Methylenchlorid, Dimethylformamid, Benzol, Toluol, Chlorbenzol, Tetrahydrofuran, Benzol/Tetrahydrofuran oder Dioxan mit einem Alkylierungsmittel wie einem entsprechenden Halogenid oder Sulfonsäureester, z.B. mit Methyliodid, Ethylbromid, Dimethylsulfat, gegebenenfalls in Gegenwart einer tertiären organischen Base oder in Gegenwart einer anorganischen Base zweckmäßigerweise bei Temperaturen zwischen 0 und 150°C, vorzugsweise bei Temperaturen zwischen 0 und 100°C, durchgeführt.

Die nachträgliche reduktive Alkylierung wird mit einer entsprechenden Carbonylverbindung wie Formaldehyd, Acetaldehyd, Propionaldehyd, Aceton in Gegenwart eines komplexen Metallhydrids wie Natriumborhydrid, Lithiumborhydrid, Natriumtriacetoxyborhydrid oder Natriumcyanoborhydrid zweckmäßigerweise bei einem pH-Wert von 6-7 und bei Raumtemperatur oder in Gegenwart eines Hydrierungskatalysators, z.B. mit Wasserstoff in Gegenwart von Palladium/Kohle, bei einem Wasserstoffdruck von 1 bis 5 bar durchgeführt. Die Methylierung kann auch in Gegenwart von Ameisensäure als Reduktionsmittel bei erhöhten Temperaturen, z.B. bei Temperaturen zwischen 60 und 120°C, durchgeführt werden.

Die Verbindungen der allgemeinen Formel I können in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der allgemeinen Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel 1, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Arginin, Cyclohexylamin, Ethanolamin, Diethanolamin und Triethanolamin in Betracht.

Die Ausgangsverbindungen der allgemeinen Formel II lassen sich nach allgemein bekannten Methoden und nach den beispielsweise in den Beispielen I bis XIV beschriebenen Verfahren herstellen.

So lassen sich Verbindungen der allgemeinen Formel II, in denen R¹, R³ und R⁴' wie vorstehend erwähnt definiert sind und R² ein Wasserstoffatom bedeutet, beispielsweise durch Umsetzung einer Verbindungen der Formel 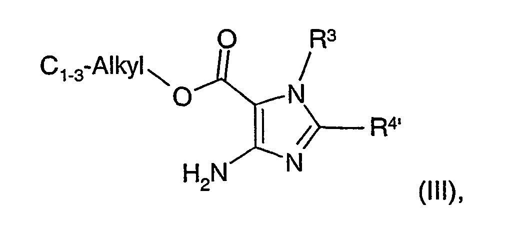 mit einem Formimido-C₁₋₃-alkylester, gegebenenfalls gefolgt von einer Alkylierung am N-1 mit einem geeigneten Alkylierungsmittel herstellen.

Verbindungen der allgemeinen Formel II, in denen R¹, R³ und R^{4'} wie vorstehend erwähnt definiert sind und R² eine Alkylsulfanyl-, eine Alkenylsulfanyl- oder Alkinylsulfanylgruppe bedeutet, lassen sich beispielsweise erhalten durch Umsetzung von Verbindungen der allgemeinen Formel III mit einem geeigneten Isothiocyanat und anschliessender Cyclisierung zu Verbindungen der allgemeinen Formel 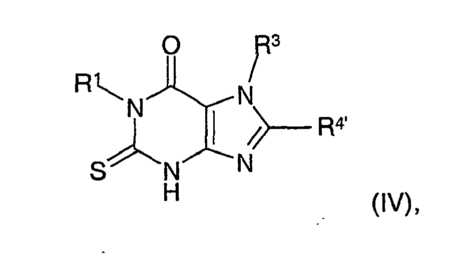 gefolgt von einer Alkylierung des Schwefelatoms.

Wird ein Acylsenföl eingesetzt, wie beispielsweise Ethoxycarbonylisothiocyanat, erhält man zunächst Verbindungen der allgemeinen Formel IV, in denen R¹ ein Wasserstoffatom darstellt, die sich durch nachfolgende Alkylierung am Schwefelatom und an N-1 in die gewünschten Verbindungen überführen lassen.

Verbindungen der allgemeinen Formel II, in denen R¹, R³ und R^{4'} wie vorstehend erwähnt definiert sind und R² eine Alkylsulfanylgruppe bedeutet, lassen sich oxidieren zu Verbindungen der Formel II', in denen R^{2'} eine Alkylsulfinyl- oder eine Alkylsulfonylgruppe darstellt.

Die vorstehend erwähnten Verbindungen der allgemeinen Formel II' stellen Ausgangsmaterialien zur Herstellungen der folgenden Verbindungen der Formel II dar.

Umsetzung mit Alkoholen und Phenolen ergibt Verbindungen, in denen der Rest R² über ein Sauerstoffatom mit dem Purinsystem verbunden ist,

Umsetzung mit Thiolen und Thiophenolen führt zu Verbindungen, in denen R² über ein Schwefelatom mit dem Purinsystem verbunden ist,

Umsetzung mit Aminen führt zu Verbindungen, in denen R² über ein Stickstoffatom an das Purinsystem gebunden ist und

Umsetzung mit metallorganischen Verbindungen wie beispielsweise Grignard-Reagentien, Alkyl- oder Aryl-Lithiumverbindungen oder Umsetzung mit CH-aciden-Verbindungen wie beispielsweise Ester, Nitrile oder Ketonen führt zu Verbindungen, in denen R² über ein Kohlenstoffatom mit dem Purinsystem verbunden ist.

Eine weitere Methode, Verbindungen der allgemeinen Formel II zu erhalten, besteht beispielsweise darin, Verbindungen der allgemeinen Formel 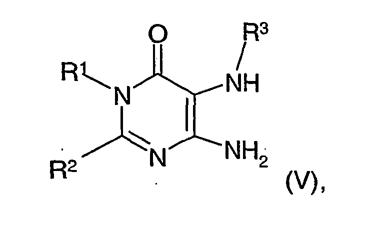 in der R¹, R² und R³ wie vorstehend erwähnt definiert sind, in Verbindungen der allgemeinen Formel II umzuwandeln.

Beispielsweise erfolgt die Umwandlung von Verbindungen der allgemeinen Formel V zu Verbindungen der allgemeinen Formel II, in denen der Rest R^{4'} über ein Stickstoffatom an das Purinsystem gebunden ist, durch Umsetzung mit einem Ameisensäureorthoester, nachfolgender Bromierung des so erhaltenen Purins an C-8 und anschliessender Umsetzung mit einem entsprechenden Amin.

Beispielsweise erfolgt die Umwandlung von Verbindungen der allgemeinen Formel V zu Verbindungen der allgemeinen Formel II, in denen der Rest R^{4'} über ein C-Atom an das C-8-Atom des Purins gebunden ist, durch Umsetzung mit einem reaktiven Derivat einer Carbonsäure R^{4'}-COOH, wobei R4' wie oben erwähnt definiert ist, und anschliessender Cyclisierung.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:

Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2" , erschienen in Proc. Natl. Acad. Sci. Vol. 90, Seiten 5757-5761 (1993), durchgeführt. Der Zellextrakt wurde von in einem Puffer (10mM Tris HCl, 0.15 M NaCl, 0.04 t.i.u. Aprotinin, 0.5% Nonidet-P40, pH 8.0) solubilisierten Zellen durch Zentrifugation bei 35,000 g für 30 Minuten bei 4°C (zur Entfernung von Zelltrümmern) gewonnen.

Der DPP-IV Assay wurde wie folgt durchgeführt:
50 µl Substratlösung (AFC; AFC ist Amido-4-trifluormethylcoumarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20*µ*l vorverdünnt zugefügt, wobei das Assaypuffervolumen dann entsprechend reduziert wurde. Die Reaktion wurde bei Raumtemperatur durchgeführt, die Inkubationsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden.

Die erhaltenen Ergebnisse sind in der folgenden Tabelle zusammengefasst:

| Verbindung (Beispiel-Nr.) | DPP IV-Hemmung IC₅₀ (nM) |
|---|---|
| 1 | 11 |
| 1(1) | 24 |
| 1(2) | 42 |
| 1(3) | 110 |
| 1(4) | 58 |
| 1(5) | 134 |
| 1(6) | 48 |
| 1(7) | 434 |
| 1(8) | 213 |
| 1(9) | 61 |
| 1(11) | 54 |
| 1(12) | 18 |
| 1(13) | 152 |
| 1(14) | 158 |
| 1(15) | 58 |
| 1(22) | 48 |
| 1(23) | 157 |
| 1(24) | 113 |
| 1(25) | 275 |
| 1(26) | 40 |
| 1(27) | 19 |
| 1(28) | 57 |

Die erfindungsgemäß hergestellten Verbindungen sind gut verträglich, da beispielsweise nach oraler Gabe von 30 mg/kg der Verbindung des Beispiels 1 an Ratten keine toxischen Nebenwirkungen oder Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ 1 und Typ 2, diabetische Komplikationen. (wie z.B. Retinopathie, Nephropathie oder Neuropathien), metabolische Azidose oder Ketose, reaktiver Hypoglykämie, Insulinresistenz, Metabolischem Syndrom, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Weiterhin sind die erfindungsgemäßen Verbindungen zur Behandlung entzündlicher Erkrankungen der Atemwege einsetzbar. Ebenso sind sie zur Prävention und Therapie von chronischen entzündlichen Darmerkrankungen wie z.B. Reizdarmsyndrom (IBS), Morbus Crohn oder Colitis ulcerosa ebenso wie bei Pankreatitis geeignet. Des weiteren wird erwartet, daß sie bei jeglicher Art von Verletzung oder Beeinträchtigung im Gastrointestinaltrakt eingesetzt werden können wie auch z.B. bei Kolitiden und Enteriden. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Auf der anderen Seite sind diese Substanzen geeignet, die Motilität der Spermien zu beeinflussen und sind damit als Kontrazeptiva zur Verwendung beim Mann einsetzbar. Des weiteren sind die Substanzen geeignet, Mangelzustände von Wachstumshormon, die mit Minderwuchs einhergehen, zu beeinflussen, sowie bei allen Indikationen sinnvoll eingesetzt werden können, bei denen Wachstumshormon verwendet werden kann. Die erfindungsgemäßen Verbindungen sind auf Grund ihrer Hemmwirkung gegen DPP IV auch geeignet zur Behandlung von verschiedenen Autoimmunerkrankungen wie z.B. rheumatoide Arthritis, Multiple Sklerose, Thyreoditiden und Basedow'scher Krankheit etc.. Darüberhinaus können sie eingesetzt werden bei viralen Erkrankungen wie auch z.B. bei HIV Infektionen, zur Stimulation der Blutbildung, bei benigner Prostatahyperplasie, bei Gingivitiden, sowie zur Behandlung von neuronalen Defekten und neurdegenerativen Erkrankungen wie z.B. Morbus Alzheimer. Beschriebene Verbindungen sind ebenso zu verwenden zur Therapie von Tumoren, insbesondere zur Veränderung der Tumorinvasion wie auch Metastatisierung, Beispiele hier sind die Anwendung bei T-Zell Lymphomen, akuter lymphoblastischer Leukämie, zellbasierende Schilddrüsenkarzinome, Basalzellkarzinome oder Brustkarzinome. Weitere Indikationen sind Schlaganfall, Ischämien verschiedenster Genese, Morbus Parkinson und Migräne. Darüberhinaus sind weitere Indikationsgebiete follikuläre und epidermale Hyperkeratosen, erhöhte Keratinozytenproliferation, Psoriasis, Enzephalomyelitiden, Glomerulonephritiden, Lipodystrophien, sowie psychosomatische, depressive und neuropsychiatrische Erkrankungen verschiedenster Genese.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570) und -Antagonisten, PPAR-gamma/alpha Modulatoren (z.B. KRP 297), alpha-Glucosidasehemmer (z.B. Acarbose, Voglibose),andere DPPIV Inhibitoren, alpha2-Antagonisten, Insulin und Insulinanaloga, GLP-1 und GLP-1 Analoga (z.B. Exendin-4) oder Amylin. Daneben Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha agonisten, PPAR-delta agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder β₃-Agonisten wie SB-418790 oder AD-9677 ebenso wie Agonisten des 5HT2c Rezeptors.

Daneben ist eine Kombination mit Medikamenten zur Beeinflussung des Bluthochdrucks wie z.B. All Antagonisten oder ACE Inhibitoren, Diuretika, β-Blocker, Ca-Antagonisten und anderen oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

### Herstellung der Ausgangsverbindungen:

### Beispiel I

### 5-Amino-3-benzyl-2-(3-tert.-butyloxycarbonylamino-piperidin-1-yl)-3H-imidazol-4-carbonsäure-ethylester

50 g (0.199 Mol) N-Benzyl-N'-cyano-O-phenyl-isoharnstoff und 40.056 g (0.2 Mol) 3-tert.-Butyloxycarbonylamino-piperidin werden in 50 ml Dimethylformamid (DMF) 4 Stunden auf 80°C erwärmt. Nach Stehen über Nacht werden 250 ml Essigester zugegeben, der Niederschlag abgesaugt, mit Essigester und Ether gewaschen und getrocknet. Aus der Mutterlauge erhält man nach Einengen und Behandeln des Rückstands mit Essigester und Ether weiteres Produkt. Gesamtausbeute: 48,0 g (67,5% d.Th.) N-Benzyl-N'-cyano-(3-tert.-butyloxy-carbonylamino-piperidin)-1-carboxamidin.
R_{f}-Wert: 0.56 (Aluminiumoxid, Methylenchlorid/Methanol = 40:1)

10.008 g (28 mMol) dieser Substanz werden in 15 ml DMF gelöst. Nach Zugabe von 4.256 g (30.8 mMol) Kaliumcarbonat wird der Ansatz drei Minuten mit Ultraschall behandelt, dann werden 3.416 ml (30.8 mMol) Bromessigsäureethylester auf einmal zugegeben und der Ansatz 36 Stunden bei Raumtemperatur gerührt, wobei zur besseren Rührbarkeit nach 8 Stunden weitere 10 ml DMF zugegeben wurden. Das Reaktionsgemisch wird mit Wasser verrührt, es wird mit Essigester extrahiert, die organische Phase getrocknet und eingedampft. Das erhaltene Harz wird durch Säulenchromatographie gereinigt (Kieselgel, Essigester/Petrolether = 3:1 bis 9:1) Man erhält 5.4 g (43.5 % d.Th.) N-Benzyl-N'-cyano-N-ethoxycarbonylmethyl-(3-tert.-butyloxy-carbonylamino-piperidin)-1-carboxamidin.
R_{f}-Wert: 0.7 (Kieselgel, Essigester/Petrolether = 4:1)

5.1 g (11.498 mMol) dieser Verbindung werden portionsweise zu einer Lösung von 0.785 g (11.536 mMol) Natriumethanolat in 25 ml Ethanol gegeben. Der Ansatz wird 40 Minuten bei 60°C gerührt, dann mit 50 ml Ethanol und 10 ml Wasser versetzt und abgekühlt. Der Niederschlag wird abgesaugt und in Methylenchlorid gelöst. Nach Trocknen und Verdampfen des Lösungsmittels erhält man 4.8 g (94.1 % d.Th.) der Titelverbindung.
R_{f}-Wert: 0.4 (Kieselgel, Essigester/Petrolether = 4:1)

Analog Beispiel I wurde erhalten:

### (1) 5-Amino-3-(3-methyl-but-2-enyl)-2-(3-tert.-butyloxycarbonylamino-piperidin-1-yl)-3H-imidazol-4-carbonsäure-ethylester

hergestellt aus Diphenyl-N-cyancarbonimidat, Glycinethylester, (3-tert.-Butyloxycarbonylamino)-piperidin und 3-Methyl-but-2-en-1-yl-bromid.
R_{f}-Wert: 0.1-0.2 (Kieselgel, Essigester/Petrolether = 1:1)

### Beispiel II

### [1-(7-Benzyl-6-oxo-6,7-dihydro-1H-purin-8-yl)-piperidin-3-yl]carbaminsäure-tert.-butylester

360 mg (0.812 mMol) 5-Amino-3-benzyl-2-(3-tert.-butyloxy-carbonylamino-piperidin-1-yl)-3H-imidazol-4-carbonsäure-ethylester und 131.472 (1.2 mMol) Ethylformidat Hydrochlorid werden in 1.3 g Phenol vorgelegt. Eine Lösung von 141.667 mg (2mMol) Natriumethylat in 2 ml Tetrahydrofuran (THF) wird unter Rühren zugetropft, das THF verdampft und der Ansatz 2 Stunden bei 150°C gehalten. Das braune Reaktionsgemisch wird über eine Kieselgelsäule gereinigt. Man erhält 50 mg (15.7 % d.Th.) der Titelverbindung.
Schmelzpunkt: 208°C.
R_{f}-Wert: 0.15 (Kieselgel, Methylenchlorid/Methanol = 15:1)

### Beispiel III

### [1-(7-Benzyl-1-methyl-6-oxo-6,7-dihydro-1H-purin-8-yl)-piperidin3-yl]carbaminsäure-tert.-butylester

Zu einer Lösung von 42 mg (0.099 mMol) der Verbindung von Beispiel II in 0.3 ml DMF werden 19 mg (0.138 mMol) Kaliumcarbonat und anschliessend 16 mg (0.113 mMol) Methyliodid gegeben. Es wird 2 Stunden bei Raumtemperatur gerührt, dann mit Wasser verrieben und mit Essigester extrahiert. Die organische Phase wird mit Wasser gewaschen und mit Aktivkohle und Magnesiumsulfat getrocknet. Nach Verdampfen erhält man 29 mg (66.8 % d.Th.) der Titelverbindung.
R_{f}-Wert: 0.3 (Kieselgel, Methylenchlorid/Methanol = 15:1)

Analog Beispiel III wurde erhalten:
(1) [1-(7-Benzyl-1-{2-oxo-2-phenyl-ethyl}-6-oxo-6,7-dihydro-1H-purin-8-yl)-piperidin-3-yl]-carbaminsäure-tert.-butylester
   hergestellt aus Beispiel II und Phenacylbromid.
   R_{f}-Wert: 0.4 (Kieselgel, Methylenchlorid/Methanol = 15:1)

### Beispiel IV

### [1-(7-Benzyl-2-methylsulfanyl-6-oxo-6,7-dihydro-1H-purin-8-yl)-piperidin-3-yl]-carbaminsäure-tert.-butylester

In eine Lösung von 10.8 g (24.345 mMol) der Verbindung von Beispiel I in 45 ml THF werden 3.186 ml (27 mMol) Ethyloxycarbonylisothiocyanat zugetropft. Der Ansatz wird eine Stunde zum Sieden erhitzt, eingedampft und der Rückstand durch Behandeln mit Diisopropylether zur Kristallisation gebracht.
Man erhält 13.5 g (96.5 % d.Th.) 3-Benzyl-2-(3-tert.-Butyloxycarbonylaminopiperidin-1-yl)-5-(N'-ethyloxycarbonyl-thioureido)-3H-imidazol-4-carbonsäureethylester.

13 g (22.620 mMol) dieser Verbindung werden in 21 ml n-Butanol vorgelegt. Nach Zugabe von 2.536 (22.6 mMol) Kalium-tert.-butylat wird 45 Minuten bei 100°C gerührt, wobei ein Niederschlag ausfällt. Nach Stehen über Nacht bei Raumtemperatur wird mit Ether versetzt, abgesaugt und getrocknet. Man erhält 10.6 g (94.7 % d.Th.) [1-(7-Benzyl-2-mercapto-6-oxo-6,7-dihydro-1H-purin-8-yl)-piperidin-3-yl]-carbaminsäure-tert.-butylester-Kalium-Salz.

10.5 g (21.225 mMol) dieser Verbindung werden in 25 ml Wasser suspendiert, und es wird Ethanol bis zur Lösung zugegeben. Nach Zugabe von 2.135 ml (21.515 mMol) Dimethylsulfat 4 Stunden bei Raumtemperatur gerührt. Der Niederschlag wird abgesaugt, mit kaltem Ethanol gewaschen und getrocknet. Man erhält 8.8 g (88.1 % d.Th.) der Titelverbindung.
R_{f}-Wert: 0.3 (Kieselgel, Methylenchlorid/Methanol = 20:1)

Analog Beispiel IV wurden erhalten:
(1) [1-(7-Benzyl-2-benzylsulfanyl-6-oxo-6,7-dihydro-1H-purin-8-yl)-piperidin-3-yl]-carbaminsäure-tert.-butylester
   hergestellt aus Verbindung I, Ethyloxycarbonylisothiocyanat und Benzylbromid.
   R_{f}-Wert: 0.65 (Kieselgel, Ethylacetat/Petrolether = 2:1)
(2) [1-(7-Allyl-2-methylsulfanyl-6-oxo-6,7-dihydro-1H-purin-8-yl)-piperidin-3-yl]-carbaminsäure-tert.-butylester
   hergestellt aus Verbindung I.1, Ethyloxycarbonylisothiocyanat und Methyliodid.
   R_{f}-Wert: 0.6 (Kieselgel, Methylenchlorid/Methanol = 10:1)
(3) [1-(7-Benzyl-2-[2-phenylethyl]sulfanyl-6-oxo-6,7-dihydro-1 H-purin-8-yl)-piperidin-3-yl]-carbaminsäure-tert.-butylester.
   hergestellt aus Verbindung 1, Ethyloxycarbonylisothiocyanat und 2-Phenylethylbromid.
   R_{f}-Wert: 0.65 (Kieselgel, Essigester/Petrolether = 2:1)

### Beispiel V

### [1-(7-Benzyl-1-methyl-2-methylsulfanyl-6-oxo-6,7-dihydro-1H-purin-8-yl)-piperidin-3-yl]-carbaminsäure-tert-butylester

Eine Suspension von 2.5 g (5.312 mMol) der Verbindung IV in 15 ml DMF wird mit 645.15 mg (5.75mMol) Kalium-tert.-Butylat versetzt. Zur entstehenden Lösung werden 922.605 mg (6.5 mMol) Methyliodid gegeben und das Gemisch über Nacht bei Raumtemperatur gerührt. Es wird Wasser zugegeben und mit Methylenchlorid extrahiert. Die organische Phase wird mit Wasser gewaschen, getrocknet und eingedampft. Der Rückstand wird mit Diisopropylether kristallisiert. Man erhält 2 g (77.7 % d.Th.) der Titelverbindung.
R_{f}-Wert: 0.55 (Kieselgel, Methylenchlorid/Methanol = 20:1)

Analog Beispiel V wurden erhalten:
(1) [1-(7-Benzyl-2-methylsulfanyl-1-phenacyl-6-oxo-6.7.dihydro-1H-purin-8-yl)-piperidin-3-yl]-carbaminsäure-tert.-butylester
   hergestellt aus Verbindung IV und Phenacylbromid.
   R_{f}-Wert: 0.7 (Aluminiumoxid, Methylenchlorid/Methanol =40:1)
(2) [1-(1-methyl-7-(3-methyl-butenyl)-2-methylsulfanyl-6-oxo-6,7-dihydro-1 H-purin-8-yl)-piperidin-3-yl]-carbaminsäure-tert.-butylester
   hergestellt aus Verbindung IV.1 und Methyliodid.
   R_{f}-Wert: 0.4 (Kieselgel, Methylenchlorid/Methanol = 20:1)
(3) [1-(1-Benzyl-7-benzyl-2-methylsulfanyl-6-oxo-6,7-dihydro-1H-purin-8-yl)-piperidin-3-yl]-carbaminsäure-tert.-butylester
   hergestellt aus Verbindung IV und Benzylbromid.
   R_{f}-Wert: 0.75 (Kieselgel, Essigester/Petrolether = 4:1)
(4) [1-(7-Benzyl-2-methylsulfanyl-1-(2-phenylethyl)-6-oxo-6,7-dihydro-1H-purin-8-yl)-piperidin-3-yl]-carbaminsäure-tert.-butylester
   hergestellt aus Verbindung IV und 2-Phenylethylbromid.
   R_{f}-Wert: 0.75 (Kieselgel, Essigester/Petrolether = 4:1)
(5) [1-(7-Benzyl-2-benzylsulfanyl-1-methyl-6-oxo-6,7-dihydro-1H-purin-8-yl)-piperidin-3-yl]-carbaminsäure-tert.-butylester
   aus Verbindung IV.1 und Methyliodid.
   hergestellt R_{f}-Wert: 0.85 (Kieselgel, Essigester/Petrolether = 1:2)
(6) [1-(7-Benzyl-2-[2-phenylethyl]sulfanyl-1-methyl-6-oxo-6,7-dihydro-1H-purin-8-yl)-piperidin-3-yl]-carbaminsäure-tert.-butylester
   hergestellt aus Verbindung IV.3 und Methyliodid.
   R_{f}-Wert: 0.65 (Kieselgel, Essigester/Petrolether = 1:2)

### Beispiel VI

### [1-(7-Benzyl-1-methyl-2-methylsulfinyl-6-oxo-6,7-dihydro-1H-purin-8-yl)-piperidin-3-yl]-carbaminsäure-tert.-butylester und [1-(7-Benzyl-1-methyl-2-methylsulfonyl-6-oxo-6,7-dihydro-1H-purin-8-yl)-piperidin-3-yl]-carbaminsäure-tert.-butylester

Eine Lösung von 250 mg (0.516 mMol) der Verbindung V in 5 ml Dichlormethan und 0.5 ml Methanol wird unter Rühren und Eiskühlung mit 120.789 mg (0.7 mMol) m-Chlor-perbenzoesäure versetzt. Nach 30 Minuten wird das Eisbad entfernt und über Nacht bei Raumtemperatur gerührt. Es werden 50 ml Methylenchlorid zugegeben und mit 10-%-iger Sodalösung ausgeschüttelt. Die organische Phase wird mit Wasser gewaschen, getrocknet und eingedampft. Man erhält 220 mg der beiden Titelverbindungen im Verhältnis 45: 55.
R_{f}-Wert: 0.1 (Sulfoxid) und 0.8 (Sulfon) (Kieselgel, Essigester)

Analog Beispiel VI wurden erhalten:
(1) [1-Methyl-7-(3-methy)-but-2-enyl)-2-methylsulfinyl-6-oxo-6,7-dihydro-1H-purin-8-yl]-piperidin-3-yl)-carbaminsäure-tert.-butylester und[1-methyl-7-(3-methyl-but-2-enyl)-2-methylsulfonyl-6-oxo-6,7-dihydro-1H-purin-8-yl-]-piperidin-3-yl)-carbaminsäure.tert.-butylester
   hergestellt aus Verbindung V.2 und m-Chlor-perbenzoesäure. Das nach einer Reaktionszeit von 80 Minuten erhaltene Produkt stellt das Sulfoxid dar, das max.10% Sulfon enthält.
   R_{f}-Wert: 0.5 (Kieselgel, Methylenchlorid/Methanol =10:1)
(2) [1-(7-Benzyl-2-methansulfonyl-1-methyl-6-oxo-6,7-dihydro-1H-purin-8-yl)-piperidin-3-yl]-carbaminsäure-tert.-butylester
   hergestellt aus Verbindung V.R_{f}-Wert: 0.75 (Kieselgel, Essigester)
   Massenspektrum (ESI⁺): m/z = 517 [M+H]⁺

### Beispiel VII

### [1-(7-Benzyl -2-benzylamino-1-methyl-6-oxo-6,7-dihydro-1H-purin-8-yl)-piperidin-3-yl]-carbaminsäure-tert.-butylester

258.312 mg des in Beispiel VI erhaltenen Gemisches und 214.313 mg Benzylamin werden 16 Stunden bei Raumtemperatur gerührt. Der Ansatz wird mit 20 ml Diisopropylether verrieben, der Niederschlag abgesaugt, in wenig Methylenchlorid gelöst und mit Diisopropylether kristallisiert. Man erhält 250 mg der Titelverbindung. R_{f}-Wert: 0.55 (Kieselgel, Essigester)

Analog Beispiel VII wurden die folgenden Verbindungen dargestellt:
(1) [1-(7-Benzyl-2-[4-fluor-benzyl]amino-1-methyl-6-oxo-6,7-dihydro-1H-purin-8-yl)-piperidin-3-yl]-carbaminsäure-tert.-butylester
   hergestellt aus Beispiel VI und 4-Fluor-benzylamin
   R_{f}-Wert: 0.49 (Kieselgel, Essigester)
(2) [1-(7-Benzyl-1-methyl-2-(2-phenylethyl)amino-6-oxo-6,7-dihydro-1H-purin-8-yl)-piperidin-3-yl]-carbaminsäure-tert.-butylester
   hergestellt aus Beispiel VI und (2-(2-Phenylethyl)amin.
   R_{f}-Wert: 0.5 (Kieselgel, Essigester)
(3) [1-(7-Benzyl-2-isopropylamino-1-methyl-6-oxo-6,7-dihydro-1H-purin-8-yl)-piperidin-3-yl-]-carbaminsäure-tert.-butylester
   hergestellt aus Beispiel VI und Isopropylamin.
   R_{f}-Wert: 0.6 (Kieselgel, Methylenchlorid/Methanol = 9:1)
(4) [1-(7-Benzyl-1-methyl-2-methylamino-6-oxo-6,7-dihydro-1H-purin-8-yl)-piperidin-3-yl]-carbaminsäure-tert.-butylester
   hergestellt aus Beispiel VI und Methylamingas.
   R_{f}-Wert: 0.27 (Kieselgel, Methylenchlorid/Methanol = 19:1)
(5) [1-(7-Benzyl-2-cyclohexylamino-1-methyl-6-oxo-6,7-dihydro-1H-purin-8-yl)-piperidin-3-yl]-carbaminsäure-tert.-butylester
   hergestellt aus Beispiel VI und Cyclohexylamin
   R_{f}-Wert: 0.65 (Kieselgel, Methylenchlorid/Methanol =9:1)
(6) [1-(7-Benzyl-2-cyclohexylmethylamino-1-methyl-6-oxo-6,7-dihydro-1H-purin-8-yl)-piperidin-3-yl]-carbaminsäure-tert.-butylester
   hergestellt aus Beispiel VI und Cyclohexylmethylamin.
   R_{f}-Wert: 0.54 (Kieselgel, Essigester)
(7) [1-(7-Benzyl-1-methyl-2-piperidino-6-oxo-6,7-dihydro-1H-purin-8-yl)-piperidin-3-yl]-carbaminsäure-tert.-butylester
   hergestellt aus Beispiel VI und Piperidin.
   R_{f}-Wert: 0.45 (Kieselgel, Methylenchlorid/Methanol = 20:1)
(8) [1-(7-Benzyl-2-dimethylamino-1-methyl-6-oxo-6,7-dihydro-1H-purin8-yl)-piperidin-3-yl]-carbaminsäure-tert.-butylester
   hergestellt aus Beispiel VI und Dimethylamin
   R_{f}-Wert: 0.65 (Kieselgel, Methylenchlorid/Methanol = 9:1)
(9) [1-(2-Amino-7-benzyl-1 -methyl-6-oxo-6,7-dihydro-1H-purin-8-yl)-piperidin-3-yl]-carbaminsäure-tert.-butylester
   hergestellt aus Beispiel VI und Ammoniak-Gas.
   R_{f}-Wert: 0.4 (Kieselgel, Methylenchlorid/Methanol =10:1)
(10) [1-(2-Benzylamino-1-methyl-7-[3-methylbut-2-enyl]-6-oxo-6,7-dihydro-1H-purin-8-yl)-piperidino-3-yl]-carbaminsäure-tert.-butylester
   hergestellt aus Beispiel VI.1 und Benzylamin
   R_{f}-Wert: 0.6 (Kieselgel, Essigester)

### Beispiel VIII

### [1-(7-Benzyl-1-methyl-2-methyl-6-oxo-6,7-dihydro-1H-purin-8-yl)-piperidin-3-yl]-carbaminsäure-tert.-butylester

Eine Lösung von 258.32 mg von Beispiel VI in 3 ml THF wird mit 0.2 ml einer 3-molaren Lösung von Methylmagnesiumbromid in Ether versetzt und das Gemisch 48 Stunden bei Raumtemperatur gerührt. Es wurden 50 ml Ether zugegeben und mit Wasser bei pH 4 ausgeschüttelt. Die organische Phase wurde getrocknet und eingedampft. Das erhaltene Produkt wurde über eine Kieselgelsäule gereinigt. Man erhält 55 mg der Titelverbindung.
R_{f}-Wert: 0.55 (Kieselgel, Methylenchlorid/Methanol = 10:1)

Analog Beispiel VIII wurde erhalten:
(1) [1-(1-methyl-7-[3-methyl-but-2-enyl]-2-[2-phenylethyl]-6-oxo-6,7-dihydro-1H-purin-8-yl)-piperidin-3-yl]-carbaminsäure-tert.-butylester
   hergestellt aus Beispiel VI.1 und (2-Phenylethyl)magnesiumbromid.
   R_{f}-Wert: 0.5 (Kieselgel; Methylenchlorid/Methanol = 10:1)

### Beispiel IX

### {1-[7-Benzyl-1-methyl-6-oxo-2-(2-oxo-2-phenyl-ethyl)-6,7-dihydro-1H-purin-8-yl]-piperidin-3-yl}-carbaminsäure-tert.-butylester

Zu einer Lösung aus 0.63 ml n-Butyllithium (1.6 M in n-Hexan) und 119 mg Diisopropylamin in 2 ml Tetrahydrofuran wird bei 0°C eine Lösung aus 132 mg Acetophenon in 1 ml Tetrahydrofuran getropft. Nach 15 Minuten wird eine Lösung aus 500 mg [1-(7-Benzyl-2-methansulfonyl-1-methyl-6-oxo-6,7-dihydro-1H-purin-8-yl)-piperidin-3-yl]-carbaminsäure-tert.-butylester in 2 ml Tetrahydrofuran zugetropft. Anschließend wird das Kühlbad entfernt und das Reaktionsgemisch wird über Nacht bei Raumtemperatur gerührt. Da laut Dünnschichtchromatogramm noch Ausgangsmaterial vorhanden ist, werden nochmals 0.94 ml n-Butyllithium (1.6 M in n-Hexan) zugegeben. Nach weiteren 24 Stunden wird die Reaktionslösung mit 50 ml Wasser verdünnt, mit 2 N Salzsäure auf pH 6 eingestellt und mit Essigester extrahiert. Die vereinigten organischen Phasen werden mit Wasser gewaschen, über Magnesiumsulfat getrocknet und eingeengt. Das Rohprodukt wird chromatographisch über eine Kieselgelsäule mit Methylenchlorid als Laufmittel gereinigt. Man erhält 54 mg der Titelverbindung.
R_{f}-Wert: 0.55 (Aluminiumoxid, Methylenchlorid)
Massenspektrum (ESI⁺): m/z = 557 [M+H]⁺

### Beispiel X

### [1-(7-Benzyl-2-cyano-1-methyl-6-oxo-6,7-dihydro-1H-purin-8-yl)-piperidin-3-yl]-carbaminsäure-tert.-butylester

Zu 258 mg [1-(7-Benzyl-2-methansulfonyl-1-methyl-6-oxo-6,7-dihydro-1H-purin-8-yl)-piperidin-3-yl]-carbaminsäure-tert.-butylester in 1 ml Methylenchlorid werden 268 mg Tetrabutylammoniumcyanid gegeben und das Reaktionsgemisch wird zwei Tage bei Raumtemperatur gerührt. Die Reaktionslösung wird über eine Kieselgelsäule mit Methylenchlorid/Methanol (97:3 auf 90:1) als Laufmittel chromatographiert. Das auf diese Weise erhaltene Produkt wird aus Diisopropylether kristallisiert.
Man erhält 126 mg der Titelverbindung.
R_{f}-Wert: 0.75 (Kieselgel, Methylenchlorid/Methanol =10:1)
Massenspektrum (ESI⁺): m/z = 464 [M+H]⁺

### Beispiel XI

### {1-[1-Benzyl-2-methyl-7-(3-methyl-but-2-enyl)-6-oxo-6,7-dihydro-1H-purin-8-yl]-piperidin-3-yl}-carbaminsäure-tert.-butylester

Ein Gemisch aus 105 mg {1-[5-Methyl-1-(3-methyl-but-2-enyl)-7-oxo-1,7-dihydro-imidazo[4,5-d][1,3]oxazin-2-yl]-piperidin-3-yl}-carbaminsäure-tert.-butylester und 40 µl Benzylamin in 1.5 ml Methylenchlorid wird zwei Tage bei 40°C gerührt. Dann werden 57 µl Triethylamin und 37 µl Phosphoroxychlorid zugegeben und das Reaktionsgemisch wird weitere sechs Stunden bei 40°C gerührt. Zur Aufarbeitung wird das Reaktionsgemisch mit wässriger Kaliumcarbonatlösung versetzt und mit Essigester extrahiert. Die vereinigten organischen Phasen werden über Magnesiumsulfat getrocknet und eingeengt. Das Rohprodukt wird chromatographisch über eine Kieselgelsäule mit Methylenchlorid/Methanol (1:0 auf 20:1) als Laufmittel gereinigt. Man erhält 30 mg der Titelverbindung.
Massenspektrum (ESI⁺): m/z = 507 [M+H]⁺

Analog Beispiel XI werden folgende Verbindungen erhalten:
(1) (1-{1-[(Naphthalin-1-yl)methyl]-2-methyl-7-(3-methyl-but-2-enyl)-6-oxo-6,7-dihydro-1H-purin-8-yl}-piperidin-3-yl)-carbaminsäure-tert.-butylester hergestellt aus Beispiel XII und 1-Aminomethyl-naphthalin. Massenspektrum (ESI⁺): m/z = 557 [M+H]⁺

### Beispiel XII

### {1-[5-Methyl-1-(3-methyl-but-2-enyl)-7-oxo-1,7-dihydro-imidazo[4,5-d][1,3]oxazin-2-yl]-piperidin-3-yl}-carbaminsäure-tert.-butylester

Ein Gemisch aus 1.0 g 5-Acetylamino-2-(3-tert.-butyloxycarbonylamino-piperidin-1-yl)-3-(3-methyl-but-2-enyl)-3H-imidazol-4-carbonsäure-ethylester, 566 mg Triphenylphosphin und 0.97 ml Triethylamin in 16 ml Toluol wird auf 80°c erhitzt und mit einer Lösung aus 702 mg 1,2-Dibromtetrachlorethan in 8 ml Toluol versetzt. Das Reaktionsgemisch wird vier Stunden bei 80°C gerührt, anschließend wird der entstandene Niederschlag abfiltriert und mit Toluol nachgewaschen. Das Filtrat wird im Vakuum eingeengt und über eine Kieselgelsäule mit Cyclohexan/Essigester als Laufmittel chromatographiert. Man erhält 804 mg der Titelverbindung.
Massenspektrum (ESI⁺): m/z = 418 [M+H]⁺

Unter den gleichen Reaktionsbedingungen wird aus Beispiel XIII.1 folgende Verbindung erhalten:
(1) 2-(3-tert.-Butyloxycarbonylamino-piperidin-1-yl)-5-isocyano-3-(3-methyl-but-2-enyl)-3H-imidazol-4-carbonsäure-ethylester
   Massenspektrum (ESI⁺): m/z = 432 [M+H]⁺

### Beispiel XIII

### 5-Acetylamino-2-(3-tert.-butyloxycarbonylamino-piperidin-1-yl)-3-(3-methyl-but-2-enyl)-3H-imidazol-4-carbonsäure-ethylester

hergestellt aus Beispiel I.1 durch Umsetzung mit Acetylchlorid in Gegenwart von Pyridin in Methylenchlorid.
Massenspektrum (ESI⁺): m/z = 464 [M+H]⁺

Analog Beispiel XIII wird folgende Verbindung erhalten:
(1) 2-(3-tert.-Butyloxycarbonylamino-piperidin-1-yl)-5-formylamino-3-(3-methyl-but-2-enyl)-3H-imidazol-4-carbonsäure-ethylester
   hergestellt aus Beispiel I.1 durch Umsetzung mit Ameisensäure und Acetanhydrid in Gegenwart von Pyridin in Methylenchlorid.
   Massenspektrum (ESI⁺): m/z = 450 [M+H]⁺

### Beispiel XIV

### (1-{1-[(Naghthalin-1-yl)methyl]-7-(3-methyl-but-2-enyl)-6-oxo-6,7-dihydro-1H-purin-8-yl}piperidin-3-yl)-carbaminsäure-tert-butylester

Zu 295 mg 2-(3-tert.-Butyloxycarbonylamino-piperidin-1-yl)-5-isocyano-3-(3-methyl-but-2-enyl)-3H-imidazol-4-carbonsäure-ethylester in 7 ml Toluol werden 210 mg 1-Aminomethyl-naphthalin und 30 mg Kuper(I)oxid gegeben. Das Reaktionsgemisch wird zehn Stunden bei 120°C gerührt. Nach Abkühlung auf Raumtemperatur wird es mit Essigester verdünnt und über Celite filtriert. Das Filtrat wird mit Wasser versetzt und mit Essigester extrahiert. Die vereinigten Extrakte werden über Magnesiumsulfat getrocknet und eingeengt. Das Rohprodukt wird über eine Kieselgelsäule mit Methylenchlorid/Methanol (1:0 auf 10:1) als Laufmittel chromatographiert. Man erhält 306 mg der Titelverbindung, die mit 5-Amino-2-(3-tert.-butoxycarbonylaminopiperidin-1-yl)-3-(3-methyl-but-2-enyl)-3H-imidazol-4-carbonsäure-ethylester verunreinigt ist.
Massenspektrum (ESI⁺): m/z = 543 [M+H]⁺

Analog Beispiel XIV wird folgende Verbindung erhalten:
(1) (1-{1-[(4-Methoxy-naphthalin-1-yl)methyl]-7-(3-methyl-but-2-enyl)-6-oxo-6,7-dihydro-1H-purin-8-yl}-piperidin-3-yl)-carbaminsäure-tert.-butylester hergestellt aus Beispiel XII.1 und 1-Aminomethyl-4-methoxy-naphthalin.
   R_{f}-Wert: 0.17 (Kieselgel, Cyclohexan/Essigester = 1:9)
   Massenspektrum (ESI⁺): m/z = 573 [M+H]⁺

### Beispiele zur Herstellung der Endprodukte

### Beispiel 1

### 8-(3-Amino-piloeridin-1-yl)-7-benzyl-2-benzylamino-1-methyl-1.7-dihydro-purin-6-on

Eine Lösung von 200 mg von Beispiel VII in 2 ml Dichlormethan wird unter Rühren und Eiskühlung mit 3 ml Trifluoressigsäure versetzt. Nach 30 Minuten wird das Eisbad entfernt und 2 Stunden weitergerührt. Das Lösungsmittel wird bei niedriger Temperatur verdampft, der Rückstand mit Ether verrieben, abgesaugt und im Vakuum getrocknet. Man erhält 150 mg (73.1 % d.Th.) des Trifluoracetats der Titelverbindung.
R_{f}-Wert: 0.45 (Aluminiumoxid, Methylenchlorid/Methanol = 20:1)
¹H-NMR-Spektrum (400 MHz, DMSO-d₆):
1.5 (m,2H), 1,7 (m,1 H), 1.95 (m, 1 H), 2.8 (m,1 H), 3.0 (m, 1 H), 3.15 (d,1 H), 3.4 (s+m,4H), 3.5(d,1H), 4.55 (d,2H), 5.35(s,2H), 7.1-7.5 (m,12H), 8.0 (s,3H), 8.1-8.3 (m,1 H)

Analog Beispiel 1 wurden die folgenden Verbindungen erhalten:
(1) 8-(3-Amino-piperidin-1-yl)-7-benzyl-2-(4-fluor-benzylamino)-1-methyl-1,7-dihydro-purin-6-on Trifluoracetat
   hergestellt aus Beispiel VII.1 und Trifluoressigsäure
   R_{f}-Wert: 0.69 (Aluminiumoxid, Methylenchlorid/Methanol = 9:1)
(2) 8-(3-Amino-piperidin-1-yl)-7 -benzyl-1-methyl-2-[(2-phenylethyl)amino]-1,7-dihydro-purin-6-on Trifluoracetat
   hergestellt aus Beispiel VII.2 und Trifluoressigsäure.
   R_{f}-Wert: 0.75 (Aluminiumoxid, Methylenchlorid/Methanol = 9:1)
(3) 8-(3-Amino-piperidin-1-yl)-7-benzyl-2-isopropylamino-1-methyl-1,7-dihydropurin-6-on Trifluoracetat
   hergestellt aus Beispiel VII.3 und Trifluoressigsäure.
   R_{f}-Wert: 0.68 (Aluminiumoxid, Methylenchlorid/Methanol = 9:1)
(4) 8-(3-Amino-piperidin-1-yl)-7-benzyl-1-methyl-2-methylamino-1,7-dihydro-purin-6-on Trifluoracetat
   hergestellt aus Beispiel VII.4 und Trifluoressigsäure.
   R_{f}-Wert: 0.26 (Aluminiumoxid, Methylenchlorid/Methanol = 9:1)
(5) 8-(3-Amino-piperidin-1-yl)-7-benzyl-2-cyclohexylamino-1-methyl-1,7-dihydro-purin-6-on Trifluoracetat
   hergestellt aus Beispiel VII.5 und Trifluoressigsäure.
   R_{f}-Wert: 0.65 (Aluminiumoxid, Methylenchlorid/Methanol = 9:1)
(6) 8-(3-Amino-piperidin-1-yl)-7-benzyl-2-cyclohexylmethylamino-1-methyl-1,7-dihydro-purin-6-on Trifluoracetat
   hergestellt aus Beispiel VII.6 und Trifluoressigsäure.
   R_{f}-Wert: 0.69 (Aluminiumoxid, Methylenchlorid/Methanol =9:1)
(7) 8-(3-Amino-piperidin-1-yl)-7-benzyl-1-methyl-2-(piperidin-1-yl)-1,7-dihydro-purin-6-on Hydrochlorid
   hergestellt aus Beispiel VII.7 und Chlorwasserstoff in Dioxan.
   R_{f}-Wert: 0.3-0.5 (Aluminiumoxid, Methylenchlorid/Methanol = 20:1)
(8) 8-(3-Amino-piperidin-1-yl)-7-benzyl-2-dimethylamino-1-methyl-1,7-dihydro-purin-6-on Trifluoracetat
   hergestellt aus Beispiel VII.8 und Trifluoressigsäure.
   R_{f}-Wert: 0.69 (Aluminiumoxid, Methylenchlorid/Methanol =9:1)
(9) 2-Amino-8-(3-amino-piperidin-1-yl)-7-benzyl-1-methyl-1,7-dihydro-purin-6-on Trifluoracetat
   hergestellt aus Beispiel VII.9 und Trifluoressigsäure.
   R_{f}-Wert: 0.2 (Aluminiumoxid, Methylenchlorid/Methanol =10:1)
(10) 8-(3-Amino-piperidin-1-yl)-2-benzylamino-1-methyl-7-(3-methyl-but-2-en-1-yl)-1,7-dihydro-purin-6-on Trifluoracetat
   hergestellt aus Beispiel VII.10 und Trifluoressigsäure.
   R_{f}-Wert: 0.55 (Aluminiumoxid, Methylenchlorid/Methanol = 10:1)
(11) 8-(3-Amino-piperidin-1-yl)-7-benzyl-1-methyl-2-methyl-1,7-dihydro-purin-6-on Trifluoracetat
   hergestellt aus Beispiel VIII und Trifluoressigsäure.
   R_{f}-Wert: 0.6 (Aluminiumoxid, Methylenchlorid/Methanol = 10:1)
(12) 8-(3-Amino-piperidin-1-yl)-1-methyl-7-(3-methyl-but-2-en-1-yl)-2-(2-phenylethyl)-1,7-dihydro-purin-6-on Trifluoracetat
   hergestellt aus Beispiel VIII.1 und Trifluoressigsäure.
   R_{f}-Wert: 0.4 (Aluminiumoxid, Methylenchlorid/Methanol = 20:1)
(13) 8-(3-Amino-piperidin-1-yl)-7-benzyl-1-methyl-1,7-dihydro-purin-6-on Hydrochlorid
   hergestellt aus Beispiel III und Chlorwasserstoff in Dioxan.
   R_{f}-Wert: 0.2-0.5 (Aluminiumoxid, Methylenchlorid/Methanol = 20:1)
(14) 8-(3-Amino-piperidin-1-yl)-7-benzyl-2-methylsulfanyl-1,7-dihydro-purin-6-on Hydrochlorid
   hergestellt aus Beispiel IV und Chlorwasserstoff in Dioxan.
   R_{f}-Wert: 0.5 (Aluminiumoxid, Methylenchlorid/Methanol = 10:1)
(15) 8-(3-Amino-piperidin-1-yl)-7-benzyl-2-benzylsulfanyl-1,7-dihydro-purin-6-on Trifluoracetat
   hergestellt aus Beispiel IV.1 und Trifluoressigsäure.
   R_{f}-Wert: 0.5-0.6 (Aluminiumoxid, Methylenchlorid/Methanol = 10:1)
(16) 8-(3-Amino-piperidin-1-yl)-7-benzyl-1-methyl-2-methylsulfanyl-1,7-dihydro-purin-6-on Hydrochlorid
   hergestellt aus Beispiel V und Chlorwasserstoff in Dioxan.
   R_{f}-Wert: 0.5 (Aluminiumoxid, Methylenchlorid/Methanol = 20:1)
(17) 8-(3-Amino-piperidin-1-yl)-7-benzyl-2-methylsulfanyl-1-(2-phenyl-2-oxo-ethyl)-1,7-dihydro-purin-6-on Hydrochlorid
   hergestellt aus Beispiel V.1 und Chlorwasserstoff in Dioxan.
   R_{f}-Wert: 0.4 (Aluminiumoxid, Methylenchlorid/Methanol =20:1)
(18) 8-(3-Amino-piperidin-1-yl)-1-benzyl-7-benzyl-2-methylsulfanyl-1,7-dihydro-purin-6-on Hydrochlorid
   hergestellt aus Beispiel V.3 und Chlorwasserstoff.
   R_{f}-Wert: 0.4-0.5 (Aluminiumoxid, Methylenchlorid/Methanol = 20:1)
(19) 8-(3-Amino-piperidin-1-yl)-7-benzyl-2-methylsulfanyl-1-(2-phenylethyl)-1,7-dihydro-purin-6-on Hydrochlorid
   hergestellt aus Beispiel V.4 und Chlorwasserstoff.
   R_{f}-Wert: 0.4 (Aluminiumoxid, Methylenchlorid/Methanol = 20:1)
(20) 8-(3-Amino-piperidin-1-yl)-7-benzyl-2-benzylsulfanyl-1-methyl-1,7-dihydro-purin-6-on
   hergestellt aus Beispiel V.5 und Chlorwasserstoff.
   R_{f}-Wert: 0.5 (Aluminiumoxid, Methylenchlorid/Methanol = 20:1)
(21) 8-(3-Amino-piperidin-1-yl)-7-benzyl-2-[(2-phenylethyl)sulfanyl]-1-methyl-1,7-dihydro-purin-6-on
   hergestellt aus Beispiel V.6 und Chlorwasserstoff.
   R_{f}-Wert: 0.55 (Alumiumoxid, Methylenchlorid/Methanol =20:1)
(22) 8-(3-Amino-piperidin-1-yl)-7-benzyl-1-(2-oxo-2-phenyl-ethyl)-1,7-dihydro-purin-6-on Trifluoracetat
   hergestellt aus Beispiel III.1 und Trifluoressigsäure.
   R_{f}-Wert: 0.65 (Aluminiumoxid, Methylenchlorid/Methanol = 10:1)
(23) 8-(3-Amino-piperidin-1-yl)-7-benzyl-1-methyl-2-(2-oxo-2-phenyl-ethyl)-1,7-dihydro-purin-6-on
   hergestellt aus Beispiel IX und Trifluoressigsäure.
   R_{f}-Wert: 0.20 (Kieselgel, Methylenchlorid/Methanol = 10:1)
   Massenspektrum (ESI⁺): m/z = 457 [M+H]⁺
(24) 8-(3-Amino-piperidin-1-yl)-7-benzyl-2-cyano-1-methyl-1,7-dihydro-purin-6-on Trifluoracetat
   hergestellt aus Beispiel X und Trifluoressigsäure.
   R_{f}-Wert: 0.50 (Aluminiumoxid, Methylenchlorid/Methanol = 20:1)
   Massenspektrum (ESI⁺): m/z = 364 [M+H]⁺
(25) 8-(3-Amino-piperidin-1-yl)-1-benzyl-2-methyl-7-(3-methyl-but-2-en-1-yl)-1,7-dihydro-purin-6-on
   hergestellt aus Beispiel XI und Trifluoressigsäure.
   Massenspektrum (ESI⁺): m/z = 407 [M+H]⁺
(26) 8-(3-Amino-piperidin-1-yl)-2-methyl-7-(3-methyl-but-2-en-1-yl)-1-[(naphthalin-1-yl)methyl]-1,7-dihydro-purin-6-on
   hergestellt aus Beispiel XI.1 und Trifluoressigsäure.
   Massenspektrum (ESI⁺): m/z = 457 [M+H]⁺
(27) 8-(3-Amino-piperidin-1-yl)-7-(3-methyl-but-2-en-1-yl)-1-[(naphthalin-1-yl)methyl]-1,7-dihydro-purin-6-on
   hergestellt aus Beispiel XIV und Trifluoressigsäure.
   Massenspektrum (ESI⁺): m/z = 443 [M+H]⁺
(28) 8-(3-Amino-piperidin-1-yl)-7-(3-methyl)-but-2-en-1-yl)-1-[(4-methoxy-naphthalin-1-yl)methyl]-1,7-dihydro-purin-6-on
   hergestellt aus Beispiel XIV.1 und Trifluoressigsäure.
   Massenspektrum (ESI⁺): m/z = 473 [M+H]⁺

Analog den vorstehenden Beispielen und anderer literaturbekannter Verfahren können auch die folgenden Verbindungen erhalten werden:
(1) 8-(3-Amino-piperidin-1-yl)-7-benzyl-1-ethyl-2-benzylamino-1,7-dihydro-purin-6-on
(2) 8-(3-Amino-piperidin-1-yl)-7-benzyl-1-propyl-2-benzylamino-1,7-dihydro-purin-6-on
(3) 8-(3-Amino-piperidin-1-yl)-7-benzyl-1-isopropyl-2-benzylamino-1,7-dihydro-purin-6-on
(4) 8-(3-Amino-piperidin-1-yl)-7-benzyl-1-butyl-2-benzylamino-1,7-dihydro-purin-6-on
(5) 8-(3-Amino-piperidin-1-yl)-7-benzyl-1-(2-methyl-propyl)-2-benzylamino-1,7 dihydro-purin-6-on
(6) 8-(3-Amino-piperidin-1-yl)-7-benzyl-1-pentyl-2-benzylamino-1,7-dihydro-purin-6-on
(7) 8-(3-Amino-piperidin-1-yl)-7-benzyl-1-(3-methyl-butyl)-2-benzylamino-1,7-dihydro-purin-6-on
(8) 8-(3-Amino-piperidin-1-yl)-7-benzyl-1-hexyl-2-benzylamino-1,7-dihydro-purin-6-on
(9) 8-(3-Amino-piperidin-1-yl)-7-benzyl-1-(4-methyl-pentyl)-2-benzylamino-1,7-dihydro-purin-6-on
(10) 8-(3-Amino-piperidin-1-yl)-7-benzyl-1-allyl-2-benzylamino-1,7-dihydro-purin-6-on
(11) 8-(3-Amino-piperidin-1-yl)-7-benzyl-1-(3-methyl-but-2-en-1-yl)-2-benzylamino-1,7-dihydro-purin-6-on
(12) 8-(3-Amino-piperidin-1-yl)-7-benzyl-1-(prop-2-in-1-yl)-2-benzylamino-1,7-dihydro-purin-6-on
(13) 8-(3-Amino-piperidin-1-yl)-7-benzyl-1-cyclopropylmethyl-2-benzylamino-1,7-dihydro-purin-6-on
(14) 8-(3-Amino-piperidin-1-yl)-7-benzyl-1-cyclohexylmethyl-2-benzylamino-1,7-dihydro-purin-6-on
(15) 8-(3-Amino-piperidin-1-yl)-7-benzyl-1-(4-chlorbenzyl)-2-benzylamino-1,7-dihydro-purin-6-on
(16) 8-(3-Amino-piperidin-1-yl)-7-benz-yl-1-(2-fluorbenzyl)-2-benzylamino-1,7-dihydro-purin-6-on
(17) 8-(3-Amino-piperidin-1-yl)-7-benzyl-1-(4-methylbenzyl)-2-benzylamino-1,7-dihydro-purin-6-on
(18) 8-(3-Amino-piperidin-1-yl)-7-benzyl-1-(2,4-dimethylbenzyl)-2-benzylamino-1,7-dihydro-purin-6-on
(19) 8-(3-Amino-piperidin-1-yl)-7-benzyl-1-(2,4-dimethoxybenzyl)-2-benzylamino-1,7-dihydro-purin-6-on
(20) 8-(3-Amino-piperidin-1-yl)-7-benzyl-1-(3-trifluormethyl-benzyl)-2-benzylamino-1,7-dihydro-purin-6-on
(21) 8-(3-Amino-piperidin-1-yl)-7-benzyl-1-(3-trifluormethoxy-benzyl)-2-benzylamino-1,7-dihydro-purin-6-on
(22) 8-(3-Amino-piperidin-1-yl)-7-benzyl-1-(3-difluormethoxy-benzyl)-2-benzylamino-1,7-dihydro-purin-6-on
(23) 8-(3-Amino-piperidin-1-yl)-7-benzyl-1-(furan-2-yl-methyl)-2-benzylamino-1,7-dihydro-purin-6-on
(24) 8-(3-Amino-piperidin-1-yl)-7-benzyl-1-(thien-2-yl-methyl)-2-benzylamino-1,7-dihydro-purin-6-on
(25) 8-(3-Amino-piperidin-1-yl)-7-benzyl-1-(3-methyl-isoxazol-5-yl-methyl)-2-benzylamino-1,7-dihydro-purin-6-on
(26) 8-(3-Amino-piperidin-1-yl)-7-benzyl-1-(pyridin-4-yl-methyl)-2-benzylamino-1,7-dihydro-purin-6-on
(27) 8-(3-Amino-piperidin-1-yl)-7-benzyl-1-[2-(4-methyl-phenyl)-2-oxo-ethyl]-2-benzylamino-1,7-dihydro-purin-6-on
(28) 8-(3-Amino-piperidin-1-yl)-7-benzyl-1-[2-(2,4-dichlor-phenyl)-2-oxo-ethyl]-2-benzylamino-1,7-dihydro-purin-6-on
(29) 8-(3-Amino-piperidin-1-yl)-7-benzyl-1-[2-(4-methoxy-phenyl)-2-oxo-ethyl]-2-benzylamino-1,7-dihydro-purin-6-on
(30) 8-(3-Amino-piperidin-1-yl)-7-benzyl-1-[2-(4-trifluormethyl-phenyl)-2-oxo-ethyl]-2-benzylamino-1,7-dihydro-purin-6-on
(31) 8-(3-Amino-piperidin-1-yl)-7-benzyl-1-[2-(4-trifluormethoxy-phenyl)-2-oxo-ethyl]-2-benzylamino-1,7-dihydro-purin-6-on
(32) 8-(3-Amino-piperidin-1-yl)-7-benzyl-1-[2-(4-difluormethoxy-phenyl)-2-oxo-ethyl]-2-benzylamino-1,7-dihydro-purin-6-on
(33) 8-(3-Amino-piperidin-1-yl)-7-benzyl-1-[2-(furan-2-yl)-2-oxo-ethyl]-2-benzylamino-1,7-dihydro-purin-6-on
(34) 8-(3-Amino-piperidin-1-yl)-7-benzyl-1-[2-(thien-2-yl)-2-oxo-ethyl]-2-benzylamino-1,7-dihydro-purin-6-on
(35) 8-(3-Amino-piperidin-1-yl)-7-benzyl-1-[2-(pyridin-3-yl)-2-oxo-ethyl]-2-benzylamino-1,7-dihydro-purin-6-on
(36) 8-(3-Amino-piperidin-1-yl)-7-benzyl-1-(2-oxopropyl)-2-benzylamino-1,7-dihydro-purin-6-on
(37) 8-(3-Amino-piperidin-1-yl)-7-benzyl-1-(2-cyclohexyl-2-oxo-ethyl)-2-benzylamino-1-,7-dihydro-purin-6-on
(38) 8-(3-Amino-piperidin-1-yl)-7-benzyl-1-ethyl-2-(2-phenylethyl)-1,7-dihydro-purin-6-on
(39) 8-(3-Amino-piperidin-1-yl)-7-benzyl-1-propyl-2-(2-phenylethyl)-1,7-dihydro-purin-6-on
(40) 8-(3-Amino-piperidin-1-yl)-7-benzyl-1-isopropyl-2-(2-phenylethyl)-1,7-dihydro-purin-6-on
(41) 8-(3-Amino-piperidin-1-yl)-7-benzyl-1-butyl-2-(2-phenylethyl)-1,7-dihydro-purin-6-on
(42) 8-(3-Amino-piperidin-1-yl)-7-benzyl-1-(2-methyl-propyl)-2-(2-phenylethyl)-1,7-dihydro-purin-6-on
(43) 8-(3-Amino-piperidin-1-yl)-7-benzyl-1-pentyl-2-(2-phenylethyl)-1,7-dihydro-purin-6-on
(44) 8-(3-Amino-piperidin-1-yl)-7-benzyl-1-(3-methyl-butyl)-2-(2-phenylethyl)-1,7-dihydro-purin-6-on
(45) 8-(3-Amino-piperidin-1-yl)-7-benzyl-1-hexyl-2-(2-phenylethyl)-1,7-dihydro-purin-6-on
(46) 8-(3-Amino-piperidin-1-yl)-7-benzyl-1-(4-methyl-pentyl)-2-(2-phenylethyl)-1,7-dihydro-purin-6-on
(47) 8-(3-Amino-piperidin-1-yl)-7-benzyl-1-allyl-2-(2-phenylethyl)-1,7-dihydro-purin-6-on
(48) 8-(3-Amino-piperidin-1-yl)-7-benzyl-1-(3-methyl-but-2-en-1-yl)-2-(2-phenylethyl)-1,7-dihydro-purin-6-on
(49) 8-(3-Amino-piperidin-1-yl)-7-benzyl-1-(prop-2-in-1-yl)-2-(2-phenylethyl)-1,7-dihydro-purin-6-on
(50) 8-(3-Amino-piperidin-1-yl)-7-benzyl-1-cyclopropylmethyl-2-(2-phenylethyl)-1,7-dihydro-purin-6-on
(51) 8-(3-Amino-piperidin-1-yl)-7-benzyl-1-cyclohexylmethyl-2-(2-phenylethyl)-1,7-dihydro-purin-6-on
(52) 8-(3-Amino-piperidin-1-yl)-7-benzyl-1-(4-chlorbenzyl)-2-(2-phenylethyl)-1,7-dihydro-purin-6-on
(53) 8-(3-Amino-piperidin-1-yl)-7-benzyl-1-(2-fluorbenzyl)-2-(2-phenylethyl)-1,7-dihydro-purin-6-on
(54) 8-(3-Amino-piperidin-1-yl)-7-benzyl-1-(4-methylbenzyl)-2-(2-phenylethyl)-1,7-dihydro-purin-6-on
(55) 8-(3-Amino-piperidin-1-yl)-7-benzyl-1-(2,4-dimethylbenzyl)-2-(2-phenylethyl)-1,7-dihydro-purin-6-on
(56) 8-(3-Amino-piperidin-1-yl)-7-benzyl-1-(2,4-dimethoxybenzyl)-2-(2-phenylethyl)-1,7-dihydro-purin-6-on
(57) 8-(3-Amino-piperidin-1-yl)-7-benzyl-1-(3-trifluormethyl-benzyl)-2-(2-phenylethyl)-1,7-dihydro-purin-6-on
(58) 8-(3-Amino-piperidin-1-yl)-7-benzyl-1-(3-trifluormethoxy-benzyl)-2-(2-phenylethyl)-1,7-dihydro-purin-6-on
(59) 8-(3-Amino-piperidin-1-yl)-7-benzyl-1-(3-difluormethoxy-benzyl)-2-(2-phenylethyl)-1,7-dihydro-purin-6-on
(60) 8-(3-Amino-piperidin-1-yl)-7-benzyl-1-(furan-2-yl-methyl)-2-(2-phenylethyl)-7-dihydro-purin-6-on
(61) 8-(3-Amino-piperidin-1-yl)-7-benzyl-1-(thien-2-yl-methyl)-2-(2-phenylethyl)-1,7-dihydro-purin-6-on
(62) 8-(3-Amino-piperidin-1-yl)-7-benzyl-1-(3-methyl-isoxazol-5-yl-methyl)-2-(2-phenylethyl)-1,7-dihydro-purin-6-on
(63) 8-(3-Amino-piperidin-1-yl)-7-benzyl-1-(pyridin-4-yl-methyl)-2-(2-phenylethyl)-1,7-dihydro-purin-6-on
(64) 8-(3-Amino-piperidin-1-yl)-7-benzyl-1-[2-(4-methyl-phenyl)-2-oxo-ethyl]-2-(2-phenylethyl)-1,7-dihydro-purin-6-on
(65) 8-(3-Amino-piperidin-1-yl)-7-benzyl-1-[2-(2,4-dichlor-phenyl)-2-oxo-ethyl]-2-(2-phenylethyl)-1,7-dihydro-purin-6-on
(66) 8-(3-Amino-piperidin-1-yl)-7-benzyl-1-[2-(4-methoxy-phenyl)-2-oxo-ethyl]-2-(2-phenylethyl)-1,7-dihydro-purin-6-on
(67) 8-(3-Amino-piperidin-1-yl)-7 -benzyl-1-[2-(4-trifluormethyl-phenyl)-2-oxo-ethyl]-2-(2-phenylethyl)-1,7-dihydro-purin-6-on
(68) 8-(3-Amino-piperidin-1-yl)-7-benzyl-1-[2-(4-trifluormethoxy-phenyl)-2-oxo-ethyl]-2-(2-phenylethyl)-1,7-dihydro-purin-6-on
(69) 8-(3-Amino-piperidin-1-yl)-7-benzyl-1-[2-(4-difluormethoxy-phenyl)-2-oxo-ethyl]-2-(2-phenylethyl)-1,7-dihydro-purin-6-on
(70) 8-(3-Amino-piperidin-1-yl)-7-benzyl-1-[2-(furan-2-yl)-2-oxo-ethyl]-2-(2-phenylethyl)-1,7-dihydro-purin-6-on
(71) 8-(3-Amino-piperidin-1-yl)-7-benzyl-1-[2-(thien-2-yl)-2-oxo-ethyl]-2-(2-phenylethyl)-1,7-dihydro-purin-6-on
(72) 8-(3-Amino-piperidin-1-yl)-7-benzyl-1-[2-(pyridin-3-yl)-2-oxo-ethyl]-2-(2-phenylethyl)-1,7-dihydro-purin-6-on
(73) 8-(3-Amino-piperidin-1-yl)-7-benzyl-1-(2-oxo-propyl)-2-(2-phenylethyl)-1,7-dihydro-purin-6-on
(74) 8-(3-Amino-piperidin-1-yl)-7-benzyl-1-(2-cyclohexyl-2-oxo-ethyl)-2-(2-phenylethyl)-1,7-dihydro-purin-6-on
(75) 8-(3-Amino-piperidin-1-yl)-7-benzyl-1-methyl-2-ethylamino-1,7-dihydro-purin-6-on
(76) 8-(3-Amino-piperidin-1-yl)-7-benzyl-1-methyl-2-propylamino-1,7-dihydro-purin-6-on
(77) 8-(3-Amino-piperidin-1-yl)-7-benzyl-1-methyl-2-butylamino-1,7-dihydro-purin-6-on
(78) 8-(3-Amino-piperidin-1-yl)-7-benzyl-1-methyl-2-(2-methyl-propylamino)-1,7-dihydro-purin-6-on
(79) 8-(3-Amino-piperidin-1-yl)-7-benzyl-1-methyl-2-(N-ethyl-N-methyl-amino)-1,7-dihydro-purin-6-on
(80) 8-(3-Amino-piperidin-1-yl)-7-benzyl-1-methyl-2-diethylamino-1,7-dihydro-purin-6-on
(81) 8-(3-Amino-piperidin-1-yl)-7-benzyl-1-methyl-2-(N-methyl-N-propyl-amino)-1,7-dihydro-purin-6-on
(82) 8-(3-Amino-piperidin-1-yl)-7-benzyl-1-methyl-2-(N-butyl-N-methyl-amino)-1,7-dihydro-purin-6-on
(83) 8-(3-Amino-piperidin-1-yl)-7-benzyl-1-methyl-2-(2-hydroxyethyl-amino)-1,7-dihydro-purin-6-on
(84) 8-(3-Amino-piperidin-1-yl)-7-benzyl-1-methyl-2-[N-methyl-N-(2-hydroxyethyl)-amino]-1,7-dihydro-purin-6-on
(85) 8-(3-Amino-piperidin-1-yl)-7-benzyl-1-methyl-2-(2-methoxy-ethylamino)-1,7-dihydro-purin-6-on
(86) 8-(3-Amino-piperidin-1-yl)-7-benzyl-1-methyl-2-(3-methoxy-propylamino)-1,7-dihydro-purin-6-on
(87) 8-(3-Amino-piperidin-1-yl)-7-benzyl-1-methyl-2-[N-methyl-N-(2-methoxy-ethyl)-amino]-1,7-dihydro-purin-6-on
(88) 8-(3-Amino-piperidin-1-yl)-7-benzyl-1-methyl-2-[(2-phenoxyethyl)-amino]-1,7-dihydro-purin-6-on
(89) 8-(3-Amino-piperidin-1-yl)-7-benzyl-1-methyl-2-[(2-aminoethyl)-amino]-1,7-dihydro-purin-6-on
(90) 8-(3-Amino-piperidin-1-yl)-7-benzyl-1-methyl-2-[(3-aminopropyl)-amino]-1,7-dihydro-purin-6-on
(91) 8-(3-Amino-piperidin-1-yl)-7-benzyl-1-methyl-2-[(2-methylamino-ethyl)-amino]-1,7-dihydro-purin-6-on
(92) 8-(3-Amino-piperidin-1-yl)-7-benzyl-1-methyl-2-[(3-dimethylamino-propyl)amino]-1,7-dihydro-purin-6-on
(93) 8-(3-Amino-piperidin-1-yl)-7-benzyl-1-methyl-2-[(2-acetylamino-ethyl)amino]-1,7-dihydro-purin-6-on
(94) 8-(3-Amino-piperidin-1-yl)-7-benzyl-1-methyl-2-[(2-benzoylamino-ethyl)amino]-1,7-dihydro-purin-6-on
(95) 8-(3-Amino-piperidin-1-yl)-7-benzyl-1-methyl-2-[(2-ethoxycarbonylaminoethyl)amino]-1,7-dihydro-purin-6-on
(96) 8-(3-Amino-piperidin-1-yl)-7-benzyl-1-methyl-2-[(2-methylsulfanyl-ethyl)amino]-1,7-dihydro-purin-6-on
(97) 8-(3-Amino-piperidin-1-yl)-7-benzyl-1-methyl-2-[(2-phenylsulfanyl-ethyl)amino]-1,7-dihydro-purin-6-on
(98) 8-(3-Amino-piperidin-1-yl)-7-benzyl-1-methyl-2-[(2-cyan-ethyl)amino]-1,7-dihydro-purin-6-on
(99) 8-(3-Amino-piperidin-1-yl)-7-benzyl-1-methyl-2-[(carboxymethyl)amino]-1,7-dihydro-purin-6-on
(100) 8-(3-Amino-piperidin-1-yl)-7-benzyl-1-methyl-2-[(ethoxycarbonylmethyl)amino]-1,7-dihydro-purin-6-on
(101) 8-(3-Amino-piperidin-1-yl)-7-benzyl-1-methyl-2-[(aminocarbonylmethyl)amino]-1,7-dihydro-purin-6-on
(102) 8-(3-Amino-piperidin-1-yl)-7-benzyl-1-methyl-2-[(ethylaminocarbonylmethyl)-amino]-1,7-dihydro-purin-6-on
(103) 8-(3-Amino-piperidin-1-yl)-7-benzyl-1-methyl-2-[(dimethylaminocarbonylmethyl)amino]-1,7-dihydro-purin-6-on
(104) 8-(3-Amino-piperidin-1-yl)-7-benzyl-1-methyl-2-[(pyrrolidin-1-yl-carbonylmethyl)amino]-1,7-dihydro-purin-6-on
(105) 8-(3-Amino-piperidin-1-yl)-7-benzyl-1-methyl-2-[(piperidin-1-yl-carbonylmethyl)amino]-1,7-dihydro-purin-6-on
(106) 8-(3-Amino-piperidin-1-yl)-7-benzyl-1-methyl-2-[(morpholin-4-ylcarbonylmethyl)amino]-1,7-dihydro-purin-6-on
(107) 8-(3-Amino-piperidin-1-yl)-7-benzyl-1-methyl-2-[(cyclopentylmethyl)amino]-1,7-dihydro-purin-6-on
(108) 8-(3-Amino-piperidin-1-yl)-7-benzyl-1-methyl-2-[(cyclobutylmethyl)amino]-1,7-dihydro-purin-6-on
(109) 8-(3-Amino-piperidin-1-yl)-7-benzyl-1-methyl-2-[(cyclopropylmethyl)amino]-1,7-dihydro-purin-6-on
(110) 8-(3-Amino-piperidin-1-yl)-7-benzyl-1-methyl-2-[(2-cyclohexyl-ethyl)amino]-1,7-dihydro-purin-6-on
(111) 8-(3-Amino-piperidin-1-yl)-7-benzyl-1-methyl-2-[(3-cyclohexyl-propyl)amino]-1,7-dihydro-purin-6-on
(112) 8-(3-Amino-piperidin-1-yl)-7-benzyl-1-methyl-2-(cyclobutylamino)-1,7-dihydro-purin-6-on
(113) 8-(3-Amino-piperidin-1-yl)-7-benzyl-1-methyl-2-(cyclopentylamino)-1,7-dihydro-purin-6-on
(114) 8-(3-Amino-piperidin-1-yl)-7-benzyl-1-methyl-2-(phenylamino)-1,7-dihydro-purin-6-on
(115) 8-(3-Amino-piperidin-1-yl)-7-benzyl-1-methyl-2-(3,4-dichlorbenzyl-amino)-1,7-dihydro-purin-6-on
(116) 8-(3-Amino-piperidin-1-yl)-7-benzyl-1-methyl-2-(4-methylbenzyl-amino)-1,7-dihydro-purin-6-on
(117) 8-(3-Amino-piperidin-1-yl)-7-benzyl-1-methyl-2-(4-methoxybenzyl-amino)-1,7-dihydro-purin-6-on
(118) 8-(3-Amino-piperidin-1-yl)-7-benzyl-1-methyl-2-(4-trifluormethylbenzyl-amino)-1,7-dihydro-purin-6-on
(119) 8-(3-Amino-piperidin-1-yl)-7-benzyl-1-methyl-2-(4-trifluormethoxybenzyl-amino)-1,7-dihydro-purin-6-on
(120) 8-(3-Amino-piperidin-1-yl)-7-benzyl-1-methyl-2-(4-difluormethoxybenzyl-amino)-1,7-dihydro-purin-6-on
(121) 8-(3-Amino-piperidin-1-yl)-7-benzyl-1-methyl-2-(3,4-methylendioxybenzyl-amino)-1,7-dihydro-purin-6-on
(122) 8-(3-Amino-piperidin-1-yl)-7-benzyl-1-methyl-2-[(furan-2-yl-methyl)amino]-1,7-dihydro-purin-6-on
(123) 8-(3-Amino-piperidin-1-yl)-7-benzyl-1-methyl-2-[(thien-2-yl-methyl)amino]-1,7-dihydro-purin-6-on
(124) 8-(3-Amino-piperidin-1-yl)-7-benzyl-1-methyl-2-[(pyridin-2-yl-methyl)amino]-1,7-dihydro-purin-6-on
(125) 8-(3-Amino-piperidin-1-yl)-7-benzyl-1-methyl-2-[(4-methylthiazol-2-ylmethyl)-amino]-1,7-dihydro-purin-6-on
(126) 8-(3-Amino-piperidin-1-yl)-7-benzyl-1-methyl-2-{[2-(pyridin-2-yl)ethyl]amino}-1,7-dihydro-purin-6-on
(127) 8-(3-Amino-piperidin-1 -yl)-7-benzyl-1 -methyl-2-(pyrrolidin-1 -yl)-1,7-dihydro-purin-6-on
(128) 8-(3-Amino-piperidin-1-yl)-7-benzyl-1-methyl-2-(morpholin-4-yl)-1,7-dihydro-purin-6-on
(129) 8-(3-Amino-piperidin-1-yl)-7-benzyl-1-methyl-2-(piperazin-1 -yl)-1,7-dihydro-purin-6-on
(130) 8-(3-Amino-piperidin-1-yl)-7-benzyl-1-methyl-2-(4-methyl-piperazin-1-yl)-1,7-dihydro-purin-6-on
(131) 8-(3-Amino-piperidin-1-yl)-7-benzyl-1-methyl-2-ethyl-1,7-dihydro-purin-6-on
(132) 8-(3-Amino-piperidin-1-yl)-7-benzyl-1-methyl-2-propyl-1,7-dihydro-purin-6-on
(133) 8-(3-Amino-piperidin-1-yl)-7-benzyl-1-methyl-2-isopropyl-1,7-dihydro-purin-6-on
(134) 8-(3-Amino-piperidin-1-yl)-7-benzyl-1-methyl-2-butyl-1,7-dihydro-purin-6-on
(135) 8-(3-Amino-piperidin-1-yl)-7-benzyl-1-methyl-2-(2-methyl-propyl)-1,7-dihydro-purin-6-on
(136) 8-(3-Amino-piperidin-1-yl)-7-benzyl-1-methyl-2-pentyl-1,7-dihydro-purin-6-on
(137) 8-(3-Amino-piperidin-1-yl)-7-benzyl-1-methyl-2-(3-methyl-butyl)-1,7-dihydro-purin-6-on
(138) 8-(3-Amino-piperidin-1-yl)-7 -benzyl-1-methyl-2-hexyl-1,7-dihydro-purin-6-on
(139) 8-(3-Amino-piperidin-1-yl)-7-benzyl-1-methyl-2-(4-methyl-pentyl)-1,7-dihydro-purin-6-on
(140) 8-(3-Amino-piperidin-1-yl)-7-benzyl-1-methyl-2-allyl-1,7-dihydro-purin-6-on
(141) 8-(3-Amino-piperidin-1-yl)-7-benzyl-1-methyl-2-(3-methyl-but-2-en-1-yl)-1,7-dihydro-purin-6-on
(142) 8-(3-Amino-piperidin-1-yl)-7-benzyl-1-methyl-2-(hex-5-en-1-yl)-1,7-dihydro-purin-6-on
(143) 8-(3-Amino-piperidin-1-yl)-7-benzyl-1-methyl-2-(pent-4-en-1-yl)-1,7-dihydro-purin-6-on
(144) 8-(3-Amino-piperidin-1-yl)-7-benzyl-1-methyl-2-(prop-2-in-1-yl)-1,7-dihydro-purin-6-on
(145) 8-(3-Amino-piperidin-1-yl)-7-benzyl-1-methyl-2-(2-phenylethenyl)-1,7-dihydro-purin-6-on
(146) 8-(3-Amino-piperidin-1-yl)-7-benzyl-1-methyl-2-[2-(4-methyl-phenyl)ethenyl]-1,7-dihydro-purin-6-on
(147) 8-(3-Amino-piperidin-1-yl)-7-benzyl-1-methyl-2-[2-(2-chlor-phenyl)ethenyl]-1,7-dihydro-purin-6-on
(148) 8-(3-Amino-piperidin-1-yl)-7-benzyl-1-methyl-2-[2-(3-fluor-phenyl)ethenyl]-1,7-dihydro-purin-6-on
(149) 8-(3-Amino-piperidin-1-yl)-7-benzyl-1-methyl-2-[2-(3,4-dimethoxy-phenyl)-ethenyl]-1,7-dihydro-purin-6-on
(150) 8-(3-Amino-piperidin-1-yl)-7-benzyl-1-methyl-2-[2-(3-chlor-phenyl)ethenyl]-1,7-dihydro-purin-6-on
(151) 8-(3-Amino-piperidin-1-yl)-7-benzyl-1-methyl-2-[2-(3-trifluormethyl-phenyl)-ethenyl]-1,7-dihydro-purin-6-on
(152) 8-(3-Amino-piperidin-1-yl)-7-benzyl-1-methyl-2-[2-(3-trifluormethoxy-phenyl)-ethenyl]-1,7-dihydro-purin-6-on
(153) 8-(3-Amino-piperidin-1-yl)-7-benzyl-1-methyl-2-(3-phenyl-prop-2-en-1-yl)-1,7-dihydro-purin-6-on
(154) 8-(3-Amino-piperidin-1-yl)-7-benzyl-1-methyl-2-benzyl-1,7-dihydro-purin-6-on
(155) 8-(3-Amino-piperidin-1-yl)-7-benzyl-1-methyl-2-phenyl-1,7-dihydro-purin-6-on
(156) 8-(3-Amino-piperidin-1-yl)-7-benzyl-1-methyl-2-(3-phenyl-propyl)-1,7-dihydro-purin-6-on
(157) 8-(3-Amino-piperidin-1-yl)-7-benzyl-1-methyl-2-(4-fluor-benzyl)-1,7-dihydro-purin-6-on
(158) 8-(3-Amino-piperidin-1-yl)-7-benzyl-l-methyl-2-(3-trifluormethyl-benzyl)-1,7-dihydro-purin-6-on
(159) 8-(3-Amino-piperidin-1-yl)-7-benzyl-1-methyl-2-(3-chlor-benzyl)-1,7-dihydro-purin-6-on
(160) 8-(3-Amino-piperidin-1-yl)-7-benzyl-1-methyl-2-(3-brom-benzyl)-1,7-dihydro-purin-6-on
(161) 8-(3-Amino-piperidin-1-yl)-7-benzyl-1-methyl-2-(3-trifluormethoxy-benzyl)-1,7-dihydro-purin-6-on
(162) 8-(3-Amino-piperidin-1-yl)-7-benzyl-1-methyl-2-(3-methoxy-benzyl)-1,7-dihydro-purin-6-on
(163) 8-(3-Amino-piperidin-1-yl)-7-benzyl-1-methyl-2-[2-(3-chlor-phenyl)ethyl]-1,7-dihydro-purin-6-on
(164) 8-(3-Amino-piperidin-1-yl)-7-benzyl-1-methyl-2-[2-(2-fluor-phenyl)ethyl]-1,7-dihydro-purin-6-on
(165) 8-(3-Amino-piperidin-1-yl)-7-benzyl-1-methyl-2-[2-(4-methyl-phenyl)ethyl]-1,7-dihydro-purin-6-on
(166) 8-(3-Amino-piperidin-1-yl)-7-benzyl-1-methyl-2-[2-(4-fluor-phenyl)ethyl]-1,7-dihydro-purin-6-on
(167) 8-(3-Amino-piperidin-1-yl)-7-benzyl-1-methyl-2-[2-(3-trifluormethyl-phenyl)-ethyl]-1,7-dihydro-purin-6-on
(168) 8-(3-Amino-piperidin-1-yl)-7-benzyl-1-methyl-2-cyclopentyl-1,7-dihydro-purin-6-on
(169) 8-(3-Amino-piperidin-1-yl)-7-benzyl-1-methyl-2-cyclohexyl-1,7-dihydro-purin-6-on
(170) 8-(3-Amino-piperidin-1-yl)-7-benzyl-1-methyl-2-(cyclohexylmethyl)-1,7-dihydro-purin-6-on
(171) 8-(3-Amino-piperidin-1-yl)-7-benzyl-1-methyl-2-(cyclopentylmethyl)-1,7-dihydro-purin-6-on
(172) 8-(3-Amino-piperidin-1-yl)-7-benzyl-1-methyl-2-(2-cyclohexyl-ethyl)-1,7-dihydro-purin-6-on
(173) 8-(3-Amino-piperidin-1-yl)-7-benzyl-1-methyl-2-(furan-2-yl)-1,7-dihydro-purin-6-on
(174) 8-(3-Amino-piperidin-1-yl)-7-benzyl-1-methyl-2-(thien-2-yl)-1,7-dihydro-purin-6-on
(175) 8-(3-Amino-piperidin-1-yl)-7-benzyl-1-methyl-2-(pyridin-2-yl)-1,7-dihydro-purin-6-on
(176) 8-(3-Amino-piperidin-1-yl)-7-benzyl-1-methyl-2-(pyridin-4-yl)-1,7-dihydro-purin-6-on
(177) 8-(3-Amino-piperidin-1-yl)-7-benzyl-1-methyl-2-(furan-2-yl-methyl)-1,7-dihydro-purin-6-on
(178) 8-(3-Amino-piperidin-1-yl)-7-benzyl-1-methyl-2-(thien-2-yl-methyl)-1,7-dihydro-purin-6-on
(179) 8-(3-Amino-piperidin-1-yl)-7-benzyl-1-methyl-2-(pyridin-2-yl-methyl)-1,7-dihydro-purin-6-on
(180) 8-(3-Amino-piperidin-1-yl)-7-benzyl-1-methyl-2-[2-(furan-2-yl)ethyl]-1,7-dihydro-purin-6-on
(181) 8-(3-Amino-piperidin-1-yl)-7-benzyl-1-methyl-2-(2-(pyridin-2-yl)ethyl]-1,7-dihydro-purin-6-on
(182) 8-(3-Amino-piperidin-1-yl)-7-benzyl-1-methyl-2-[3-(furan-2-yl)propyl]-1,7-dihydro-purin-6-on
(183) 8-(3-Amino-piperidin-1-yl)-7-benzyl-1-methyl-2-[3-(pyridin-2-yl)propyl]-1,7-dihydro-purin-6-on
(184) 8-(3-Amino-piperidin-1-yl)-2-benzylamino-1 -methyl-7-(3-chlor-but-2-en-1 -yl)-1,7-dihydro-purin-6-on
(185) 8-(3-Amino-piperidin-1-yl)-2-benzylamino-1-methyl-7 -(3-chlor-prop-2-en-1-yl)-1,7-dihydro-purin-6-on
(186) 8-(3-Amino-piperidin-1-yl)-2-benzylamino-1-methyl-7-(3-trifluormethyl-3-chlor-prop-2-en-1-yl)-1,7-dihydro-purin-6-on
(187) 8-(3-Amino-piperidin-1-yl)-2-benzylamino-1-methyl-7-(3.3-dichlor-prop-2-en-1-yl)-1,7-dihydro-purin-6-on
(188) 8-(3-Amino-piperidin-1-yl)-2-benzylamino-1-methyl-7-(but-2-in-1-yl)-1,7-dihydro-purin-6-on
(189) 8-(3-Amino-piperidin-1-yl)-2-benzylamino-1-methyl-7-(cyclohex-1-en-1-yl-methyl)-1,7-dihydro-purin-6-on
(190) 8-(3-Amino-piperidin-1-yl)-2-benzylamino-1-methyl-7-(cyclohexylmethyl)-1,7-dihydro-purin-6-on
(191) 8-(3-Amino-piperidin-1-yl)-2-benzylamino-1-methyl-7-(4-fluor-benzyl)-1,7-dihydro-purin-6-on
(192) 8-(3-Amino-piperidin-1-yl)-2-benzylamino-1-methyl-7-(2-chlor-benzyl)-1,7-dihydro-purin-6-on
(193) 8-(3-Amino-piperidin-1-yl)-2-benzylamino-1-methyl-7-(3-brom-benzyl)-1,7-dihydro-purin-6-on
(194) 8-(3-Amino-piperidin-1-yl)-2-benzylamino-1-methyl-7-(3-methyl-benzyl)-1,7-dihydro-purin-6-on
(195) 8-(3-Amino-piperidin-1-yl)-2-benzylamino-1-methyl-7-(3-trifluormethyl-benzyl)-1,7-dihydro-purin-6-on
(196) 8-(3-Amino-piperidin-1-yl)-2-benzylamino-1-methyl-7-(2-cyan-benzyl)-1,7-dihydro-purin-6-on
(197) 8-(3-Amino-piperidin-1-yl)-2-benzylamino-1-methyl-7-(2-methoxy-benzyl)-1,7--dihydro-purin-6-on
(198) 8-(3-Amino-piperidin-1-yl)-2-benzylamino-1-methyl-7-(3-trifluormethoxy-benzyl)-1,7-dihydro-purin-6-on
(199) 8-(3-Amino-piperidin-1-yl)-2-benzylamino-1-methyl-7-(3-difluormethoxy-benzyl)-1,7-dihydro-purin-6-on
(200) 8-(3-Amino-piperidin-1-yl)-2-benzylamino-1-methyl-7-(3.4-difluor-benzyl)-1,7-dihydro-purin-6-on
(201) 8-(3-Amino-piperidin-1-yl)-2-benzylamino-1-methyl-7-(naphth-1-yl-methyl)-1,7-dihydro-purin-6-on
(202) 8-(3-Amino-piperidin-1-yl)-2-benzylamino-1-methyl-7-(furan-2-yl-methyl)-1,7-dihydro-purin-6-on
(203) 8-(3-Amino-piperidin-1-yl)-2-benzylamino-1-methyl-7-(thien-2-yl-methyl)-1,7-dihydro-purin-6-on
(204) 8-(3-Amino-piperidin-1-yl)-2-benzylamino-1-methyl-7-(pyridin-2-yl-methyl)-1,7-dihydro-purin-6-on
(205) 8-(3-Amino-piperidin-1-yl)-2-(2-phenylethyl)-1-methyl-7-(3-chlor-but-2-en-1-yl)-1,7-dihydro-purin-6-on
(206) 8-(3-Amino-piperidin-1-yl)-2-(2-phenylethyl)-1-methyl-7-(3-chlor-prop-2-en-1-yl)-1,7-dihydro-purin-8-on
(207) 8-(3-Amino-piperidin-1-yl)-2-(2-phenylethyl)-1-methyl-7-(3-trifluormethyl-3-chlor-prop-2-en-1-yl)-1,7-dihydro-purin-6-on
(208) 8-(3-Amino-piperidin-1-yl)-2-(2-phenylethyl)-1-methyl-7-(3.3-dichlor-prop-2-en-1-yl)-1,7-dihydro-purin-6-on
(209) 8-(3-Amino-piperidin-1-yl)-2-(2-phenylethyl)-1-methyl-7-(but-2-in-1-yl)-1,7-dihydro-purin-6-on
(210) 8-(3-Amino-piperidin-1-yl)-2-(2-phenylethyl)-1-methyl-7-(cyclohex-1-en-1-yl-methyl)-1,7-dihydro-purin-6-on
(211) 8-(3-Amino-piperidin-1-yl)-2-(2-phenylethyl)-1-methyl-7-(cyclohexylmethyl)-1,7-dihydro-purin-6-on
(212) 8-(3-Amino-piperidin-1-yl)-2-(2-phenylethyl)-1-methyl-7-(4-fluor-benzyl)-1,7-dihydro-purin-6-on
(213) 8-(3-Amino-piperidin-1-yl)-2-(2-phenylethyl)-1-methyl-7-(2-chlor-benzyl)-1,7-dihydro-purin-6-on
(214) 8-(3-Amino-piperidin-1-yl)-2-(2-phenylethyl)-1-methyl-7-(3-brom-benzyl)-1,7-dihydro-purin-6-on
(215) 8-(3-Amino-piperidin-1-yl)-2-(2-phenylethyl)-1-methyl-7-(3-methyl-benzyl)-1,7-dihydro-purin-6-on
(216) 8-(3-Amino-piperidin-1-yl)-2-(2-phenylethyl)-1-methyl-7-(3-trifluormethyl-benzyl)-1,7-dihydro-purin-6-on
(217) 8-(3-Amino-piperidin-1-yl)-2-(2-phenylethyl)-1-methyl-7-(2-cyan-benzyl)-1,7-dihydro-purin-6-on
(218) 8-(3-Amino-piperidin-1-yl)-2-(2-phenylethyl)-1-methyl-7-(2-methoxy-benzyl)-1,7-dihydro-purin-6-on
(219) 8-(3-Amino-piperidin-1-yl)-2-(2-phenylethyl)-1-methyl-7-(3-trifluormethoxy-benzyl)-1,7-dihydro-purin-6-on
(220) 8-(3-Amino-piperidin-1-yl)-2-(2-phenylethyl)-1-methyl-7-(3-difluormethoxy-benzyl)-1,7-dihydro-purin-6-on
(221) 8-(3-Amino-piperidin-1-yl)-2-(2-phenylethyl)-1-methyl-7-(3,4-difluor-benzyl)-1,7-dihydro-purin-6-on
(222) 8-(3-Amino-piperidin-1-yl)-2-(2-phenylethyl)-1-methyl-7-(naphth-1-yl-methyl)-1,7-dihydro-purin-6-on
(223) 8-(3-Amino-piperidin-1-yl)-2-(2-phenylethyl)-1-methyl-7-(furan-2-yl-methyl)-1,7-dihydro-purin-6-on
(224) 8-(3-Amino-piperidin-1-yl)-2-(2-phenylethyl)-1-methyl-7-(thien-2-yl-methyl)-1,7-dihydro-purin-6-on
(225) 8-(3-Amino-piperidin-1-yl)-2-(2-phenylethyl)-1-methyl-7-(pyridin-2-yl-methyl)-1,7-dihydro-purin-6-on
(226) 8-(3-Amino-piperidin-1-yl)-2-(2-phenylethyl)-1-methyl-7-benzyl-1,7-dihydro-purin-6-on
(227) 8-(3-Amino-pyrrolidin-1-yl)-2-benzylamino-1-methyl-7-benzyl-1,7-dihydro-purin-6-on
(228) 8-(Piperidin-3-yl)-2-benzylamino-1-methyl-7-benzyl-1,7-dihydro-purin-6-on
(229) 8-(Piperidin-4-yl)-2-benzylamino-1-methyl-7-benzyl-1,7-dihydro-purin-6-on
(230) 8-(3-Amino-hexahydroazepin-1-yl)-2-benzylamino-1-methyl-7-benzyl-1,7-dihydro-purin-6-on
(231) 8-(4-Amino-hexahydroazepin-1-yl)-2-benzylamino-1-methyl-7-benzyl-1,7-dihydro-purin-6-on
(232) 8-(Piperazin-1-yl)-2-benzylamino-1-methyl-7-benzyl-1,7-dihydro-purin-6-on
(233) 8-(1,4-Diazepan-1-yl)-2-benzylamino-1-methyl-7-benzyl-1,7-dihydro-purin-6-on
(234) 8-(2-Amino-cyclohexylamino)-2-benzylamino-1-methyl-7-benzyl-1,7-dihydro-purin-6-on
(235) 8-(3-Amino-cyclohexyl)-2-benzylamino-1-methyl-7-benzyl-1,7-dihydro-purin-6-on
(236) 8-(3-Amino-pyrrolidin-1-yl)-2-(2-phenylethyl)-1-methyl-7-benzyl-1,7-dihydro-purin-6-on
(237) 8-(Piperidin-3-yl)-2-(2-phenylethyl)-1-methyl-7-benzyl-1,7-dihydro-purin-6-on
(238) 8-(Piperidin-4-yl)-2-(2-phenylethyl)-1-methyl-7-benzyl-1,7-dihydro-purin-6-on
(239) 8-(3-Amino-hexahydroazepin-1-yl)-2-(2-phenylethyl)-1-methyl-7-benzyl-1,7-dihydro-purin-6-on
(240) 8-(4-Amino-hexahydroazepin-1-yl)-2-(2-phenylethyl)-1-methyl-7-benzyl-1,7-dihydro-purin-6-on
(241) 8-(Piperazin-1-yl)-2-(2-phenylethyl)-1-methyl-7-benzyl-1,7-dihydro-purin-6-on.
(242) 8-(1,4-Diazepan-1-yl)-2-(2-phenylethyl)-1-methyl-7-benzyl-1,7-dihydro-purin-6-on
(243) 8-(2-Amino-cyclohexylamino)-2-(2-phenylethyl)-1-methyl-7-benzyl-1,7-dihydro-purin-6-on
(244) 8-(3-Amino-cyclohexyl)-2-(2-phenylethyl)-1-methyl-7-benzyl-1,7-dihydro-purin-6-on
(245) 8-(3-Amino-piperidin-1-yl)-2-benzylamino-1-methyl-7-(but-2-en-1-yl)-1,7-dihydro-purin-6-on
(246) 8-(3-Amino-piperidin-1-yl)-2-benzylamino-1-methyl-7-(2-methyl-but-2-en-1-yl)-1,7-dihydro-purin-6-on
(247) 8-(3-Amino-piperidin-1-yl)-2-benzylamino-1-methyl-7-(2,3-dimethyl-but-2-en-1-yl)-1,7-dihydro-purin-6-on
(248) 8-(3-Amino-piperidin-1-yl)-2-benzylamino-1-methyl-7-(cyclopent-1-en-1-yl-methyl)-1,7-dihydro-purin-6-on
(249) 8-(3-Amino-piperidin-1-yl)-7-benzyl-1-(2-phenyl-2-oxo-ethyl)-1,7-dihydro-purin-6-on
(250) 8-(3-Amino-piperidin-1-yl)-7-benzyl-1-[2-(2-amino-phenyl)-2-oxo-ethyl]-1,7-dihydro-purin-6-on
(251) 8-(3-Amino-piperidin-1-yl)-7-benzyl-1-[2-(2-acetylamino-phenyl)-2-oxo-ethyl]-1,7-dihydro-purin-6-on
(252) 8-(3-Amino-piperidin-1-yl)-7-benzyl-1-[2-(2-methylsulfonylamino-phenyl)-2-oxo-ethyl]-1,7-dihydro-purin-6-on
(253) 8-(3-Amino-piperidin-1-yl)-7-benzyl-1-[2-(2-hydroxy-phenyl)-2-oxo-ethyl]-1,7-dihydro-purin-6-on
(254) 8-(3-Amino-piperidin-1-yl)-7-benzyl-1-[2-(2-methoxy-phenyl)-2-oxo-ethyl]-1,7-dihydro-purin-6-on
(255) 8-(3-Amino-piperidin-1-yl)-7-benzyl-1-{2-[2-(ethoxycarbonylmethoxy)-phenyl]-2-oxo-ethyl}-1,7-dihydro-purin-6-on
(256) 8-(3-Amino-piperidin-1-yl)-7-benzyl-1-{2-[2-(aminocarbonylmethoxy)-phenyl]-2-oxo-ethyl}-1,7-dihydro-purin-6-on
(257) 8-(3-Amino-piperidin-1-yl)-7-benzyl-1-{2-[2-(methylaminocarbonyl-methoxy)-phenyl]-2-oxo-ethyl}-1,7-dihydro-purin-6-on
(258) 8-(3-Amino-piperidin-1-yl)-7-benzyl-1-{2-[2-(ethylaminocarbonyl-methoxy)-phenyl]-2-oxo-ethyl}-1,7-dihydro-purin-6-on
(259) 8-(3-Amino-piperidin-1-yl)-7-benzyl-1-{2-[2-(dimethylaminocarbonyl-methoxy)-phenyl]-2-oxo-ethyl}-1,7-dihydro-purin-6-on
(260) 8-(3-Amino-piperidin-1-yl)-7-benzyl-1-(naphth-1-yl-methyl)-1,7-dihydro-purin-6-on
(261) 8-(3-Amino-piperidin-1-yl)-7-benzyl-1-(isochinolin-1-yl-methyl)-1,7-dihydro-purin-6-on
(262) 8-(3-Amino-piperidin-1-yl)-7-benzyl-1-(chinazolin-2-yl-methyl)-1,7-dihydro-purin-6-on
(263) 8-(3-Amino-piperidin-1-yl)-7-benzyl-1-(chinolin-4-yl-methyl)-1,7-dihydro-purin-6-on
(264) 8-(3-Amino-piperidin-1-yl)-7-benzyl-1-(chinazolin-4-yl-methyl)-1,7-dihydro-purin-6-on
(265) 8-(3-Amino-piperidin-1-yl)-7-benzyl-1-(2-phenylethyl)-1,7-dihydro-purin-6-on
(266) 8-[(2-Aminoethyl)amino]-7-benzyl-1-methyl-2-benzylamino-1,7-dihydro-purin-6-on
(267) 8-[N-Methyl-N-(2-aminoethyl)-amino]-7-benzyl-1-methyl-2-benzylamino-1,7-dihydro-purin-6-on
(268) 8-[N-Ethyl-N-(2-aminoethyl)-amino]-7-benzyl-1-methyl-2-benzylamino-1,7-dihydro-purin-6-on
(269) 8-(3-Amino-piperidin-1-yl)-7-benzyl-1-methyl-2-[2-(2-cyanphenyl)ethyl]-1,7-dihydro-purin-6-on
(270) 8-(3-Amino-piperidin-1-yl)-7-benzyl-1-methyl-2-[2-(2-aminocarbonyl-phenyl)-ethyl]-1,7-dihydro-purin-6-on
(271) 8-(3-Amino-piperidin-1-yl)-7-benzyl-1-methyl-2-[2-(2-dimethylaminocarbonyl-phenyl)ethyl]-1,7-dihydro-purin-6-on
(272) 8-(3-Amino-piperidin-1-yl)-7-benzyl-1-methyl-2-[2-(2-methylsulfonyl-phenyl)-ethyl]-1,7-dihydro-purin-6-on
(273) 8-(3-Amino-piperidin-1-yl)-7-benzyl-1-methyl-2-[2-(2-methylsulfonylamino-phenyl)ethyl]-1,7-dihydro-purin-6-on
(274) 8-(3-Amino-piperidin-1-yl)-7-benzyl-1-methyl-2-[(2-methylsulfonylamino-ethyl)-amino]-1,7-dihydro-purin-6-on
(275) 8-(3-Amino-piperidin-1-yl)-7-benzyl-1-methyl-2-[(2-phenylsulfonylamino-ethyl)-amino]-1,7-dihydro-purin-6-on
(276) 8-(3-Amino-piperidin-1-yl)-7-benzyl-1-methyl-2-[(naphth-1-yl-methyl)amino]-1,7-dihydro-purin-6-on
(277) 8-(3-Amino-piperidin-1-yl)-7-benzyl-1-methyl-2-[(chinazolin-2-yl-methyl)amino]-1,7-dihydro-purin-6-on
(278) 8-(3-Amino-piperidin-1-yl)-7-benzyl-1-methyl-2-[(isochinolin-1-yl-methyl)-amino]-1,7-dihydro-purin-6-on
(279) 8-(3-Amino-piperidin-1-yl)-7-benzyl-1-(4,5-dimethyl-oxazol-2-yl-methyl)-1,7-dihydro-purin-6-on
(280) 8-(3-Amino-piperidin-1-yl)-7-benzyl-1-(4,5-dimethyl-thiazol-2-yl-methyl)-1,7-dihydro-purin-6-on
(281) 8-(3-Amino-piperidin-1-yl)-7-benzyl-1-(4,6-dimethyl-pyrimidin-2-yl-methyl)-1,7-dihydro-purin-6-on
(282) 8-(3-Amino-piperidin-1-yl)-7-benzyl-1-(pyrazin-2-yl-methyl)-1,7-dihydro-purin-6-on
(283) 8-(3-Amino-piperidin-1-yl)-7-benzyl-1-methyl-2-[(3-methyl-isoxazol-5-yl-methyl)-amino]-1,7-dihydro-purin-6-on
(284) 8-(3-Amino-piperidin-1-yl)-7-benzyl-1-methyl-2-[(4,5-dimethyl-oxazol-2-yl-methyl)-amino]-1,7-dihydro-purin-6-on
(285) 8-(3-Amino-piperidin-1-yl)-7-benzyl-1-methyl-2-[(pyrazin-2-yl-methyl)-amino]-1,7-dihydro-purin-6-on

### Beispiel 2

### Dragées mit 75 mg Wirksubstanz

### 1 Dragéekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.
Kerngewicht: 230 mg
Stempel: 9 mm, gewölbt

Die so hergestellten Dragéekerne werden mit einem Film überzogen, der im wesentlichen aus Hydroxypropylmethylcellulose besteht. Die fertigen Filmdragées werden mit Bienenwachs geglänzt.
Dragéegewicht: 245 mg.

### Beispiel 3

### Tabletten mit 100 mg Wirksubstanz

### Zusammensetzung:

### 1 Tablette enthält:

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 4

### Tabletten mit 150 mg Wirksubstanz

### Zusammensetzung:

### 1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 5

### Hartgelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel 6

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 7

### Suspension mit 50 mg Wirksubstanz

### 100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 8

### Ampullen mit 10 mg Wirksubstanz

### Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 9

### Ampullen mit 50 mg Wirksubstanz

### Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Purinderivate der allgemeinen Formel 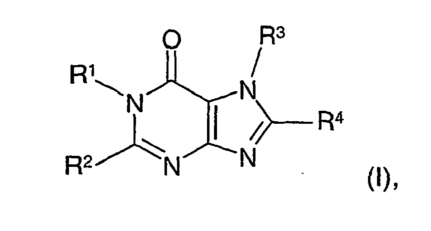 in der
R¹ ein Wasserstoffatom,
eine C₁₋₈-Alkylgruppe,
eine C₃₋₈-Alkenylgruppe,
eine C₃₋₄-Alkenylgruppe, die durch eine C₁₋₂-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl- oder Morpholin-4-ylcarbonyl-gruppe substituiert ist,
eine C₃₋₈-Alkinylgruppe,
eine durch eine Gruppe Rₐ substituierte C₁₋₆-Alkylgruppe, wobei
Rₐ eine C₃₋₇-Cycloalkyl-, Heteroaryl-, Cyano-, Carboxy-, C₁₋₃-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonylgruppe bedeutet,
eine durch eine Phenylgruppe substituierte C₁₋₆-Alkylgruppe, wobei der Phenylring durch die Gruppen R¹⁰ bis R¹⁴ substituiert ist und
R¹⁰ ein Wasserstoffatom,
ein Fluor-, Chlor-, Brom- oder lodatom,
eine C₁₋₄-Alkyl-, Hydroxy- oder C₁₋₄-Alkyfoxygruppe,
eine Nitro-, Amino-, C₁₋₃-Alkylamino-, Di-(C₁₋₃-alkyl)-amino-, Cyan-C₁₋₃-alkylamino-, *N*-(Cyan-C₁₋₃-alkyl)-*N*-(C₁₋₃-alkyl)-amino-, C₁₋₃-Alkyloxy-carbonyl-C₁₋₃-alkylamino-, Pyrrolidin-1-yl-, Piperidin-1-yl-, Morpholin-4-yl-, Piperazin-1-yl- oder 4-(C₁₋₃-Alkyl)-piperazin-1-yl-Gruppe,
eine Formylamino-, C₁₋₃-Alkyl-carbonylamino-, C₃₋₆-Cycloalkyl-carbonylamino-, C₃₋₆-Cycloalkyl-C₁₋₃-alkyl-carbonylamino-, Arylcarbonylamino-, Aryl-C₁₋₃-alkylcarbonylamino-, C₁₋₃-Alkyloxy-carbonylamino-, Aminocarbonylamino-, C₁₋₃-Alkylaminocarbonylamino-, Di-(C₁₋₃-alkyl)-aminocarbonylamino-, Pyrrolidin-1-yl-carbonylamino-, Piperidin-1-yl-carbonylamino-, Morpholin-4-yl-carbonylamino-, Piperazin-1-yl-carbonylamino- oder 4-(C₁₋₃-Alkyl)-piperazin-1-yl-carbonylamino-, C₁₋₃-Alkyl-sulfonylamino-, Bis-(C₁₋₃-alkylsulfonyl)-amino-, Aminosulfonylamino-, C₁₋₃-Alkylamino-sulfonylamino-, Di-(C₁₋₃-alkyl)-amino-sulfonylamino-, Pyrrolidin-1-yl-sulfonylamino-, Piperidin-1-yl-sulfonylamino-, Morpholin-4-yl-sulfonylamino-, Piperazin-1-yl-sulfonylamino- oder 4-(C₁₋₃-Alkyl)-piperazin-1-yl-sulfonylamino-, (C₁₋₃-Alkylamino)-thiocarbonylamino-, (C₁₋₃-Alkyloxy-carbonylamino)-carbonylamino-, Arylsulfonylamino- oder Aryl-C₁₋₃-alkyl-sulfonylaminogruppe,
eine *N*-(C₁₋₃-Alkyl)-formylamino-, *N*-(C₁₋₃-Alkyl)-N-(C₁₋₃-alkyl-carbonyl)-amino-, *N-*(C₁₋₃-Alkyl)-*N*-(C₃₋₆-cycloalkyl-carbonyl)-amino-, *N*-(C₁₋₃-Alkyl)-*N*-(C₃₋₆-cycloalkyl-C₁₋₃-alkyl-carbonyl)-amino-, *N*-(C₁₋₃-Alkyl)-*N*-(arylcarbonyl)-amino-, *N*-(C₁₋₃-Alkyl)-*N*-(aryl-C₁₋₃-alkyl-carbonyl)-amino-, *N*-(C₁₋₃-Alkyl)-*N*-(C₁₋₃-alkyloxycarbonyl)-amino-, *N*-(Aminocarbonyl)-*N*-(C₁₋₃-alkyl)-amino-, *N*-(C₁₋₃-Alkyl-aminocarbonyl)-*N*-(C₁₋₃-alkyl)-amino-, *N*-[Di-(C₁₋₃-alkyl)aminocarbonyl]-*N*-(C₁₋₃-alkyl)-amino-, *N*-(C₁₋₃-Alkyl)-*N*-(C₁₋₃-alkyl-sulfonyl)-amino-, *N*-(C₁₋₃-Alkyl)-*N*-(arylsulfonyl)-amino- oder *N*-(C₁₋₃-Alkyl)-*N*-(aryl-C₁₋₃-alkyl-sulfonyl)-aminogruppe,
eine 2-Oxo-imidazolidin-1-yl-, 2,4-Dioxo-imidazolidin-1-yl-, 2,5-Dioxo-imidazolidin-1-yl- oder 2-Oxo-hexahydropyrimidin-1-ylgruppe, in der das Stickstoffatom in 3-Stellung jeweils durch eine Methyl- oder Ethylgruppe substituiert sein kann,
eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-; Piperazin-1-yl-carbonyl- oder 4-(C₁₋₃-Alkyl)-piperazin-1-yl-carbonylgruppe,
eine C₁₋₃-Alkyl-carbonyl- oder eine Arylcarbonylgruppe,
eine Carboxy-C₁₋₃-alkyl-, C₁₋₃-Alkyloxy-carbonyl-C₁₋₃-alkyl-, Cyan-C₁₋₃-alkyl-, Aminocarbonyl-C₁₋₃-alkyl-, C₁₋₃-Alkyl-aminocarbonyl-C₁₋₃-alkyl-, Di-(C₁₋₃-alkyl)-aminocarbonyl-C₁₋₃-alkyl-, Pyrrolidin-1-yl-carbonyl-C₁₋₃-alkyl-, Piperidin-1-yl-carbonyl-C₁₋₃-alkyl-, Morpholin-4-yl-carbonyl-C₁₋₃-alkyl-, Piperazin-1-yl-carbonyl-C₁₋₃-alkyl- oder 4-(C₁₋₃-Alkyl)-piperazin-1-yl-carbonyl-C₁₋₃-alkylgruppe,
eine Carboxy-C₁₋₃-alkyloxy-, C₁₋₃-Alkyloxy-carbonyl-C₁₋₃-alkyloxy-, Cyan-C₁₋₃-alkyloxy-, Aminocarbonyl-C₁₋₃-alkyloxy-, C₁₋₃-Alkyl-aminocarbonyl-C₁₋₃-alkyloxy-, Di-(C₁₋₃-alkyl)-aminocarbonyl-C₁₋₃-alkyloxy-, Pyrrolidin-1-yl-carbonyl-C₁₋₃-alkyloxy-, Piperidin-1-yl-carbonyl-C₁₋₃-alkyloxy-, Morpholin-4-yl-carbonyl-C₁₋₃-alkyloxy-, Piperazin-1-yl-carbonyl-C₁₋₃-alkyloxy- oder 4-(C₁₋₃-Alkyl)-piperazin-1-yl-carbonyl-C₁₋₃-alkyloxygruppe,
eine Hydroxy-C₁₋₃-alkyl-, C₁₋₃-Alkyloxy-C₁₋₃-alkyl-, Amino-C₁₋₃-alkyl-, C₁₋₃-Alkylamino-C₁₋₃-alkyl-, Di-(C₁₋₃-alkyl)-amino-C₁₋₃-alkyl-, Pyrrolidin-1-yl-C₁₋₃-alkyl-, Piperidin-1-yl-C₁₋₃-alkyl-, Morpholin-4-yl-C₁₋₃-alkyl-, Piperazin-1-yl-C₁₋₃-alkyl-, 4-(C₁₋₃-Alkyl)-piperazin-1-yl-C₁₋₃-alkylgruppe,
eine Hydroxy-C₁₋₃-alkyloxy-, C₁₋₃-Alkyloxy-C₁₋₃-alkyloxy-, C₁₋₃-Alkylsulfanyl-C₁₋₃-alkyloxy-, C₁₋₃-Alkylsulfinyl-C₁₋₃-alkyloxy-, C₁₋₃-Alkylsulfonyl-C₁₋₃-alkyloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, Pyrrolidin-1-yl-C₁₋₃-alkyloxy-, Piperidin-1-yl-C₁₋₃-alkyloxy-, Morpholin-4-yl-C₁₋₃-alkyloxy-, Piperazin-1-yl-C₁₋₃-alkyloxy-, 4-(C₁₋₃-Alkyl)-piperazin-1-yl-C₁₋₃-alkyloxygruppe,
eine Mercapto-, C₁₋₃-Alkylsulfanyl-, C₁₋₃-Alkysulfinyl-, C₁₋₃-Alkylsulfonyl-, C₁₋₃-Alkylsulfonyloxy-, Arylsulfonyloxy-, Trifluormethylsulfanyl-, Trifluormethylsulfinyl- oder Trifluormethylsulfonylgruppe,
eine Sulfo-, Aminosulfonyl-, C₁₋₃-Alkyl-aminosulfonyl-, Di-(C₁₋₃-Alkyl)-aminosulfonyl-, Pyrrolidin-1-yl-sulfonyl-, Piperidin-1-yl-sulfonyl-, Morpholin-4-yl-sulfonyl-, Piperazin-1-yl-sulfonyl- oder 4-(C₁₋₃-Alkyl)-piperazin-1 -yl-sulfonylgruppe,
eine durch 1 bis 3 Fluoratome substituierte Methyl- oder Methoxygruppe,
eine durch 1 bis 5 Fluoratome substituierte Ethyl- oder Ethyloxygruppe,
eine C₂₋₄-Alkenyl- oder C₂₋₄-Alkinylgruppe,
eine C₃₋₄-Alkenyloxy- oder C₃₋₄-Alkinyloxygruppe,
eine C₃₋₆-Cycloalkyl- oder C₃₋₆-Cycloalkyloxygruppe,
eine C₃₋₆-Cycloalkyl-C₁₋₃-alkyl- oder C₃₋₆-Cycloalkyl-C₁₋₃-alkyloxygruppe oder
eine Aryl-, Aryloxy-, Aryl-C₁₋₃-alkyl- oder Aryl-C₁₋₃-alkyloxygruppe,
R¹¹ und R¹², die gleich oder verschieden sein können, jeweils ein Wasserstoffatom, ein Fluor-, Chlor-, Brom- oder lodatom, eine C₁₋₃-Alkyl-, Trifluormethyl-, Hydroxy-, C₁₋₃-Alkyloxy- oder Cyangruppe, oder
R¹¹ zusammen mit R¹², sofern diese an benachbarte Kohlenstoffatome gebunden sind, auch eine Methylendioxy-, Difluormethylendioxy- oder eine geradkettige C₃₋₅-Alkylengruppe und
R¹³ und R¹⁴, die gleich oder verschieden sein können, jeweils ein Wasserstoffatom, ein Fluor-, Chlor- oder Bromatom, eine Trifluormethyl-, C₁₋₃-Alkyl- oder C₁₋₃-Alkyloxygruppe bedeuten,
eine Phenyl-C₁₋₄-alkylgruppe, in der der Alkylteil durch eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-Gruppe substituiert ist und der Phenylteil durch die Gruppen R¹⁰ bis R¹⁴ substituiert ist, wobei R¹⁰ bis R¹⁴ wie vorstehend erwähnt definiert sind,
eine durch die Gruppen R¹⁰ bis R¹⁴ substituierte Phenylgruppe, wobei R¹⁰ bis R¹⁴ wie vorstehend erwähnt definiert sind,
eine Phenyl-C₂₋₃-alkenylgruppe, in der der Phenylteil durch die Gruppen R¹⁰ bis R¹⁴ substituiert ist, wobei R¹⁰ bis R¹⁴ wie vorstehend erwähnt definiert sind,
eine Phenyl-(CH₂)ₘ-A-(CH₂)ₙ-Gruppe, in der der Phenylteil durch R¹⁰ bis R¹⁴ substituiert ist, wobei R¹⁰ bis R¹⁴ wie vorstehend erwähnt definiert sind und
A eine Carbonylgruppe, m die Zahl 0, 1 oder 2 und n die Zahl 1, 2 oder 3 bedeuten,
eine Phenylcarbonylmethylgruppe, in der der Phenylteil durch R¹⁰ bis R¹⁴ substituiert ist, wobei R¹⁰ bis R¹⁴ wie vorstehend erwähnt definiert sind und der Methylteil durch eine C₁₋₃-Alkylgruppe substituiert ist,
eine Phenyl-(CH₂)ₘ-B-(CH₂)ₙ-Gruppe, in der der Phenylteil durch R¹⁰ bis R¹⁴ substituiert ist, wobei R¹⁰ bis R¹⁴, m und n wie vorstehend erwähnt definiert sind und
B eine Methylengruppe, die durch eine Hydroxy-, C₁₋₃-Alkyloxy-, Amino-, C₁₋₃-Alkylamino-, Di-(C₁₋₃-alkyl)-amino-, Mercapto-, C₁₋₃-Alkylsulfanyl-, C₁₋₃-Alkylsulfinyl- oder C₁₋₃-Alkylsulfonylgruppe substituiert ist und gegebenenfalls zusätzlich durch eine Methyl- oder Ethylgruppe substituiert ist, bedeutet,
eine Naphthyl-C₁₋₃-alkylgruppe, in der der Naphthylteil durch die Gruppen R¹⁰ bis R¹⁴ substituiert ist, wobei R¹⁰ bis R¹⁴ wie vorstehend erwähnt definiert sind,
eine Naphthyl-(CH₂)ₘ-A-(CH₂)ₙ-Gruppe, in der der Naphthylteil durch R¹⁰ bis R¹⁴ substituiert ist, wobei R¹⁰ bis R¹⁴, A, m und n wie vorstehend erwähnt definiert sind,
eine Naphthyl-(CH₂)ₘ-B-(CH₂)ₙ-Gruppe, in der der Naphthylteil durch R¹⁰ bis R¹⁴ substituiert ist, wobei R¹⁰ bis R¹⁴, B, m und n wie vorstehend erwähnt definiert sind,
eine [1,4]Naphthochinon-2-yl-, Chromen-4-on-3-yl-, 1-Oxoindan-2-yl-, 1,3-Dioxo-indan-2-yl- oder 2,3-Dihydro-3-oxo-benzofuran-2-ylgruppe
eine Heteroaryl-(CH₂)ₘ-A-(CH₂)ₙ-Gruppe, wobei A, m und n wie vorstehend erwähnt definiert sind,
eine Heteroaryl-(CH₂)ₘ-B-(CH₂)ₙ-Gruppe, wobei B, m und n wie vorstehend erwähnt definiert sind,
eine C₁₋₆-Alkyl-A-(CH₂)ₙ-Gruppe, wobei A und n wie vorstehend erwähnt definiert sind,
eine C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B-(CH₂)ₙ-Gruppe, wobei B, m und n wie vorstehend erwähnt definiert sind,
eine R²¹-A-(CH₂)ₙ-Gruppe, in der R²¹ eine C₁₋₃-Alkyloxycarbonyl-, Aminocarbonyl-, C₁₋₃-Alkylaminocarbonyl-, Di-(C₁₋₃-alkyl)aminocarbonyl-, Pyrrolidin-1-yl-carbonyl-, Piperidin-1-yl-carbonyl- oder Morpholin-4-yl-carbonyl-, Piperazin-1-yl-carbonyl-, 4-Methylpiperazin-1-yl-carbonyl- oder 4-Ethylpiperazin-1-yl-carbonyl-Gruppe bedeutet und A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴ und m wie vorstehend erwähnt sind und D eine Sauerstoff- oder Schwefelatom, eine Imino-, C₁₋₃-Alkylimino-, Sulfinyl- oder Sulfonylgruppe bedeutet,
eine Naphthyl-(CH₂)ₘ-D-C₁₋₃-alkylgruppe, in der der Naphthylteil durch die Gruppen R¹⁰ bis R¹⁴ substituiert ist, wobei R¹⁰ bis R¹⁴, D und m wie vorstehend erwähnt sind,
eine durch eine Gruppe R_{b} substituierte C₂₋₆-Alkylgruppe, wobei
R_{b} durch mindestens zwei Kohlenstoffatome vom Ringstickstoffatom in 1-Stellung des Puringerüstes isoliert ist und
R_{b} eine Hydroxy-, C₁₋₃-Alkyloxy-, Mercapto-, C₁₋₃-Alkylsulfanyl-, C₁₋₃-Alkylsulfinyl, C₁₋₃-Alkylsulfonyl-, Amino-, C₁₋₃-Alkyl-carbonylamino-, C₃₋₆-Cycloalkyl-carbonylamino-, Arylcarbonylamino-, C₁₋₃-Alkylamino-, Di-(C₁₋₃-alkyl)-amino-, Pyrrolidin-1-yl-, Piperidin-1-yl-, Morpholin-4-yl-; Piperazin-1-yl- oder 4-(C₁₋₃-Alkyl)-piperazin-1-ylgruppe bedeutet,
eine C₃₋₆-Cycloalkylgruppe,
oder eine Amino- oder Arylcarbonylaminogruppe,
R² ein Wasserstoffatom,
eine C₁₋₈-Alkylgruppe,
eine C₃₋₈-Alkenylgruppe,
eine C₃₋₄-Alkenylgruppe, die durch eine C₁₋₂-Alkyloxy-carbonyl-, Aminocarbonyl-, C₁₋₃-Alkylamino-carbonyl-, Di-(C₁₋₃-alkyl)-amino-carbonyl-, Pyrrolidin-1-ylcarbonyl-, Piperidin-1-ylcarbonyl- oder Morpholin-4-ylcarbonyl-gruppe substituiert ist,
eine C₃₋₈-Alkinylgruppe,
eine C₃₋₆-Cycloalkyl-Gruppe,
eine durch eine Gruppe Rₐ substituierte C₁₋₆-Alkylgruppe, wobei Rₐ wie vorstehend erwähnt definiert ist,
eine Phenylgruppe, die durch R¹⁰ bis R¹⁴ substituiert ist, wobei R¹⁰ bis R¹⁴ wie vorstehend erwähnt definiert sind,
eine durch eine Phenylgruppe substituierte C₁₋₆-Alkylgruppe, wobei der Phenylring durch die Gruppen R¹⁰ bis R¹⁴ substituiert ist und R¹⁰ bis R¹⁴ wie vorstehend erwähnt definiert sind,
eine Phenyl-C₁₋₄-alkylgruppe, in der der Alkylteil durch eine Cyan-, Carboxy-, C₁₋₃-Alkyloxy-carbonyl-, Aminocarbonyl-, C₁₋₃-Alkyl-aminocarbonyl-, Di-(C₁₋₃-alkyl)-aminocarbonyl-, Pyrrolidin-1-yl-carbonyl-, Piperidin-1-yl-carbonyl-, Morpholin-4-yl-carbonyl-Gruppe substituiert ist und der Phenylteil durch die Gruppen R¹⁰ bis R¹⁴ substituiert ist, wobei R¹⁰ bis R¹⁴ wie vorstehend erwähnt definiert sind,
eine Phenyl-C₂₋₃-alkenyl-Gruppe, in der der Phenylteil durch R¹⁰ bis R¹⁴ substituiert ist, wobei R¹⁰ bis R¹⁴ wie vorstehend erwähnt definiert sind,
eine Heteroaryl-Gruppe,
eine Phenyl-(CH₂)ₘ-A- oder Phenyl-(CH₂)ₘ-A-(CH₂)ₙ-Gruppe, in der der Phenylteil jeweils durch R¹⁰ bis R¹⁴ substituiert ist, wobei A, R¹⁰ bis R¹⁴, m und n wie vorstehend erwähnt definiert sind,
eine Phenylcarbonylmethylgruppe, in der der Phenylteil durch R¹⁰ bis R¹⁴ substituiert ist, wobei R¹⁰ bis R¹⁴ wie vorstehend erwähnt definiert sind und der Methylteil durch eine C₁₋₃-Alkylgruppe substituiert ist,
eine Phenyl-(CH₂)ₘ-B- oder Phenyl-(CH₂)ₘ-B-(CH₂)ₙ-Gruppe, in der der Phenylteil jeweils durch R¹⁰ bis R¹⁴ substituiert ist, wobei B, R¹⁰ bis R¹⁴, m und n wie vorstehend erwähnt definiert sind,
eine Naphthyl-C₁₋₃-alkylgruppe, in der der Naphthylteil durch die Gruppen R¹⁰ bis R¹⁴ substituiert ist, wobei R¹⁰ bis R¹⁴ wie vorstehend erwähnt definiert sind,
eine Naphthyl-(CH₂)ₘ-A- oder Naphthyl-(CH₂)ₘ-A-(CH₂)ₙ-Gruppe, in der der Naphthylteil jeweils durch R¹⁰ bis R¹⁴ substituiert ist, wobei R¹⁰ bis R¹⁴, A, m und n wie vorstehend erwähnt definiert sind,
eine Naphthyl-(CH₂)ₘ-B- oder Naphthyl-(CH₂)ₘ-B-(CH₂)ₙ-Gruppe, in der der Naphthylteil jeweils durch R¹⁰ bis R¹⁴ substituiert ist, wobei R¹⁰ bis R¹⁴, B, m und n wie vorstehend erwähnt definiert sind,
eine Heteroaryl-(CH₂)ₘ-A- oder Heteroaryl-(CH₂)ₘ-A-(CH₂)ₙ-Gruppe, wobei A, m und n wie vorstehend erwähnt definiert sind,
eine Heteroaryl-(CH₂)ₘ-B- oder Heteroaryl-(CH₂)ₘ-B-(CH₂)ₙ-Gruppe, wobei B, m und n wie vorstehend erwähnt definiert sind,
eine C₁₋₆-Alkyl-A- oder C₁₋₆-Alkyl-A-(CH₂)ₙ-Gruppe, wobei A und n wie vorstehend erwähnt definiert sind,
eine C₃₋₇-Cycloalkyl-(CH₂)ₘ-A- oder C₃₋₇-Cycloalkyl-(CH₂)ₘ-A-(CH₂)ₙ-Gruppe, wobei A, m und n wie vorstehend erwähnt definiert sind,
eine C₃₋₇-Cycloalkyl-(CH₂)ₘ-B- oder C₃₋₇-Cycloalkyl-(CH₂)ₙ-B-(CH₂)ₙ-Gruppe, wobei B, m und n wie vorstehend erwähnt definiert sind,
eine R²¹-A-(CH₂)ₙ-Gruppe, in der R²¹, A und n wie vorstehend erwähnt definiert sind,
eine Phenyl-(CH₂)ₘ-D-C₁₋₃-alkylgruppe, in der der Phenylteil durch die Gruppen R¹⁰ bis R¹⁴ substituiert ist, wobei R¹⁰ bis R¹⁴, D und m wie vorstehend erwähnt sind,
eine Naphthyl-(CH₂)ₘ-D-C₁₋₃-alkylgruppe, in der der Naphthylteil durch die Gruppen R¹⁰ bis R¹⁴ substituiert ist, wobei R¹⁰ bis R¹⁴, D und m wie vorstehend erwähnt sind,
eine durch eine Gruppe R_{b} substituierte C₁₋₆-Alkylgruppe, wobei R_{b} wie vorstehend erwähnt definiert ist,
eine Cyano-, Carboxy-, C₁₋₃-Alkyloxy-carbonyl-, Aminocarbonyl-, C₁₋₃-Alkylaminocarbonyl-, Di-(C₁₋₃-alkyl)-amino-carbonyl-, Pyrrolidin-1-ylcarbonyl-, Piperidin-1-ylcarbonyl-, Morpholin-4-ylcarbonyl-, Piperazin-1-ylcarbonyl-, 4-Methylpiperazin-1-ylcarbonyl- oder 4-Ethylpiperazin-1-ylcarbonyl-Gruppe,
eine Amino-, C₁₋₆-Alkylamino- oder Di-(C₁₋₆-alkyl)-aminogruppe,
eine durch die Reste R¹⁵ und R¹⁶ substituierte Aminogruppe, in der
R¹⁵ ein Wasserstoffatom oder eine C₁₋₆-Alkylgruppe und
R¹⁶ eine C₁₋₆-Alkylgruppe, die durch Rₐ substituiert ist, bedeuten, wobei Rₐ wie vorstehend erwähnt definiert ist,
eine durch die Reste R¹⁵ und R¹⁷ substituierte Aminogruppe, in der
R¹⁵ wie vorstehend erwähnt definiert ist und
R¹⁷ eine C₂₋₆-Alkylgruppe, die durch eine Hydroxy-, C₁₋₃-Alkyloxy-, Aryloxy-, Mercapto-, C₁₋₃-Alkylsulfanyl-, C₁₋₃-Alkylsulfinyl-, C₁₋₃-Alkylsulfonyl-, C₁₋₃-Alkylsulfonylamino-, Arylsulfanyl-, Arylsulfinyl-, Arylsulfonyl-, Arylsulfonylamino-, C₁₋₃-Alkyl-carbonylamino-, C₃₋₆-Cycloalkyl-carbonylamino-, Arylcarbonylamino-, C₁₋₃-Alkyl-oxycarbonylamino-, Aminocarbonylamino-, C₁₋₃-Alkylaminocarbonylamino-, Di-(C₁₋₃-alkyl)-aminocarbonylamino-, Amino-, C₁₋₃-Alkylamino-, Di-(C₁₋₃-alkyl)-amino-, Pyrrolidin-1-yl-, Piperidin-1-yl-, Morpholin-4-yl-, Piperazin-1-yl- oder 4-(C₁₋₃-Alkyl)-piperazin-1-yl-Gruppe substituiert ist, bedeuten,
eine C₃₋₆-Cycloalkylamino- oder *N*-(C₃₋₆-Cycloalkyl)-*N*-(C₁₋₃-alkyl)-amino-Gruppe,
eine Phenylamino- oder *N*-(Phenyl)-*N*-(C₁₋₃-alkyl)-amino-Gruppe, in der der Phenylteil jeweils durch R¹⁰ bis R¹⁴ substituiert ist, wobei R¹⁰ bis R¹⁴ wie vorstehend erwähnt definiert sind,
eine Phenyl-C₁₋₆-alkylamino- oder *N*-(Phenyl-C₁₋₆-alkyl)-*N*-(C₁₋₃-alkyl)-amino-Gruppe, in der der Phenylteil jeweils durch R¹⁰ bis-R¹⁴ substituiert ist, wobei R¹⁰ bis R¹⁴ wie vorstehend erwähnt definiert sind,
eine Naphthylamino- oder *N*-(Naphthyl)-*N*-(C₁₋₃-alkyl)-amino-Gruppe,
eine Naphthyl-C₁₋₆-alkylamino- oder *N*-(Naphthyl-C₁₋₆-alkyl)-*N*-(C₁₋₃-alkyl)-aminoGruppe,
eine Heteroarylamino- oder *N*-(Heteroaryl)-*N*-(C₁₋₃-alkyl)-amino-Gruppe,
eine Pyrrolidin-1-yl-, Piperidin-1-yl-, Homopiperidin-1-yl-, Morpholin-4-yl-, Homomorpholin-4-yl-, Piperazin-1-yl-, 4-(C₁₋₃-Alkyl)-piperazin-1-yl-, Homopiperazin-1-yl-oder 4-(C₁₋₃-Alkyl)-homopiperazin-1-yl-Gruppe, oder
eine C₁₋₆-Al kyloxy-, C₃₋₆-Cycloalkyloxy- oder C₃₋₆-Cycloalkyl-C₁₋₆-alkyloxy-Gruppe,
eine C₁₋₆-Alkylsulfanyl-, C₃₋₆-Cycloalkylsulfanyl- oder C₃₋₆-Cycloalkyl-C₁₋₆-alkylsulfanyl-Gruppe,
eine Phenyloxy- oder Phenylsulfanylgruppe, in der der Phenylteil jeweils durch R¹⁰ bis R¹⁴ substituiert ist, wobei R¹⁰ bis R¹⁴ wie vorstehend erwähnt definiert sind,
eine Phenyl-C₁₋₆-alkyloxy- oder Phenyl-C₁₋₆-alkylsulfanylgruppe, in der der Phenylteil jeweils durch R¹⁰ bis R¹⁴ substituiert ist, wobei R¹⁰ bis R¹⁴ wie vorstehend erwähnt definiert sind,
eine Naphthyloxy- oder eine Naphthylsulfanyl-Gruppe, in der der Naphthylteil jeweils durch R¹⁰ bis R¹⁴ substituiert ist, wobei R¹⁰ bis R¹⁴ wie vorstehend erwähnt definiert sind,
eine Naphthyl-C₁₋₆-alkyloxy- oder Naphthyl-C₁₋₆-alkylsulfanyl-Gruppe, in der der Naphthylteil jeweils durch R¹⁰ bis R¹⁴ substituiert ist, wobei R¹⁰ bis R¹⁴ wie vorstehend erwähnt definiert sind,
eine Heteroaryloxy- oder Heteroarylsulfanyl-Gruppe oder
eine Heteroaryl-C₁₋₆-alkyloxy- oder Heteroaryl-C₁₋₆-alkylsulfanyl-Gruppe,
R³ eine C₁₋₈-Alkylgruppe,
eine durch die Gruppe R_{c} substituierte C₁₋₄-Alkylgruppe, wobei
R_{c} eine gegebenenfalls durch eine oder zwei C₁₋₃-Alkylgruppen substituierte C₃₋₇-Cycloalkylgruppe,
eine gegebenenfalls durch eine oder zwei C₁₋₃-Alkylgruppen substituierte C₅₋₇-Cycloalkenylgruppe,
eine Arylgruppe oder
eine Furanyl-, Thienyl-, Oxazolyl-, Isoxazolyl-, Thiazolyl-, Isothiazolyl-, Pyridyl-, Pyridazinyl-, Pyrimidyl- oder Pyrazinylgruppe bedeutet, wobei die vorstehend erwähnten heterocyclischen Reste jeweils durch eine oder zwei C₁₋₃-Alkylgruppen oder durch ein Fluor-, Chlor-, Brom- oder lodatom oder durch eine Trifluormethyl-, Cyan- oder C₁₋₃-Alkyloxygruppe substituiert sein können,
eine C₃₋₈-Alkenylgruppe,
eine durch ein Fluor-, Chlor- oder Bromatom oder eine Trifluormethylgruppe substituierte C₃₋₆-Alkenylgruppe,
eine C₃₋₈-Alkinylgruppe,
eine Arylgruppe oder
eine Aryl-C₂₋₄-alkenylgruppe,
und
R⁴ eine Azetidin-1-yl- oder Pyrrolidin-1-ylgruppe, die in 3-Stellung durch eine Amino-, C₁₋₃-Alkylamino- oder Di-(C₁₋₃-alkyl)-amino-Gruppe substituiert ist und zusätzlich durch eine oder zwei C₁₋₃-Alkylgruppen substituiert sein kann,
eine Piperidin-1-yl- oder Hexahydroazepin-1-ylgruppe, die in 3-Stellung oder in 4-Stellung durch eine Amino-, C₁₋₃-Alkylamino- oder Di-(C₁₋₃-alkyl)amino-Gruppe substituiert ist und zusätzlich durch eine oder zwei C₁₋₃-Alkylgruppen substituiert sein kann,
eine 3-Amino-piperidin-1-ylgruppe, in der der Piperidin-1-yl-Teil zusätzlich durch eine Aminocarbonyl-, C₁₋₂-Alkyl-aminocarbonyl-, Di-(C₁₋₂-alkyl)-aminocarbonyl-, Pyrrolidin-1-yl-carbonyl-, (2-Cyan-pyrrolidin-1-yl-)carbonyl-, Thiazolidin-3-yl-carbonyl-, (4-Cyan-thiazolidin-3-yl)carbonyl-, Piperidin-1-ylcarbonyl- oder Morpholin-4-ylcarbonyl-Gruppe substituiert ist,
eine 3-Amino-piperidin-1-ylgruppe, in der der Piperidin-1-yl-Teil in 4-Stellung oder in 5-Stellung zusätzlich durch eine Hydroxy- oder Methoxygruppe substituiert ist,
eine 3-Amino-piperidin-1-ylgruppe, in der die Methylengruppe in 2-Stellung oder in 6-Stellung durch eine Carbonylgruppe ersetzt ist,
eine in 3-Stellung durch eine Amino-, C₁₋₃-Alkylamino- oder Di-(C₁₋₃-alkyl)-aminogruppe substituierte Piperidin-1-yl- oder Hexahydroazepin-1-yl-gruppe, in denen jeweils zwei Wasserstoffatome am Kohlenstoffgerüst der Piperidin-1-yl- oder Hexahydroazepin-1-yl-gruppe durch eine geradkettige Alkylenbrücke ersetzt sind, wobei diese Brücke 2 bis 5 Kohlenstoffatome enthält, wenn die zwei Wasserstoffatome sich am selben Kohlenstoffatom befinden, oder 1 bis 4 Kohlenstoffatome enthält, wenn sich die Wasserstoffatome an benachbarten Kohlenstoffatomen befinden, oder 1 bis 4 Kohlenstoffatome enthält, wenn sich die Wasserstoffatome an Kohlenstoffatomen befinden, die durch ein Atom getrennt sind, oder 1 bis 3 Kohlenstoffatome enthält, wenn sich die zwei Wasserstoffatome an Kohlenstoffatomen befinden, die durch zwei Atome getrennt sind,
eine Azetidin-1-yl-, Pyrrolidin-1yl-, Piperidin-1-yl- oder Hexahydroazepin-1-ylgruppe, die durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine gegebenenfalls am Kohlenstoffgerüst durch eine oder zwei C₁₋₃-Alkylgruppen substituierte Piperazin-1-yl- oder [1,4]Diazepan-1-ylgruppe,
eine gegebenenfalls am Kohlenstoffgerüst durch eine oder zwei C₁₋₃-Alkylgruppen substituierte 3-Imino-piperazin-1-yl-, 3-Imino-[1,4]diazepan-1-yl- oder 5-Imino-[1,4]diazepan-1-ylgruppe,
eine gegebenenfalls durch eine oder zwei C₁₋₃-Alkylgruppen substituierte [1,4]Diazepan-1-ylgruppe, die in 6-Stellung durch eine Aminogruppe substituiert ist,
eine C₃₋₇-Cycloalkylgruppe, die durch eine Amino-, C₁₋₃-Alkylamino- oder Di-(C₁₋₃-alkyl)-aminogruppe substituiert ist,
eine C₃₋₇-Cycloalkylgruppe, die durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkylgruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkylgruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkylaminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine *N*-(C₃₋₇-Cycloalkyl)-*N*-(C₁₋₃-alkyl)-aminogruppe, in der der Cycloalkylteil durch eine Amino-, C₁₋₃-Alkylamino- oder Di-(C₁₋₃-alkyl)-aminogruppe substituiert ist, wobei die beiden Stickstoffatome am Cycloalkylteil durch mindestens zwei Kohlenstoffatome voneinander getrennt sind,
eine C₃₋₇-Cycloalkylaminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine *N*-(C₃₋₇-Cycloalkyl)-*N*-(C₁₋₃-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine C₃₋₇-Cycloalkyl-C₁₋₂-alkyl-aminogruppe, in der der Cycloalkylteil durch eine Amino-, C₁₋₃-Alkylamino- oder Di-(C₁₋₃-alkyl)-aminogruppe substituiert ist,
eine *N*-(C₃₋₇-Cycloalkyl-C₁₋₂-alkyl)-*N*-(C₁₋₂-alkyl)-aminogruppe, in der der Cycloalkylteil durch eine Amino-, C₁₋₃-Alkylamino- oder Di-(C₁₋₃-alkyl)-aminogruppe substituiert ist,
eine C₃₋₇-Cycloalkyl-C₁₋₂-alkyl-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)amino-C₁₋₃-alkylgruppe substituiert ist,
eine *N*-(C₃₋₇-Cycloalkyl-C₁₋₂-alkyl)-*N*-(C₁₋₂-alkyl)-aminogruppe, in der der Cycloalkylteil durch eine Amino-C₁₋₃-alkyl-, C₁₋₃-Alkylamino-C₁₋₃-alkyl- oder eine Di-(C₁₋₃-alkyl)-amino-C₁₋₃-alkylgruppe substituiert ist,
eine R¹⁹-C₂₋₄-Alkylamino-Gruppe, in der R¹⁹ durch mindestens zwei Kohlenstoffatome vom Stickstoffatom des C₂₋₄-Alkylamino-Teils getrennt ist und
R¹⁹ eine Amino-, C₁₋₃-Alkylamino- oder Di-(C₁₋₃-alkyl)-aminogruppe darstellt,
eine R¹⁹-C₂₋₄-Alkylamino-Gruppe, in der das Stickstoffatom des C₂₋₄-Alkylamino-Teils durch eine C₁₋₃-Alkylgruppe substituiert ist und R¹⁹ durch mindestens zwei Kohlenstoffatome vom Stickstoffatom des C₂₋₄-Alkylamino-Teils getrennt ist, wobei R¹⁹ wie vorstehend erwähnt definiert ist,
eine durch den Rest R²⁰ substituierte Aminogruppe, in der
R²⁰ eine Azetidin-3-yl, Azetidin-2-ylmethyl-, Azetidin-3-ylmethyl-, Pyrrolidin-3-yl-, Pyrrolidin-2-ylmethyl-, Pyrrolidin-3-ylmethyl-, Piperidin-3-yl-, Piperidin-4-yl-, Piperidin-2-ylmethyl-, Piperidin-3-ylmethyl- oder Piperidin-4-ylmethylgruppe darstellt, wobei die für R²⁰ erwähnten Reste jeweils durch eine oder zwei C₁₋₃-Alkylgruppen substituiert sein können,
eine durch den Rest R²⁰ und eine C₁₋₃-Alkylgruppe substituierte Aminogruppe, in der R²⁰ wie vorstehend erwähnt definiert ist, wobei die für R²⁰ erwähnten Reste jeweils durch eine oder zwei C₁₋₃-Alkylgruppen substituiert sein können,
eine R¹⁹-C₃₋₄-alkyl-gruppe, in der der C₃₋₄-Alkylteil geradkettig ist und zusätzlich durch eine oder zwei C₁₋₃-Alkylgruppen substituiert sein kann, wobei R¹⁹ wie vorstehend erwähnt definiert ist,
eine 3-Amino-2-oxo-piperidin-5-yl- oder 3-Amino-2-oxo-1-methyl-piperidin-5-yl-Gruppe,
eine Pyrrolidin-3-yl-, Piperidin-3-yl-, Piperidin-4-yl, Hexahydroazepin-3-yl- oder Hexahydroazepin-4-ylgruppe, die in 1-Stellung durch eine Amino-, C₁₋₃-Alkylamino- oder Di-(C₁₋₃-alkyl)aminogruppe substituiert ist,
oder eine Azetidin-2-yl-C₁₋₂-alkyl-, Azetidin-3-yl-C₁₋₂-alkyl, Pyrrolidin-2-yl-C₁₋₂-alkyl-, Pyrrolidin-3-yl-, Pyrrolidin-3-yl-C₁₋₂-alkyl-, Piperidin-2-yl-C₁₋₂-alkyl-, Piperidin-3-yl-, Piperidin-3-yl-C₁₋₂-alkyl-, Piperidin-4-yl- oder Piperidin-4-yl-C₁₋₂-alkylgruppe, wobei die vorstehend erwähnten Gruppen jeweils durch eine oder zwei C₁₋₃-Alkylgruppen substituiert sein können, bedeuten,
wobei unter den bei der Definition der vorstehend genannten Reste erwähnten Arylgruppen Phenyl- oder Naphthylgruppen zu verstehen sind, welche unabhängig voneinander durch Rₕ mono- oder disubstituiert sein können, wobei die Substituenten gleich oder verschieden sein können und Rₕ ein Fluor-, Chlor-, Brom- oder lodatom, eine Trifluormethyl-, Cyan-, Nitro-, Amino-, Aminocarbonyl-, Aminosulfonyl-, Methylsulfonyl, Acetylamino-, Methylsulfonylamino-, C₁₋₃-Alkyl-, Cyclopropyl-, Ethenyl-, Ethinyl-, Hydroxy-, C₁₋₃-Alkyloxy-, Difluormethoxy- oder Trifluormethoxygruppe darstellt,
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
oder eine 1,2-Dihydro-2-oxo-pyridinyl-, 1,4-Dihydro-4-oxo-pyridinyl-, 2,3-Dihydro-3-oxo-pyridazinyl-, 1,2,3,6-Tetrahydro-3,6-dioxo-pyridazinyl-, 1,2-Dihydro-2-oxo-pyrimidinyl-, 3,4-Dihydro-4-oxo-pyrimidinyl-, 1,2,3,4-Tetrahydro-2,4-dioxo-pyrimidinyl-, 1,2-Dihydro-2-oxo-pyrazinyl-, 1-,2,3,4-Tetrahydro-2,3-dioxo-pyrazinyl-, 2,3-Dihydro-2-oxo-indolyl-, 2,3-Dihydrobenzofuranyl-, 2,3-Dihydro-2-oxo-1*H*-benzimidazolyl-,2,3-Dihydro-2-oxo-benzoxazolyl-, 1,2-Dihydro-2-oxo-chinolinyl-, 1,4-Dihydro-4-oxo-chinolinyl-, 1,2-Dihydro-1-oxo-isochinolinyl-, 1,4-Dihydro-4-oxo-cinnolinyl-, 1,2-Dihydro-2-oxo-chinazolinyl-, 3,4-Dihydro-4-oxo-chinazolinyl-, 1,2,3,4-Tetrahydro-2,4-dioxo-chinazolinyl-, 1,2-Dihydro-2-oxochinoxalinyl-, 1,2,3,4-Tetrahydro-2,3-dioxo-chinoxalinyl-, 1,2-Dihydro-1-oxo-phthalazinyl-, 1,2,3,4-Tetrahydro-1,4-dioxo-phthalazinyl-, Chromanyl-, Cumarinyl-, 2,3-Dihydro-benzo[1,4]dioxinyl- oder 3,4-Dihydro-3-oxo-2*H*-benzo[1,4]oxazinyl-Gruppe zu verstehen ist,
wobei die vorstehend erwähnten Heteroarylgruppen durch R¹⁰ bis R¹⁴ substituiert sein können, wobei R¹⁰ bis R¹⁴ wie vorstehend erwähnt definiert sind,
wobei, soweit nichts anderes erwähnt wurde, die vorstehend erwähnten Alkyl-, Alkenyl- und Alkinylgruppen geradkettig oder verzweigt sein können,
sowie die am Ringstickstoffatom in 3-Stellung oder in 9-Stellung des Hypoxanthingerüstes N-oxidierten oder methylierten oder ethylierten Derivate,
sowie die Derivate, in denen die 6-Oxogruppe des Hypoxanthingerüstes durch eine Thioxogruppe ersetzt ist,
mit der Maßgabe, daß die Verbindungen
8-(Piperidin-4-ylmethyl)-7-(4-fluorbenzyl)-1,7-dihydro-purin-6-on,
8-(1-Methyl-piperidin-4-ylmethyl)-7-(4-fluorbenzyl)-1,7-dihydro-purin-6-on,
8-(Piperidin-4-ylamino)-7-(4-fluorbenzyl)-1,7-dihydro-purin-6-on und 8-(1-Methyl-piperidin-4-ylamino)-7-(4-fluorbenzyl)-1,7-dihydro-purin-6-on
ausgeschlossen sind,
deren Tautomere, Enantiomere, Diastereomere, deren Gemische, und deren Salze.

2. Purinderivate der allgemeinen Formel I gemäß Anspruch 1,
in der R¹, R² und R³ wie in Anspruch 1 erwähnt definiert sind und
R⁴ eine Pyrrolidin-1-ylgruppe, die in 3-Stellung durch eine Aminogruppe substituiert ist,
eine Piperidin-1-ylgruppe, die in 3-Stellung durch eine Aminogruppe substituiert ist,
eine Piperidin-3-yl- oder Piperidin-4-ylgruppe,
eine Hexahydroazepin-1-yl-gruppe, die in 3-Stellung oder in 4-Stellung durch eine Aminogruppe substituiert ist,
eine Piperazin-1-yl- oder [1,4]Diazepan-1-ylgruppe,
eine (2-Aminocyclohexyl)amino-Gruppe,
eine Cyclohexylgruppe, die in 3-Stellung durch eine Aminogruppe substituiert ist,
oder eine N-(2-Aminoethyl)-methylamino- oder eine N-(2-Aminoethyl)-ethylamino-Gruppe bedeutet,
deren Tautomere, Enantiomere, Diastereomere, deren Gemische, und deren Salze.

3. Purinderivate der allgemeinen Formel 1 gemäß Anspruch 1, in der
R¹ ein Wasserstoffatom,
eine C₁₋₆-Alkylgruppe,
eine C₃₋₆-Alkenylgruppe,
eine C₃₋₄-Alkinylgruppe,
eine C₃₋₆-Cycloalkylmethyl-Gruppe,
eine Phenyl-C₁₋₃-alkyl-Gruppe, in der der Phenylteil durch R¹⁰ und R¹¹ substituiert ist,
wobei
R¹⁰ ein Wasserstoffatom, ein Fluor- Chlor- oder Bromatom,
eine Methyl- oder Trifluormethyl-Gruppe,
eine Cyan-, Aminocarbonyl-, Dimethylaminocarbonyl- oder Methylsulfonyl-Gruppe,
eine Amino-, Acetylamino- oder Methylsulfonylamino-Gruppe,
eine Hydroxy-, Methoxy-, Difluormethoxy-, Trifluormethoxy-, Carboxymethoxy-, Methoxycarbonylmethoxy-, Ethyloxycarbonylmethoxy-, Aminocarbonylmethoxy-, Methylaminocarbonylmethoxy-, Ethylaminocarbonylmethoxy- oder Dimethylaminocarbonylmethoxy-Gruppe und
R¹¹ ein Wasserstoffatom, ein Fluor- oder Chloratom,
oder eine Methyl- oder Methoxy-Gruppe bedeuten,
eine Naphthylmethylgruppe, in der der Naphthylteil durch R¹⁰ und R¹¹ substituiert ist, wobei R¹⁰ und R¹¹ wie vorstehend erwähnt definiert sind,
eine Heteroarylmethyl-Gruppe, wobei unter dem Begriff
Heteroaryl eine Furanyl-, Thienyl-, Oxazolyl-, Isoxazolyl-, Thiazolyl-, Pyridyl-, Pyrimidinyl-, Pyrazinyl-, Chinolinyl-, Isochinolinyl- oder Chinazolinylgruppe zu verstehen ist und die vorstehend erwähnten Heteroarylgruppen durch R¹⁰ und R¹¹ substituiert sind, wobei R¹⁰ und R¹¹ wie vorstehend erwähnt definiert sind,
eine Phenylcarbonylmethylgruppe, in der der Phenylteil durch R¹⁰ und R¹¹ substituiert ist, wobei R¹⁰ und R¹¹ wie vorstehend erwähnt definiert sind,
eine Furanylcarbonylmethyl-, Thienylcarbonylmethyl- oder Pyridylcarbonylmethylgruppe,
oder eine 2-Oxo-propyl- oder Cyclohexylcarbonylmethylgruppe,
R² ein Wasserstoffatom,
eine C₁₋₆-Alkylgruppe,
eine C₃₋₆-Alkenylgruppe,
eine C₃₋₄-Alkinylgruppe,
eine C₃₋₆-Cycloalkyl- oder C₃₋₆-Cycloalkyl-C₁₋₃-alkyl-Gruppe,
eine Phenylgruppe, die durch R¹⁰ und R¹¹ substituiert ist, wobei R¹⁰ und R¹¹ wie vorstehend erwähnt definiert sind,
eine Phenyl-C₁₋₃-alkyl-Gruppe, in der der Phenylteil durch R¹⁰ und R¹¹ substituiert ist, wobei R¹⁰ und R¹¹ wie vorstehend erwähnt definiert sind,
eine Phenyl-C₂₋₃-alkenyl-Gruppe, in der der Phenylteil durch R¹⁰ und R¹¹ substituiert ist, wobei R¹⁰ und R¹¹ wie vorstehend erwähnt definiert sind,
eine Phenylcarbonylmethylgruppe, in der der Phenylteil durch R¹⁰ und R¹¹ substituiert ist, wobei R¹⁰ und R¹¹ wie vorstehend erwähnt definiert sind,
eine Furanyl-, Thienyl- oder Pyridylgruppe,
eine Furanyl-C₁₋₃-alkyl-, Thienyl-C₁₋₃-alkyl- oder Pyridyl-C₁₋₃-alkyl-Gruppe,
eine Cyanogruppe,
eine Amino-, C₁₋₄-Alkylamino- oder Di-(C₁₋₄-alkyl)-aminogruppe,
eine durch die Reste R¹⁵ und R¹⁶ substituierte Aminogruppe, in der
R¹⁵ ein Wasserstoffatom oder eine Methyl- oder Ethylgruppe und
R¹⁶ eine C₁₋₄-Alkylgruppe, die durch eine Cyan-, Carboxy-, Methoxycarbonyl-, Ethyloxycarbonyl-, Aminocarbonyl-, Methylaminocarbonyl-, Dimethylaminocarbonyl-, Ethylaminocarbonyl-, Diethylaminocarbonyl-, Pyrrolidin-1-yl-carbonyl- oder Morpholin-4-ylcarbonyl-Gruppe substituiert ist, bedeuten,
eine durch die Reste R¹⁵ und R¹⁷ substituierte Aminogruppe, in der
R¹⁵ wie vorstehend erwähnt definiert ist und
R¹⁷ eine geradkettige C₂₋₄-Alkylgruppe, die jeweils endständig durch eine Amino-, Methylamino-, Dimethylamino-, Acetylamino-, Ethyloxycarbonylamino-, Phenylcarbonylamino-, Methylsulfonylamino-, Phenylsulfonylamino-, Hydroxy-, Methoxy-, Phenyloxy-, Methylsulfanyl- oder Phenylsulfanyl-Gruppe substituiert ist, bedeutet,
eine Pyrrolidin-1yl-, Piperidin-1-yl-, Morpholin-4-yl-, Piperazin-1-yl- oder 4-Methylpiperazin-1-yl-Gruppe,
eine C₃₋₆-Cycloalkylamino- oder C₃₋₆-Cycloalkyl-C₁₋₃-alkylamino-Gruppe,
eine Phenylaminogruppe,
eine Phenyl-C₁₋₃-alkylaminogruppe, in der der Phenylteil durch R¹⁰ und R¹¹ substituiert ist, wobei R¹⁰ und R¹¹ wie vorstehend erwähnt definiert sind,
eine Naphthylmethylaminogruppe,
eine Heteroaryl-C₁₋₂-alkylaminogruppe, wobei der Begriff Heteroaryl wie vorstehend erwähnt definiert ist, oder
eine Methylsulfanyl-, Benzylsulfanyl- oder (2-Phenylethyl)sulfanyl-gruppe,
R³ eine C₄₋₆-Alkenylgruppe,
eine C₃₋₄-Alkenylgruppe, die durch ein Fluor- Chlor- oder Bromatom oder eine Trifluormethylgruppe substituiert ist,
eine 2-Butin-1-ylgruppe oder
eine durch die Gruppe R_{c} substituierte Methylgruppe, wobei
R_{c} eine 1-Cyclopenten-1-yl- oder 1-Cyclohexen-1-yl- -Gruppe,
eine gegebenenfalls durch ein Fluor-, Chlor-, Brom- oder lodatom, durch eine Methyl-, Trifluormethyl-, Cyan-, Methoxy-, Difluormethoxy- oder Trifluormethoxygruppe substituierte Phenylgruppe,
eine Phenylgruppe, die durch zwei Fluoratome substituiert ist,
eine Naphthylgruppe oder
eine Furanyl-, Thienyl-, oder Pyridylgruppe bedeutet,
und
R⁴ eine Piperidin-1-ylgruppe, die in 3-Stellung durch eine Aminogruppe substituiert ist,
eine Hexahydroazepin-1-yl-gruppe, die in 3-Stellung oder in 4-Stellung durch eine Aminogruppe substituiert ist,
eine (2-Aminocyclohexyl)amino-Gruppe,
eine-Cyclohexylgruppe, die in 3-Stellung durch eine Aminogruppe substituiert ist, oder
eine N-(2-Aminoethyl)-methylamino- oder eine N-(2-Aminoethyl)-ethylamino-Gruppe bedeuten,
wobei soweit nichts anderes erwähnt wurde, die vorstehend erwähnten Alkyl-, Alkenyl- und Alkinylgruppen geradkettig oder verzweigt sein können,
deren Tautomere, Enantiomere, Diastereomere, deren Gemische und deren Salze.

4. Purinderivate der allgemeinen Formel I gemäß Anspruch 3, in der
R¹ ein Wasserstoffatom,
eine Methyl-, Benzyl- oder 2-Phenylethylgruppe,
eine Naphthylmethyl- oder Methoxynaphthylmethylgruppe oder
eine Phenylcarbonylmethylgruppe,
R² ein Wasserstoffatom,
eine Methyl- oder 2-Phenylethylgruppe,
eine Phenylcarbonylmethyl-Gruppe,
eine Cyanogruppe,
eine Amino-, Methylamino-, Dimethylamino-, Isopropylamino-, Cyclohexylamino- oder (Cyclohexylmethyl)amino-Gruppe,
eine Benzylamino-, Fluorbenzylamino- oder (2-Phenylethyl)amino-Gruppe oder
eine Piperidin-1-ylgruppe,
R³ eine Benzyl- oder 3-Methyl-but-2-en-1-yl-Gruppe
und
R⁴ eine (3-Amino-piperidin-1-yl)-Gruppe bedeuten,
deren Tautomere, Enantiomere, Diastereomere, deren Gemische und deren Salze.

5. Folgende Verbindungen der allgemeinen Formel I gemäß Anspruch 1:
(1) 8-(3-Amino-piperidin-1-yl)-7-benzyl-2-benzylamino-1-methyl-1,7-dihydro-purin-6-on,
(2) 8-(3-Amino-piperidin-1-yl)-7-benzyl-2-(4-fluor-benzylamino)-1-methyl-1,7-dihydro-purin-6-on,
(3) 8-(3-Amino-piperidin-1-yl)-7-benzyl-1-methyl-2-[(2-phenylethyl)amino]-1,7-dihydro-purin-6-on,
(4) 8-(3-Amino-piperidin-1-yl)-7-benzyl-2-isopropylamino-1-methyl-1,7-dihydro-purin-6-on,
(5) 8-(3-Amino-piperidin-1-yl)-7-benzyl-1-methyl-2-methylamino-1,7-dihydro-purin-6-on,
(6) 8-(3-Amino-piperidin-1-yl)-7-benzyl-2-cyclohexylamino-1-methyl-1,7-dihydro-purin-6-on,
(7) 8-(3-Amino-piperidin-1-yl)-7-benzyl-2-[(cyclohexylmethyl)amino]-1-methyl-1,7-dihydro-purin-6-on,
(8) 8-(3-Amino-piperidin-1-yl)-7-benzyl-1-methyl-2-(piperidin-1-yl)-1,7-dihydro-purin-6-on,
(9) 8-(3-Amino-piperidin-1-yl)-7-benzyl-2-dimethylamino-1-methyl-1,7-dihydro-purin-6-on,
(10) 2-Amino-8-(3-amino-piperidin-1-yl)-7-benzyl-1-methyl-1,7-dihydro-purin-6-on,
(11) 8-(3-Amino-piperidin-1-yl)-2-benzylamino-1-methyl-7-(3-methyl-but-2-en-1-yl)-1,7-dihydro-purin-6-on,
(12) 8-(3-Amino-piperidin-1-yl)-7-benzyl-1-methyl-2-methyl-1,7-dihydro-purin-6-on,
(13) 8-(3-Amino-piperidin-1-yl)-1-methyl-7-(3-methyl-but-2-en-1-yl)-2-(2-phenylethyl)-1,7-dihydro-purin-6-on,
(14) 8-(3-Amino-piperidin-1-yl)-7-benzyl-1-methyl-1,7-dihydro-purin-6-on,
(15) 8-(3-Amino-piperidin-1-yl)-7-benzyl-1-(2-oxo-2-phenyl-ethyl)-1,7-dihydro-purin-6-on,
(16) 8-(3-Amino-piperidin-1-yl)-2-methyl-7-(3-methyl-but-2-en-1-yl)-1-[(naphthalin-1-yl)methyl]-1,7-dihydro-purin-6-on,
(17) 8-(3-Amino-piperidin-1-yl)-7-(3-methyl-but-2-en-1-yl)-1-[(naphthalin-1-yl)methyl]-1,7-dihydro-purin-6-on und
(18) 8-(3-Amino-piperidin-1-yl)-7-(3-methyl-but-2-en-1-yl)-1-[(4-methoxy-naphthalin-1-yl)methyl]-1,7-dihydro-purin-6-on
sowie deren Tautomere, Enantiomere, Diastereomere, deren Gemische und deren Salze.

6. Physiologisch verträgliche Salze der Verbindungen nach mindestens einem der Ansprüche 1 bis 5 mit anorganischen oder organischen Säuren oder Basen.

7. Arzneimittel, enthaltend eine Verbindung nach mindestens einem der Ansprüche 1 bis 5 oder ein physiologisch verträgliches Salz gemäß Anspruch 6 neben gegebenenfalls einem oder mehreren inerten Trägerstoffen und/oder Verdünnungsmitteln.

8. Verwendung einer Verbindung nach mindestens einem der Ansprüche 1 bis 6 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

9. Verfahren zur Herstellung eines Arzneimittels gemäß Anspruch 7, **dadurch gekennzeichnet, daß** auf nichtchemischen Weg eine Verbindung nach mindestens einem der Ansprüche 1 bis 6 in einen oder mehrere inerte Trägerstoffe und/oder Verdünnungsmittel eingearbeitet wird.

10. Verfahren zur Herstellung der Verbindungen der allgemeinen Formel I gemäß den Ansprüchen 1 bis 6, **dadurch gekennzeichnet, daß**
a) zur Herstellung von Verbindungen der allgemeinen Formel 1, in der R⁴ eine der in Anspruch 1 erwähnten Gruppen, die eine Imino-, Amino-, Alkylamino- oder Dialkylaminogruppen enthalten, bedeutet,
eine Verbindung der allgemeinen Formel 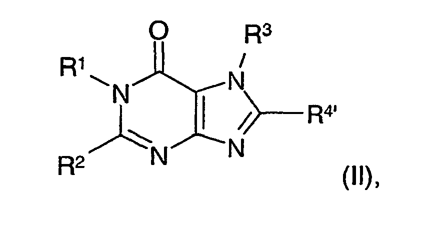 in der R¹, R² und R³ wie in Anspruch 1 erwähnt definiert sind und
R^{4'} eine der in Anspruch 1 für R⁴ erwähnten Gruppen bedeutet, die eine Imino-, Amino- oder Alkylaminogruppe enthalten, wobei die Imino-, Amino- bzw. Alkylaminogruppe durch eine Schutzgruppe wie beispielsweise eine tert.-Butyloxycarbonyl-, 2.2.2-Trichlorethoxycarbonyl- oder Carbobenzyloxycarbonylgruppe substituiert ist, entschützt und anschließend gegebenenfalls alkyliert wird
und gewünschtenfalls anschließend ein während den Umsetzungen zum Schutze von reaktiven Gruppen verwendeter Schutzrest abgespalten wird und/oder
eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure, übergeführt wird.

## Claims

1. Purine derivatives of general formula 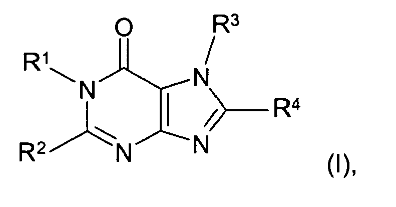 wherein
R¹ denotes a hydrogen atom,
a C₁₋₈-alkyl group,
a C₃₋₈-alkenyl group,
a C₃₋₄-alkenyl group which is substituted by a C₁₋₂-alkyloxy-carbonyl, aminocarbonyl, C₁₋₃-alkylamino-carbonyl, di-(C₁₋₃-alkyl)-amino-carbonyl, pyrrolidin-1-ylcarbonyl, piperidin-1-ylcarbonyl or morpholin-4-ylcarbonyl group,
a C₃₋₈-alkynyl group,
a C₁₋₆-alkyl group substituted by a group Rₐ, where
Rₐ denotes a C₃₋₇-cycloalkyl, heteroaryl, cyano, carboxy, C₁₋₃-alkyloxycarbonyl, aminocarbonyl, C₁₋₃-alkylamino-carbonyl, di-(C₁₋₃-alkyl)-aminocarbonyl, pyrrolidin-1-ylcarbonyl, piperidin-1-ylcarbonyl, morpholin-4-ylcarbonyl, piperazin-1-ylcarbonyl, 4-methylpiperazin-1-ylcarbonyl or 4-ethylpiperazin-1-ylcarbonyl group,
a C₁₋₆-alkyl group substituted by a phenyl group, where the phenyl ring is substituted by the groups R¹⁰ to R¹⁴ and
R¹⁰ denotes a hydrogen atom,
a fluorine, chlorine, bromine or iodine atom,
a C₁₋₄-alkyl, hydroxy or C₁₋₄-alkyloxy group,
a nitro, amino, C₁₋₃-alkylamino, di-(C₁₋₃-alkyl)-amino, cyano-C₁₋₃-alkylamino, *N*-(cyano-C₁₋₃-alkyl)-*N*-(C₁₋₃-alkyl)-amino, C₁₋₃-alkyloxy-carbonyl-C₁₋₃-alkylamino, pyrrolidin-1-yl, piperidin-1-yl, morpholin-4-yl, piperazin-1-yl or 4-(C₁₋₃-alkyl)-piperazin-1-yl group,
a formylamino, C₁₋₃-alkyl-carbonylamino, C₃₋₆-cycloalkyl-carbonylamino, C₃₋₆-cycloalkyl-C₁₋₃-alkyl-carbonylamino, arylcarbonylamino, aryl-C₁₋₃-alkyl-carbonylamino, C₁₋₃-alkyloxy-carbonylamino, aminocarbonylamino, C₁₋₃-alkyl-aminocarbonylamino, di-(C₁₋₃-alkyl)-aminocarbonylamino, pyrrolidin-1-yl-carbonylamino, piperidin-1-yl-carbonylamino, morpholin-4-yl-carbonylamino, piperazin-1-yl-carbonylamino or 4-(C₁₋₃-alkyl)-piperazin-1-yl-carbonylamino, C₁₋₃-alkyl-sulphonylamino, bis-(C₁₋₃-alkylsulphonyl)-amino, aminosulphonylamino, C₁₋₃-alkylamino-sulphonylamino, di-(C₁₋₃-alkyl)-amino-sulphonylamino, pyrrolidin-1-yl-sulphonylamino, piperidin-1-yl-sulphonylamino, morpholin-4-yl-sulphonylamino, piperazin-1-yl-sulphonylamino or 4-(C₁₋₃-alkyl)-piperazin-1-yl-sulphonylamino, (C₁₋₃-alkylamino)-thiocarbonylamino, (C₁₋₃-alkyloxy-carbonylamino)-carbonylamino, arylsulphonylamino or aryl-C₁₋₃-alkyl-sulphonylamino group,
an *N*-(C₁₋₃-alkyl)-formylamino, *N*-(C₁₋₃-alkyl)-*N*-(C₁₋₃-alkyl-carbonyl)-amino, *N*-(C₁₋₃-alkyl)-*N*-(C₃₋₆-cycloalkyl-carbonyl)-amino, *N*-(C₁₋₃-alkyl)-*N*-(C₃₋₆-cycloalkyl-C₁₋₃-alkyl-carbonyl)-amino, *N*-(C₁₋₃-alkyl)-*N*-(arylcarbonyl)-amino, *N*-(C₁₋₃-alkyl)-*N*-(aryl-C₁₋₃-alkyl-carbonyl)-amino, *N*-(C₁₋₃-alkyl)-*N-*(C₁₋₃-alkyloxy-carbonyl)-amino, *N*-(aminocarbonyl)-*N*-(C₁₋₃-alkyl)-amino, *N*-(C₁₋₃-alkyl-aminocarbonyl)-*N*-(C₁₋₃-alkyl)-amino, *N*-[di-(C₁₋₃-alkyl)aminocarbonyl]-*N*-(C₁₋₃-alkyl)-amino, *N*-(C₁₋₃-alkyl)-*N*-(C₁₋₃-alkylsulphonyl)-amino, *N*-(C₁₋₃-alkyl)-*N*-(arylsulphonyl)-amino or *N*-(C₁₋₃-alkyl)-*N*-(aryl-C₁₋₃-alkyl-sulphonyl)-amino group,
a 2-oxo-imidazolidin-1-yl, 2,4-dioxo-imidazolidin-1-yl, 2,5-dioxo-imidazolidin-1-yl or 2-oxo-hexahydropyrimidin-1-yl group wherein the nitrogen atom in the 3 position may be substituted in each case by a methyl or ethyl group,
a cyano, carboxy, C₁₋₃-alkyloxy-carbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl or 4-(C₁₋₃-alkyl)-piperazin-1-yl-carbonyl group,
a C₁₋₃-alkyl-carbonyl or an arylcarbonyl group,
a carboxy-C₁₋₃-alkyl, C₁₋₃-alkyloxy-carbonyl-C₁₋₃-alkyl, cyano-C₁₋₃-alkyl, aminocarbonyl-C₁₋₃-alkyl, C₁₋₃-alkyl-aminocarbonyl-C₁₋₃-alkyl, di-(C₁₋₃-alkyl)-aminocarbonyl-C₁₋₃-alkyl, pyrrolidin-1-yl-carbonyl-C₁₋₃-alkyl, piperidin-1-yl-carbonyl-C₁₋₃-alkyl, morpholin-4-yl-carbonyl-C₁₋₃-alkyl, piperazin-1-yl-carbonyl-C₁₋₃-alkyl or 4-(C₁₋₃-alkyl)-piperazin-1-yl-carbonyl-C₁₋₃-alkyl group,
a carboxy-C₁₋₃-alkyloxy, C₁₋₃-alkyloxy-carbonyl-C₁₋₃-alkyloxy, cyano-C₁₋₃-alkyloxy, aminocarbonyl-C₁₋₃-alkyloxy, C₁₋₃-alkyl-aminocarbonyl-C₁₋₃-alkyloxy, di-(C₁₋₃-alkyl)-aminocarbonyl-C₁₋₃-alkyloxy, pyrrolidin-1-yl-carbonyl-C₁₋₃-alkyloxy, piperidin-1-yl-carbonyl-C₁₋₃-alkyloxy, morpholin-4-yl-carbonyl-C₁₋₃-alkyloxy, piperazin-1-yl-carbonyl-C₁₋₃-alkyloxy or 4-(C₁₋₃-alkyl)-piperazin-1-yl-carbonyl-C₁₋₃-alkyloxy group,
a hydroxy-C₁₋₃-alkyl, C₁₋₃-alkyloxy-C₁₋₃-alkyl, amino-C₁₋₃-alkyl, C₁₋₃-alkylamino-C₁₋₃-alkyl, di-(C₁₋₃-alkyl)-amino-C₁₋₃-alkyl, pyrrolidin-1-yl-C₁₋₃-alkyl, piperidin-1-yl-C₁₋₃-alkyl, morpholin-4-yl-C₁₋₃-alkyl, piperazin-1-yl-C₁₋₃-alkyl, 4-(C₁₋₃-alkyl)-piperazin-1-yl-C₁₋₃-alkyl group,
a hydroxy-C₁₋₃-alkyloxy, C₁₋₃-alkyloxy-C₁₋₃-alkyloxy, C₁₋₃-alkylsulphanyl-C₁₋₃-alkyloxy, C₁₋₃-alkylsulphinyl-C₁₋₃-alkyloxy, C₁₋₃-alkylsulphonyl-C₁₋₃-alkyloxy, amino-C₁₋₃-alkyloxy, C₁₋₃-alkylamino-C₁₋₃-alkyloxy, di-(C₁₋₃-alkyl)-amino-C₁₋₃-alkyloxy, pyrrolidin-1-yl-C₁₋₃-alkyloxy, piperidin-1-yl-C₁₋₃-alkyloxy, morpholin-4-yl-C₁₋₃-alkyloxy, piperazin-1-yl-C₁₋₃-alkyloxy, 4-(C₁₋₃-alkyl)-piperazin-1-yl-C₁₋₃-alkyloxy group,
a mercapto, C₁₋₃-alkylsulphanyl, C₁₋₃-alkysulphinyl, C₁₋₃-alkylsulphonyl, C₁₋₃-alkylsulphonyloxy, arylsulphonyloxy, trifluoromethylsulphanyl, trifluoromethylsulphinyl or trifluoromethylsulphonyl group,
a sulpho, aminosulphonyl, C₁₋₃-alkyl-aminosulphonyl, di-(C₁₋₃-alkyl)-aminosulphonyl, pyrrolidin-1-yl-sulphonyl, piperidin-1-yl-sulphonyl, morpholin-4-yl-sulphonyl, piperazin-1-yl-sulphonyl or 4-(C₁₋₃-alkyl)-piperazin-1-yl-sulphonyl group,
a methyl or methoxy group substituted by 1 to 3 fluorine atoms,
an ethyl or ethyloxy group substituted by 1 to 5 fluorine atoms,
a C₂₋₄-alkenyl or C₂₋₄-alkynyl group,
a C₃₋₄-alkenyloxy or C₃₋₄-alkynyloxy group,
a C₃₋₆-cycloalkyl or C₃₋₆-cycloalkyloxy group,
a C₃₋₆-cycloalkyl-C₁₋₃-alkyl or C₃₋₆-cycloalkyl-C₁₋₃-alkyloxy group or
an aryl, aryloxy, aryl-C₁₋₃-alkyl or aryl-C₁₋₃-alkyloxy group,
R¹¹ and R¹², which may be identical or different, in each case represent a hydrogen atom, a fluorine, chlorine, bromine or iodine atom, a C₁₋₃-alkyl, trifluoromethyl, hydroxy, C₁₋₃-alkyloxy or cyano group, or
R¹¹ together with R¹², if they are bound to adjacent carbon atoms, also represent a methylenedioxy, difluoromethylenedioxy or a straight-chain C₃₋₅-alkylene group and
R¹³ and R¹⁴, which may be identical or different, in each case represent a hydrogen atom, a fluorine, chlorine or bromine atom, a trifluoromethyl, C₁₋₃-alkyl or C₁₋₃-alkyloxy group,
a phenyl-C₁₋₄-alkyl group wherein the alkyl moiety is substituted by a cyano, carboxy, C₁₋₃-alkyloxy-carbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl group and the phenyl moiety is substituted by the groups R¹⁰ to R¹⁴, while R¹⁰ to R¹⁴ are as hereinbefore defined,
a phenyl group substituted by the groups R¹⁰ to R¹⁴, where R¹⁰ to R¹⁴ are as hereinbefore defined,
a phenyl-C₂₋₃-alkenyl group wherein the phenyl moiety is substituted by the groups R¹⁰ to R¹⁴, where R¹⁰ to R¹⁴ are as hereinbefore defined,
a phenyl-(CH₂)ₘ-A-(CH₂)ₙ group wherein the phenyl moiety is substituted by R¹⁰ to R¹⁴, where R¹⁰ to R¹⁴ are as hereinbefore defined and
A represents a carbonyl group, m represents the number 0, 1 or 2 and
n represents the number 1, 2 or 3,
a phenylcarbonylmethyl group wherein the phenyl moiety is substituted by R¹⁰ to R¹⁴, where R¹⁰ to R¹⁴ are as hereinbefore defined and the methyl moiety is substituted by a C₁₋₃-alkyl group,
a phenyl-(CH₂)ₘ-B-(CH₂)ₙ group wherein the phenyl moiety is substituted by R¹⁰ to R¹⁴, where R¹⁰ to R¹⁴, m and n are as hereinbefore defined and
B denotes a methylene group which is substituted by a hydroxy, C₁₋₃-alkyloxy, amino, C₁₋₃-alkylamino, di-(C₁₋₃-alkyl)-amino, mercapto, C₁₋₃-alkylsulphanyl, C₁₋₃-alkylsulphinyl or C₁₋₃-alkylsulphonyl group and is optionally additionally substituted by a methyl or ethyl group,
a naphthyl-C₁₋₃-alkyl group wherein the naphthyl moiety is substituted by the groups R¹⁰ to R¹⁴, where R¹⁰ to R¹⁴ are as hereinbefore defined,
a naphthyl-(CH₂)ₘ-A-(CH₂)ₙ group wherein the naphthyl moiety is substituted by R¹⁰ to R¹⁴, where R¹⁰ to R¹⁴, A, m and n are as hereinbefore defined,
a naphthyl-(CH₂)ₘ-B-(CH₂)ₙ group wherein the naphthyl moiety is substituted by R¹⁰ to R¹⁴, where R¹⁰ to R¹⁴, B, m and n are as hereinbefore defined,
a [1,4]naphthoquinon-2-yl, chromen-4-on-3-yl, 1-oxoindan-2-yl, 1,3-dioxo-indan-2-yl or 2,3-dihydro-3-oxo-benzofuran-2-yl group,
a heteroaryl-(CH₂)ₘ-A-(CH₂)ₙ group where A, m and n are as hereinbefore defined,
a heteroaryl-(CH₂)ₘ-B-(CH₂)ₙ group where B, m and n are as hereinbefore defined,
a C₁₋₆-alkyl-A-(CH₂)ₙ group where A and n are as hereinbefore defined,
a C₃₋₇-cycloalkyl-(CH₂)ₘ-A-(CH₂)ₙ group where A, m and n are as hereinbefore defined,
a C₃₋₇-cycloalkyl-(CH₂)ₘ-B-(CH₂)ₙ group where B, m and n are as hereinbefore defined,
an R²¹-A-(CH₂)ₙ group wherein R²¹ denotes a C₁₋₃-alkyloxycarbonyl, aminocarbonyl, C₁₋₃-alkylaminocarbonyl, di-(C₁₋₃-alkyl)aminocarbonyl, pyrrolidin-1-yl-carbonyl, piperidin-1-yl-carbonyl or morpholin-4-yl-carbonyl, piperazin-1-yl-carbonyl, 4-methylpiperazin-1-yl-carbonyl or 4-ethylpiperazin-1-yl-carbonyl group and A and n are as hereinbefore defined,
a phenyl-(CH₂)ₘ-D-C₁₋₃-alkyl group wherein the phenyl moiety is substituted by the groups R¹⁰ to R¹⁴, where R¹⁰ to R¹⁴ and m are as mentioned hereinbefore and D denotes an oxygen or sulphur atom, an imino, C₁₋₃-alkylimino, sulphinyl or sulphonyl group,
a naphthyl-(CH₂)ₘ-D-C₁₋₃-alkyl group wherein the naphthyl moiety is substituted by the groups R¹⁰ to R¹⁴, where R¹⁰ to R¹⁴, D and m are as mentioned hereinbefore,
a C₂₋₆-alkyl group substituted by a group R_{b}, where
R_{b} is isolated from the cyclic nitrogen atom in the 1 position of the purine skeleton by at least two carbon atoms and
R_{b} denotes a hydroxy, C₁₋₃-alkyloxy, mercapto, C₁₋₃-alkylsulphanyl, C₁₋₃-alkylsulphinyl, C₁₋₃-alkylsulphonyl, amino, C₁₋₃-alkyl-carbonylamino, C₃₋₆-cycloalkyl-carbonyl-amino, arylcarbonylamino, C₁₋₃-alkylamino, di-(C₁₋₃-alkyl)-amino, pyrrolidin-1-yl, piperidin-1-yl, morpholin-4-yl, piperazin-1-yl or 4-(C₁₋₃-alkyl)-piperazin-1-yl group,
a C₃₋₆-cycloalkyl group,
or an amino or arylcarbonylamino group,
R² denotes a hydrogen atom,
a C₁₋₈-alkyl group,
a C₃₋₈-alkenyl group,
a C₃₋₄-alkenyl group which is substituted by a C₁₋₂-alkyloxy-carbonyl, aminocarbonyl, C₁₋₃-alkylamino-carbonyl, di-(C₁₋₃-alkyl)-amino-carbonyl, pyrrolidin-1-ylcarbonyl, piperidin-1-ylcarbonyl or morpholin-4-ylcarbonyl group, a C₃₋₈-alkynyl group,
a C₃₋₆-cycloalkyl group,
a C₁₋₆-alkyl group substituted by a group Rₐ, where Rₐ is as hereinbefore defined,
a phenyl group which is substituted by R¹⁰ to R¹⁴, where R¹⁰ to R¹⁴ are as hereinbefore defined,
a C₁₋₆-alkyl group substituted by a phenyl group, wherein the phenyl ring is substituted by the groups R¹⁰ to R¹⁴ and R¹⁰ to R¹⁴ are as hereinbefore defined,
a phenyl-C₁₋₄-alkyl group wherein the alkyl moiety is substituted by a cyano, carboxy, C₁₋₃-alkyloxy-carbonyl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl group and the phenyl moiety is substituted by the groups R¹⁰ to R¹⁴, where R¹⁰ to R¹⁴ are as hereinbefore defined,
a phenyl-C₂₋₃-alkenyl group wherein the phenyl moiety is substituted by R¹⁰ to R¹⁴, where R¹⁰ to R¹⁴ are as hereinbefore defined,
a heteroaryl group,
a phenyl-(CH₂)ₘ-A or phenyl-(CH₂)ₘ-A-(CH₂)ₙ group wherein the phenyl moiety is substituted in each case by R¹⁰ to R¹⁴, while A, R¹⁰ to R¹⁴, m and n are as hereinbefore defined,
a phenylcarbonylmethyl group wherein the phenyl moiety is substituted by R¹⁰ to R¹⁴, where R¹⁰ to R¹⁴ are as hereinbefore defined and the methyl moiety is substituted by a C₁₋₃-alkyl group,
a phenyl-(CH₂)ₘ-B or phenyl-(CH₂)ₘ-B-(CH₂)ₙ group wherein the phenyl moiety is substituted in each case by R ° to R¹⁴, wherein B, R¹⁰ to R¹⁴, m and n are as hereinbefore defined,
a naphthyl-C₁₋₃-alkyl group wherein the naphthyl moiety is substituted by the groups R¹⁰ to R¹⁴, where R¹⁰ to R¹⁴ are as hereinbefore defined, a naphthyl-(CH₂)ₘ-A or naphthyl-(CH₂)ₘ-A-(CH₂)ₙ group wherein the naphthyl moiety is substituted in each case by R¹⁰ to R¹⁴, where R¹⁰ to R¹⁴, A, m and n are as hereinbefore defined,
a naphthyl-(CH₂)ₘ-B or naphthyl-(CH₂)ₘ-B-(CH₂)ₙ group wherein the naphthyl moiety is substituted in each case by R¹⁰ to R¹⁴, where R¹⁰ to R¹⁴, B, m and n are as hereinbefore defined,
a heteroaryl-(CH₂)ₘ-A or heteroaryl-(CH₂)ₘ-A-(CH₂)ₙ group where A, m and n are as hereinbefore defined,
a heteroaryl-(CH₂)ₘ-B or heteroaryl-(CH₂)ₘ-B-(CH₂)ₙ group where B, m and n are as hereinbefore defined,
a C₁₋₆-alkyl-A or C₁₋₆-alkyl-A-(CH₂)ₙ group where A and n are as hereinbefore defined,
a C₃₋₇-cycloalkyl-(CH₂)ₘ-A or C₃₋₇-cycloalkyl-(CH₂)ₘ-A-(CH₂)ₙ group where A, m and n are as hereinbefore defined,
a C₃₋₇-cydoalkyl-(CH₂)ₘ-B or C₃₋₇-cydoalkyl-(CH₂)ₘ-B-(CH₂)ₙ group where B, m and n are as hereinbefore defined,
an R²¹-A-(CH₂)ₙ group wherein R²¹, A and n are as hereinbefore defined,
a phenyl-(CH₂)ₘ-D-C₁₋₃-alkyl group wherein the phenyl moiety is substituted by the groups R¹⁰ to R¹⁴, where R¹⁰ to R¹⁴, D and m are as mentioned hereinbefore,
a naphthyl-(CH₂)ₘ-D-C₁₋₃-alkyl group wherein the naphthyl moiety is substituted by the groups R¹⁰ to R¹⁴, where R¹⁰ to R¹⁴, D and m are as mentioned hereinbefore,
a C₁₋₆-alkyl group substituted by a group R_{b}, where R_{b} is as hereinbefore defined,
a cyano, carboxy, C₁₋₃-alkyloxy-carbonyl, aminocarbonyl, C₁₋₃-alkylaminocarbonyl, di-(C₁₋₃-alkyl)-amino-carbonyl, pyrrolidin-1-ylcarbonyl, piperidin-1-ylcarbonyl, morpholin-4-ylcarbonyl, piperazin-1-ylcarbonyl, 4-methylpiperazin-1-ylcarbonyl or 4-ethylpiperazin-1-ylcarbonyl group,
an amino, C₁₋₆-alkylamino or di-(C₁₋₆-alkyl)-amino group,
an amino group substituted by the groups R¹⁵ and R¹⁶ wherein
R¹⁵ denotes a hydrogen atom or a C₁₋₆-alkyl group and
R¹⁶ denotes a C₁₋₆-alkyl group which is substituted by Rₐ, where Rₐ is as hereinbefore defined,
an amino group substituted by the groups R¹⁵ and R¹⁷ wherein
R¹⁵ is as hereinbefore defined and
R¹⁷ denotes a C₂₋₆-alkyl group which is substituted by a hydroxy, C₁₋₃-alkyloxy, aryloxy, mercapto, C₁₋₃-alkylsulphanyl, C₁₋₃-alkylsulphinyl, C₁₋₃-alkylsulphonyl, C₁₋₃-alkylsulphonylamino, arylsulphanyl, arylsulphinyl, arylsulphonyl, arylsulphonylamino, C₁₋₃-alkylcarbonylamino, C₃₋₆-cycloalkyl-carbonylamino, arylcarbonylamino, C₁₋₃-alkyl-oxycarbonylamino, aminocarbonylamino, C₁₋₃-alkyl-aminocarbonylamino, di-(C₁₋₃-alkyl)-aminocarbonylamino, amino, C₁₋₃-alkylamino, di-(C₁₋₃-alkyl)-amino, pyrrolidin-1-yl, piperidin-1-yl, morpholin-4-yl, piperazin-1-yl or 4-(C₁₋₃-alkyl)-piperazin-1-yl group,
a C₃₋₆-cycloalkylamino or *N*-(C₃₋₆₋cycloalkyl)-*N*-(C₁₋₃-alkyl)-amino group,
a phenylamino or *N*-(phenyl)-*N*-(C₁₋₃-alkyl)-amino group wherein the phenyl moiety is substituted in each case by R¹⁰ to R¹⁴, where R¹⁰ to R¹⁴ are as hereinbefore defined,
a phenyl-C₁₋₆-alkylamino or *N*-(phenyl-C₁₋₆-alkyl)-*N*-(C₁₋₃-alkyl)-amino group wherein the phenyl moiety is substituted in each case by R¹⁰ to R¹⁴, where R¹⁰ to R¹⁴ are as hereinbefore defined,
a naphthylamino or *N*-(naphthyl)-*N*-(C₁₋₃-alkyl)-amino group,
a naphthyl-C₁₋₆-alkylamino or *N*-(naphthyl-C₁₋₆-alkyl)-*N*-(C₁₋₃-alkyl)-amino group,
a heteroarylamino or *N*-(heteroaryl)-*N*-(C₁₋₃-alkyl)-amino group,
a pyrrolidin-1-yl, piperidin-1-yl, homopiperidin-1-yl, morpholin-4-yl, homomorpholin-4-yl, piperazin-1-yl, 4-(C₁₋₃-alkyl)-piperazin-1-yl, homopiperazin-1-yl or 4-(C₁₋₃-alkyl)-homopiperazin-1-yl group, or
a C₁₋₆-alkyloxy, C₃₋₆-cydoalkyloxy or C₃₋₆-cycloalkyl-C₁₋₆-alkyloxy group,
a C₁₋₆-alkylsulphanyl, C₃₋₆-cycloalkylsulphanyl or C₃₋₆-cydoalkyl-C₁₋₆-alkylsulphanyl group,
a phenyloxy or phenylsulphanyl group wherein the phenyl moiety is substituted in each case by R¹⁰ to R¹⁴, where R¹⁰ to R¹⁴ are as hereinbefore defined,
a phenyl-C₁₋₆-alkyloxy or phenyl-C₁₋₆-alkylsulphanyl group wherein the phenyl moiety is substituted in each case by R¹⁰ to R¹⁴, where R¹⁰ to R¹⁴ are as hereinbefore defined,
a naphthyloxy or a naphthylsulphanyl group wherein the naphthyl moiety is substituted in each case by R¹⁰ to R¹⁴, where R¹⁰ to R¹⁴ are as hereinbefore defined,
a naphthyl-C₁₋₆-alkyloxy or naphthyl-C₁₋₆-alkylsulphanyl group wherein the naphthyl moiety is substituted in each case by R¹⁰ to R¹⁴, where R¹⁰ to R¹⁴ are as hereinbefore defined,
a heteroaryloxy or heteroarylsulphanyl group or
a heteroaryi-C₁₋₆-alkyloxy or heteroaryl-C₁₋₆-alkylsulphanyl group,
R³ denotes a C₁₋₈-alkyl group,
a C₁₋₄-alkyl group substituted by the group R_{c}, where
R_{c} denotes a C₃₋₇-cycloalkyl group optionally substituted by one or two C₁₋₃-alkyl groups,
a C₅₋₇-cycloalkenyl group optionally substituted by one or two C₁₋₃-alkyl groups,
an aryl group or
a furanyl, thienyl, oxazolyl, isoxazolyl, thiazolyl, isothiazolyl, pyridyl, pyridazinyl, pyrimidyl or pyrazinyl group, while the above-mentioned heterocyclic groups may each be substituted by one or two C₁₋₃-alkyl groups or by a fluorine, chlorine, bromine or iodine atom or by a trifluoromethyl, cyano or C₁₋₃-alkyloxy group,
a C₃₋₈-alkenyl group,
a C₃₋₆-alkenyl group substituted by a fluorine, chlorine or bromine atom or a trifluoromethyl group,
a C₃₋₈-alkynyl group,
an aryl group or
an aryl-C₂₋₄-alkenyl group,
and
R⁴ denotes an azetidin-1-yl or pyrrolidin-1-yl group which is substituted in the 3 position by an amino, C₁₋₃-alkylamino or di-(C₁₋₃-alkyl)-amino group and may additionally be substituted by one or two C₁₋₃-alkyl groups,
a piperidin-1-yl or hexahydroazepin-1-yl group which is substituted in the 3 position or in the 4 position by an amino, C₁₋₃-alkylamino or di-(C₁₋₃-alkyl)amino group and may additionally be substituted by one or two C₁₋₃-alkyl groups,
a 3-amino-piperidin-1-yl group wherein the piperidin-1-yl-moiety is additionally substituted by an aminocarbonyl, C₁₋₂-alkyl-aminocarbonyl, di-(C₁₋₂-alkyl)-aminocarbonyl, pyrrolidin-1-yl-carbonyl, (2-cyano-pyrrolidin-1-yl-)carbonyl, thiazolidin-3-yl-carbonyl, (4-cyano-thiazolidin-3-yl)carbonyl, piperidin-1-ylcarbonyl or morpholin-4-ylcarbonyl group,
a 3-amino-piperidin-1-yl group wherein the piperidin-1-yl moiety is additionally substituted in the 4 position or 5 position by a hydroxy or methoxy group,
a 3-amino-piperidin-1-yl group wherein the methylene group is replaced in the 2 position or 6 position by a carbonyl group,
a piperidin-1-yl or hexahydroazepin-1-yl group substituted in the 3 position by an amino, C₁₋₃-alkylamino or di-(C₁₋₃-alkyl)-amino group, wherein two hydrogen atoms on the carbon skeleton of the piperidin-1-yl or hexahydroazepin-1-yl group are each replaced by a straight-chain alkylene bridge, this bridge containing 2 to 5 carbon atoms if the two hydrogen atoms are on the same carbon atom, or 1 to 4 carbon atoms if the hydrogen atoms are on adjacent carbon atoms, or 1 to 4 carbon atoms if the hydrogen atoms are on carbon atoms which are separated by one atom, or 1 to 3 carbon atoms if the two hydrogen atoms are on carbon atoms which are separated by two atoms,
an azetidin-1-yl, pyrrolidin-1-yl, piperidin-1-yl or hexahydroazepin-1-yl group which is substituted by an amino-C₁₋₃-alkyl, C₁₋₃-alkylamino-C₁₋₃-alkyl or a di-(C₁₋₃-alkyl)amino-C₁₋₃-alkyl group,
a piperazin-1-yl or [1,4]diazepan-1-yl group optionally substituted on the carbon skeleton by one or two C₁₋₃-alkyl groups,
a 3-imino-piperazin-1-yl, 3-imino-[1,4]diazepan-1-yl or 5-imino-[1,4]diazepan-1-yl group optionally substituted on the carbon skeleton by one or two C₁₋₃-alkyl groups,
a [1,4]diazepan-1-yl group optionally substituted by one or two C₁₋₃-alkyl groups, which is substituted in the 6 position by an amino group,
a C₃₋₇-cycloalkyl group which is substituted by an amino, C₁₋₃-alkylamino or di-(C₁₋₃-alkyl)-amino group,
a C₃₋₇-cycloalkyl group which is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkyl-C₁₋₂-alkyl group wherein the cycloalkyl moiety is substituted by an amino, C₁₋₃-alkylamino or di-(C₁₋₃-alkyl)-amino group,
a C₃₋₇-cycloalkyl-C₁₋₂-alkyl group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkylamino group wherein the cycloalkyl moiety is substituted by an amino, C₁₋₃-alkylamino or di-(C₁₋₃-alkyl)-amino group, wherein the two nitrogen atoms on the cycloalkyl moiety are separated from one another by at least two carbon atoms,
an *N*-(C₃₋₇-cycloalkyl)-*N*-(C₁₋₃-alkyl)-amino group wherein the cycloalkyl moiety is substituted by an amino, C₁₋₃-alkylamino or di-(C₁₋₃-alkyl)-amino group, wherein the two nitrogen atoms on the cycloalkyl moiety are separated from one another by at least two carbon atoms,
a C₃₋₇-cycloalkylamino group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
an *N*-(C₃₋₇-cycloalkyl)-*N*-(C₁₋₃-alkyl)-amino group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
a C₃₋₇-cycloalkyl-C₁₋₂-alkyl-amino group wherein the cycloalkyl moiety is substituted by an amino, C₁₋₃-alkylamino or di-(C₁₋₃-alkyl)-amino group,
an *N*-(C₃₋₇-cycloalkyl-C₁₋₂-alkyl)-*N*-(C₁₋₂-alkyl)-amino group wherein the cycloalkyl moiety is substituted by an amino, C₁₋₃-alkylamino or di-(C₁₋₃-alkyl)-amino group,
a C₃₋₇-cycloalkyl-C₁₋₂-alkyl-amino group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
an *N*-(C₃₋₇cycloalkyl-C₁₋₂-alkyl)-*N*-(C₁₋₂-alkyl)-amino group wherein the cycloalkyl moiety is substituted by an amino-C₁₋₃-alkyl, C₁₋₃-alkylamino-C₁₋₃-alkyl or a di-(C₁₋₃-alkyl)amino-C₁₋₃-alkyl group,
an R¹⁹-C₂₋₄-alkylamino group wherein R¹⁹ is separated from the nitrogen atom of the C₂₋₄-alkylamino moiety by at least two carbon atoms and
R¹⁹ denotes an amino, C₁₋₃-alkylamino or di-(C₁₋₃-alkyl)-amino group,
an R¹⁹-C₂₋₄-alkylamino group wherein the nitrogen atom of the C₂₋₄-alkylamino moiety is substituted by a C₁₋₃-alkyl group and R¹⁹ is separated from the nitrogen atom of the C₂₋₄-alkylamino moiety by at least two carbon atoms, where R¹⁹ is as hereinbefore defined,
an amino group substituted by the group R²⁰ wherein
R²⁰ denotes an azetidin-3-yl, azetidin-2-ylmethyl, azetidin-3-ylmethyl, pyrrolidin-3-yl, pyrrolidin-2-ylmethyl, pyrrolidin-3-ylmethyl, piperidin-3-yl, piperidin-4-yl, piperidin-2-ylmethyl, piperidin-3-ylmethyl or piperidin-4-ylmethyl group, while the groups mentioned for R²⁰ may each be substituted by one or two C₁₋₃-alkyl groups,
an amino group substituted by the group R²⁰ and a C₁₋₃-alkyl group wherein R²⁰ is as hereinbefore defined, wherein the groups mentioned for R²⁰ may each be substituted by one or two C₁₋₃-alkyl groups,
an R¹⁹-C₃₋₄-alkyl group wherein the C₃₋₄-alkyl moiety is straight-chained and may additionally be substituted by one or two C₁₋₃-alkyl groups, where R¹⁹ is as hereinbefore defined,
a 3-amino-2-oxo-piperidin-5-yl or 3-amino-2-oxo-1-methyl-piperidin-5-yl group,
a pyrrolidin-3-yl, piperidin-3-yl, piperidin-4-yl, hexahydroazepin-3-yl or hexahydroazepin-4-yl group which is substituted in the 1 position by an amino, C₁₋₃-alkylamino or di-(C₁₋₃-alkyl)amino group,
or an azetidin-2-yl-C₁₋₂-alkyl, azetidin-3-yl-C₁₋₂-alkyl, pyrrolidin-2-yl-C₁₋₂-alkyl, pyrrolidin-3-yl, pyrrolidin-3-yl-C₁₋₂-alkyl, piperidin-2-yl-C₁₋₂-alkyl, piperidin-3-yl, piperidin-3-yl-C₁₋₂-alkyl, piperidin-4-yl or piperidin-4-yl-C₁₋₂-alkyl group, wherein the above-mentioned groups may each be substituted by one or two C₁₋₃-alkyl groups,
while by the aryl groups mentioned in the definition of the above groups are meant phenyl or naphthyl groups, which may be mono- or disubstituted by Rₕ independently of one another, where the substituents are identical or different and Rₕ denotes a fluorine, chlorine, bromine or iodine atom, a trifluoromethyl, cyano, nitro, amino, aminocarbonyl, aminosulphonyl, methylsulphonyl, acetylamino, methylsulphonylamino, C₁₋₃-alkyl, cyclopropyl, ethenyl, ethynyl, hydroxy, C₁₋₃-alkyloxy, difluoromethoxy or trifluoromethoxy group,
by the heteroaryl groups mentioned in the definitions of the above-mentioned groups are meant a pyrrolyl, furanyl, thienyl, pyridyl, indolyl, benzofuranyl, benzothiophenyl, quinolinyl or isoquinolinyl group,
or a pyrrolyl, furanyl, thienyl or pyridyl group wherein one or two methyne groups are replaced by nitrogen atoms,
or an indolyl, benzofuranyl, benzothiophenyl, quinolinyl or isoquinolinyl group wherein one to three methyne groups are replaced by nitrogen atoms,
or a 1,2-dihydro-2-oxo-pyridinyl, 1,4-dihydro-4-oxo-pyridinyl, 2,3-dihydro-3-oxo-pyridazinyl, 1,2,3,6-tetrahydro-3,6-dioxo-pyridazinyl, 1,2-dihydro-2-oxo-pyrimidinyl, 3,4-dihydro-4-oxo-pyrimidinyl, 1,2,3,4-tetrahydro-2,4-dioxo-pyrimidinyl, 1,2-dihydro-2-oxo-pyrazinyl, 1,2,3,4-tetrahydro-2,3-dioxo-pyrazinyl, 2,3-dihydro-2-oxo-indolyl, 2,3-dihydrobenzofuranyl, 2,3-dihydro-2-oxo-1*H*-benzimidazolyl, 2,3-dihydro-2-oxo-benzoxazolyl, 1,2-dihydro-2-oxo-quinolinyl, 1,4-dihydro-4-oxo-quinolinyl, 1,2-dihydro-1-oxo-isoquinolinyl, 1,4-dihydro-4-oxo-cinnolinyl, 1,2-dihydro-2-oxo-quinazolinyl, 3,4-dihydro-4-oxo-quinazolinyl, 1,2,3,4-tetrahydro-2,4-dioxo-quinazolinyl, 1,2-dihydro-2-oxoquinoxalinyl, 1,2,3,4-tetrahydro-2,3-dioxo-quinoxalinyl, 1,2-dihydro-1-oxo-phthalazinyl, 1,2,3,4-tetrahydro-1,4-dioxo-phthalazinyl, chromanyl, cumarinyl, 2,3-dihydro-benzo[1,4]dioxinyl or 3,4-dihydro-3-oxo-2*H*-benzo[1,4]oxazinyl group,
wherein the above-mentioned heteroaryl groups may be substituted by R¹⁰ to R¹⁴, where R¹⁰ to R¹⁴ are as hereinbefore defined,
and, unless otherwise stated, the above-mentioned alkyl, alkenyl and alkynyl groups may be straight-chain or branched,
as well as the derivatives which are N-oxidised or methylated or ethylated at the cyclic nitrogen atom in the 3 position or 9 position of the hypoxanthine skeleton,
as well as the derivatives wherein the 6-oxo group of the hypoxanthine skeleton is replaced by a thioxo group,
with the proviso that the compounds
8-(piperidin-4-ylmethyl)-7-(4-fluorobenzyl)-1,7-dihydro-purin-6-one,
8-(1-methyl-piperidin-4-ylmethyl)-7-(4-fluorobenzyl)-1,7-dihydro-purin-6-one,
8-(piperidin-4-ylmethyl)-7-(4-fluorobenzyl)-1,7-dihydro-purin-6-one and
8-(1-methyl-piperidin-4-ylmethyl)-7-(4-fluorobenzyl)-1,7-dihydro-purin-6-one are excluded,
the tautomers, enantiomers, diastereomers, the mixtures thereof and the salts thereof.

2. Purine derivatives of general formula I according to claim 1,
wherein R¹, R² and R³ are defined as in claim 1 and
R⁴ denotes a pyrrolidin-1-yl group which is substituted in the 3 position by an amino group,
a piperidin-1-yl group which is substituted in the 3 position by an amino group,
a piperidin-3-yl or piperidin-4-yl group,
a hexahydroazepin-1-yl group which is substituted in the 3 position or 4 position by an amino group,
a piperazin-1-yl or [1,4]diazepan-1-yl group,
a (2-aminocyclohexyl)amino group,
a cyclohexyl group which is substituted in the 3 position by an amino group,
or an N-(2-aminoethyl)-methylamino or an N-(2-aminoethyl)-ethylamino group, the tautomers, enantiomers, diastereomers, the mixtures thereof and the salts thereof.

3. Purine derivatives of general formula I according to claim 1, wherein
R¹ denotes a hydrogen atom,
a C₁₋₆-alkyl group,
a C₃₋₆-alkenyl group,
a C₃₋₄-alkynyl group,
a C₃₋₆-cycloalkylmethyl group,
a phenyl-C₁₋₃-alkyl group wherein the phenyl moiety is substituted by R¹⁰ and R¹¹, where
R¹⁰ denotes a hydrogen atom, a fluorine, chlorine or bromine atom,
a methyl or trifluoromethyl group,
a cyano, aminocarbonyl, dimethylaminocarbonyl or methylsulphonyl group,
an amino, acetylamino or methylsulphonylamino group,
a hydroxy, methoxy, difluoromethoxy, trifluoromethoxy, carboxymethoxy, methoxycarbonylmethoxy, ethyloxycarbonylmethoxy, aminocarbonylmethoxy, methylaminocarbonylmethoxy, ethylaminocarbonylmethoxy or dimethylaminocarbonylmethoxy group and
R¹¹ denotes a hydrogen atom, a fluorine or chlorine atom,
or a methyl or methoxy group,
a naphthylmethyl group wherein the naphthyl moiety is substituted by R¹⁰ and R¹¹, where R¹⁰ and R¹¹ are as hereinbefore defined,
a heteroarylmethyl group where the term
heteroaryl denotes a furanyl, thienyl, oxazolyl, isoxazolyl, thiazolyl, pyridyl, pyrimidinyl, pyrazinyl, quinolinyl, isoquinolinyl or quinazolinyl group and the above-mentioned heteroaryl groups are substituted by R¹⁰ and R¹¹, where R¹⁰ and R¹¹ are as hereinbefore defined,
a phenylcarbonylmethyl group wherein the phenyl moiety is substituted by R¹⁰ and R¹¹, where R¹⁰ and R¹¹ are as hereinbefore defined,
a furanylcarbonylmethyl, thienylcarbonylmethyl or pyridylcarbonylmethyl group,
or a 2-oxo-propyl or cyclohexylcarbonylmethyl group,
R² denotes a hydrogen atom,
a C₁₋₆-alkyl group,
a C₃₋₆-alkenyl group,
a C₃₋₄-alkynyl group,
a C₃₋₆-cydoalkyl or C₃₋₆-cycloalkyl-C₁₋₃-alkyl group,
a phenyl group which is substituted by R¹⁰ and R¹¹, where R¹⁰ and R¹¹ are as hereinbefore defined,
a phenyl-C₁₋₃-alkyl group wherein the phenyl moiety is substituted by R¹⁰ and R¹¹, where R¹⁰ and R¹¹ are as hereinbefore defined,
a phenyl-C₂₋₃-alkenyl group wherein the phenyl moiety is substituted by R¹⁰ and R¹¹, where R¹⁰ and R are as hereinbefore defined,
a phenylcarbonylmethyl group wherein the phenyl moiety is substituted by R¹⁰ and R¹ where R¹⁰ and R¹¹ are as hereinbefore defined,
a furanyl, thienyl or pyridyl group,
a furanyl-C₁₋₃-alkyl, thienyl-C₁₋₃-alkyl or pyridyl-C₁₋₃-alkyl group,
a cyano group,
an amino, C₁₋₄-alkylamino or di-(C₁₋₄-alkyl)-amino group,
an amino group substituted by the groups R¹⁵ and R¹⁶ wherein
R¹⁵ denotes a hydrogen atom or a methyl or ethyl group and
R¹⁶ denotes a C₁₋₄-alkyl group which is substituted by a cyano, carboxy, methoxycarbonyl, ethyloxycarbonyl, aminocarbonyl, methylaminocarbonyl, dimethylaminocarbonyl, ethylaminocarbonyl, diethylaminocarbonyl, pyrrolidin-1-ylcarbonyl or morpholin-4-ylcarbonyl group,
an amino group substituted by the groups R¹⁵ and R¹⁷ wherein
R¹⁵ is as hereinbefore defined and
R¹⁷ denotes a straight-chain C₂₋₄-alkyl group which is terminally substituted in each case by an amino, methylamino, dimethylamino, acetylamino, ethyloxycarbonylamino, phenylcarbonylamino, methylsulphonylamino, phenylsulphonylamino, hydroxy, methoxy, phenyloxy, methylsulphanyl or phenylsulphanyl group,
a pyrrolidin-1-yl, piperidin-1-yl, morpholin-4-yl, piperazin-1-yl or 4-methyl-piperazin-1-yl group,
a C₃₋₆-cycloalkylamino or C₃₋₆-cycloalkyl-C₁₋₃-alkylamino group,
a phenylamino group,
a phenyl-C₁₋₃-alkylamino group wherein the phenyl moiety is substituted by R¹⁰ and R¹¹, where R¹⁰ and R¹¹ are as hereinbefore defined,
a naphthylmethylamino group,
a heteroaryl-C₁₋₂-alkylamino group, where the term heteroaryl is as hereinbefore defined, or
a methylsulphanyl, benzylsulphanyl or (2-phenylethyl)sulphanyl group,
R³ denotes a C₄₋₆-alkenyl group,
a C₃₋₄-alkenyl group which is substituted by a fluorine, chlorine or bromine atom or a trifluoromethyl group,
a 2-butyn-1-yl group or
a methyl group substituted by the group R_{c}, where
R_{c} denotes a 1-cyclopenten-1-yl-or 1-cyclohexen-1-yl group,
a phenyl group optionally substituted by a fluorine, chlorine, bromine or iodine atom, by a methyl, trifluoromethyl, cyano, methoxy, difluoromethoxy or trifluoromethoxy group,
a phenyl group which is substituted by two fluorine atoms,
a naphthyl group or
a furanyl, thienyl or pyridyl group,
and
R⁴ denotes a piperidin-1-yl group which is substituted in the 3 position by an amino group,
a hexahydroazepin-1-yl group which is substituted in the 3 position or 4 position by an amino group,
a (2-aminocyclohexyl)amino group,
a cyclohexyl group which is substituted in the 3 position by an amino group, or
an N-(2-aminoethyl)-methylamino or an N-(2-aminoethyl)-ethylamino group,
while unless otherwise stated, the above-mentioned alkyl, alkenyl and alkynyl groups may be straight-chain or branched,
the tautomers, enantiomers, diastereomers, the mixtures thereof and the salts thereof.

4. Purine derivatives of general formula I according to claim 3, wherein
R¹ denotes a hydrogen atom,
a methyl, benzyl or 2-phenylethyl group,
a naphthylmethyl or methoxynaphthylmethyl group or
a phenylcarbonylmethyl group,
R² denotes a hydrogen atom,
a methyl or 2-phenylethyl group,
a phenylcarbonylmethyl group,
a cyano group,
an amino, methylamino, dimethylamino, isopropylamino, cyclohexylamino or (cyclohexylmethyl)amino group,
a benzylamino, fluorobenzylamino or (2-phenylethyl)amino group or
a piperidin-1-yl group,
R³ denotes a benzyl or 3-methyl-but-2-en-1-yl group
and
R⁴ denotes a (3-amino-piperidin-1-yl) group,
the tautomers, enantiomers, diastereomers, the mixtures thereof and the salts thereof.

5. The following compounds of general formula I according to claim 1:
(1) 8-(3-amino-piperidin-1-yl)-7-benzyl-2-benzylamino-1-methyl-1,7-dihydro-purin-6-one,
(2) 8-(3-amino-piperidin-1-yl)-7-benzyl-2-(4-fluoro-benzylamino)-1-methyl-1,7-dihydro-purin-6-one,
(3) 8-(3-amino-piperidin-1-yl)-7-benzyl-1-methyl-2-[(2-phenylethyl)amino]-1,7-dihydro-purin-6-one,
(4) 8-(3-amino-piperidin-1-yl)-7-benzyl-2-isopropylamino-1-methyl-1,7-dihydro-purin-6-one,
(5) 8-(3-amino-piperidin-1-yl)-7-benzyl-1-methyl-2-methylamino-1,7-dihydro-purin-6-one,
(6) 8-(3-amino-piperidin-1-yl)-7-benzyl-2-cyclohexylamino-1-methyl-1,7-dihydro-purin-6-one,
(7) 8-(3-amino-piperidin-1-yl)-7-benzyl-2-[(cydohexylmethyl)amino]-1-methyl-1,7-dihydro-purin-6-one,
(8) 8-(3-amino-piperidin-1-yl)-7-benzyl-1-methyl-2-(piperidin-1-yl)-1,7-dihydro-purin-6-one,
(9) 8-(3-amino-piperidin-1-yl)-7-benzyl-2-dimethylamino-1-methyl-1,7-dihydro-purin-6-one,
(10) 2-amino-8-(3-amino-piperidin-1-yl)-7-benzyl-1-methyl-1,7-dihydro-purin-6-one,
(11) 8-(3-amino-piperidin-1-yl)-2-benzylamino-1-methyl-7-(3-methyl-but-2-en-1-yl)-1,7-dihydro-purin-6-one,
(12) 8-(3-amino-piperidin-1-yl)-7-benzyl-1-methyl-2-methyl-1,7-dihydro-purin-6-one,
(13) 8-(3-amino-piperidin-1-yl)-1-methyl-7-(3-methyl-but-2-en-1-yl)-2-(2-phenylethyl)-1,7-dihydro-purin-6-one,
(14) 8-(3-amino-piperidin-1-yl)-7-benzyl-1-methyl-1,7-dihydro-purin-6-one,
(15) 8-(3-amino-piperidin-1-yl)-7-benzyl-1-(2-oxo-2-phenyl-ethyl)-1,7-dihydro-purin-6-one,
(16) 8-(3-amino-piperidin-1-yl)-2-methyl-7-(3-methyl-but-2-en-1-yl)-1-[(naphthalen-1-yl)methyl]-1,7-dihydro-purin-6-one,
(17) 8-(3-amino-piperidin-1-yl)-7-(3-methyl-but-2-en-1-yl)-1-[(naphthalen-1-yl)methyl]-1,7-dihydro-purin-6-one and
(18) 8-(3-amino-piperidin-1-yl)-7-(3-methyl-but-2-en-1-yl)-1-[(4-methoxynaphthalen-1-yl)methyl]-1,7-dihydro-purin-6-one,
as well as the tautomers, enantiomers, diastereomers, the mixtures thereof and the salts thereof.

6. Physiologically acceptable salts of the compounds according to at least one of claims 1 to 5 with inorganic or organic acids or bases.

7. Pharmaceutical compositions containing a compound according to at least one of claims 1 to 5 or a physiologically acceptable salt according to claim 6 optionally together with one or more inert carriers and/or diluents.

8. Use of a compound according to at least one of claims 1 to 6 for preparing a pharmaceutical composition which is suitable for treating type I and type II diabetes mellitus, arthritis, obesity, allograft transplantation and calcitonin-induced osteoporosis.

9. Process for preparing a pharmaceutical composition according to claim 7, **characterised in that** a compound according to at least one of claims 1 to 6 is incorporated in one or more inert carriers and/or diluents by a non-chemical method.

10. Process for preparing the compounds of general formula I according to claims 1 to 6, **characterised in that**
a) in order to prepare compounds of general formula I wherein R⁴ denotes one of the groups mentioned in claim 1 which contain an imino, amino, alkylamino or dialkylamino group,
a compound of general formula 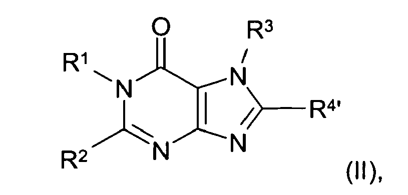
wherein R¹, R² and R³ are defined as in claim 1 and
R^{4'} denotes one of the groups mentioned in claim 1 for R⁴ which contain an imino, amino or alkylamino group, wherein the imino, amino or alkylamino group is substituted by a protective group, such as, for example, a tert.-butyloxycarbonyl, 2,2,2-trichloroethoxycarbonyl or carbobenzyloxycarbonyl group, is deprotected and optionally subsequently alkylated
and subsequently, if desired, any protecting group used during the reactions to protect reactive groups is cleaved and/or
a compound of general formula I thus obtained is resolved into its stereoisomers and/or
a compound of general formula I thus obtained is converted into its salts, particularly for pharmaceutical use into the physiologically acceptable salts thereof with an inorganic or organic acid.

## Revendications

1. Dérivés de purine de la formule générale : 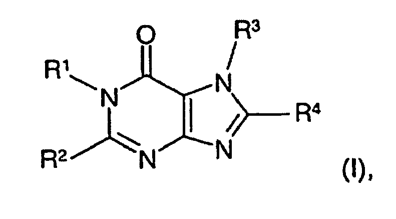 dans laquelle
R¹ représente un atome d'hydrogène,
un groupe alkyle en C₁₋₈,
un groupe alcényle en C₃₋₈,
un groupe alcényle en C₃₋₄, qui est substitué par un groupe (alkyloxy en C₁₋₂)carbonyle, aminocarbonyle, (alkyle en C₁₋₃)aminocarbonyle, di(alkyle en C₁₋₃)aminocarbonyle, pyrrolidin-1-ylcarbonyle, pipéridin-1-ylcarbonyle ou morpholin-4-ylcarbonyle,
un groupe alcynyle en C₃₋₈,
un groupe alkyle en C₁₋₆, substitué par un groupe Rₐ, où
Rₐ représente un groupe cycloalkyle en C₃₋₇, hétéroaryle, cyano, carboxy, (alkyloxy en C₁₋₃)carbonyle, aminocarbonyle, (alkyle en C₁₋₃)aminocarbonyle, di(alkyle en C₁₋₃)aminocarbonyle, pyrrolidin-1-ylcarbonyle, pipéridin-1-ylcarbonyle, morpholin-4-ylcarbonyle, pipérazin-1-ylcarbonyle, 4-méthylpipérazin-1-ylcarbonyle ou 4-éthylpipérazin-1-ylcarbonyle,
un groupe alkyle en C₁₋₆ substitué par un groupe phényle, où le cycle phényle est substitué par les groupes R¹⁰ à R¹⁴ et
R¹⁰ représente un atome d'hydrogène,
un atome de fluor, de chlore, de brome ou d'iode,
un groupe alkyle en C₁₋₄, hydroxy ou alkyloxy en C₁₋₄,
un groupe nitro, amino, (alkyle en C₁₋₃)amino, di(alkyle en C₁₋₃)amino, cyano(alkyle en C₁₋₃)amino, N-(cyano(alkyle en C₁₋₃))-N-(alkyle en C₁₋₃)amino, (alkyloxy en C₁₋₃)carbonyl(alkyle en C₁₋₃)amino, pyrrolidin-1-yle, pipéridin-1-yle, morpholin-4-yle, pipérazin-1-yle ou 4-(alkyle en C₁₋₃)pipérazin-1-yle,
un groupe formylamino, (alkyle en C₁₋₃)carbonylamino, (cycloalkyle en C₃₋₆)carbonylamino, (cycloalkyle en C₃₋₆)(alkyle en C₁₋₃)carbonylamino, arylcarbonylamino, aryl(alkyle en C₁₋₃)carbonylamino, (alkyloxy en C₁₋₃)carbonylamino, aminocarbonylamino, (alkyle en C₁₋₃)aminocarbonylamino, di(alkyle en C₁₋₃)aminocarbonylamino, pyrrolidin-1-ylcarbonylamino, pipéridin-1-ylcarbonylamino, morpholin-4-ylcarbonylamino, pipérazin-1-ylcarbonylamino ou 4-(alkyle en C₁₋₃)pipérazin-1-ylcarbonylamino, (alkyle en C₁₋₃)sulfonylamino, bis(alkylsulfonyl en C₁₋₃)amino, aminosulfonylamino, (alkyle en C₁-₃)aminosulfonylamino, di(alkyle en C₁₋₃)aminosulfonylamino, pyrrolidin-1-ylsulfonylamino, pipéridin-1-ylsulfonylamino, morpholin-4-ylsulfonylamino, pipérazin-1-ylsulfonylamino ou 4-(alkyle en C₁₋₃)pipérazin-1-ylsulfonylamino, (alkylamino en C₁₋₃)thiocarbonylamino, ((alkyloxy en C₁₋₃)carbonylamino)-carbonylamino, arylsulfonylamino ou aryl(alkyle en C₁₋₃)sulfonylamino,
un groupe N-(alkyle en C₁₋₃)formylamino, N-(alkyle en C₁₋₃)-N-((alkyle en C₁₋₃-carbonyl))amino, N-(alkyle en C₁₋₃)-N-((cycloalkyle en C₃₋₆)-carbonyl)amino, N-(alkyle en C₁₋₃)-N-((cycloalkyle en C₃₋₆)-(alkyle en C₁₋₃)-carbonyl)amino, N-(alkyle en C₁₋₃)-N-(arylcarbonyl)amino, N-(alkyle en C₁₋₃)-N-((arylalkyle en C₁₋₃)-carbonyl)amino, N-(alkyle en C₁₋₃)-N-((alkyloxy en C₁₋₃)-carbonyl)amino, N-(aminocarbonyl)-N-(alkyle en C₁₋₃)amino, N-(alkyle en C₁₋₃)-aminocarbonyl)-N-(alkyle en C₁₋₃)amino, N-[di(alkyle en C₁₋₃)aminocarbonyl]-N-(alkyle en C₁₋₃)amino, N-(alkyle en C₁₋₃)-N-((alkyle en C₁₋₃)-sulfonyl)amino, N-(alkyle en C₁₋₃)-N-(arylsulfonyl)amino ou N-(alkyle en C₁₋₃)-N-(aryl-(alkyle en C₁₋₃)-sulfonyl)amino,
un groupe 2-oxoimidazolidin-1-yle, 2,4-dioxoimidazolidin-1-yle, 2,5-dioxoimidazolidin-1-yle ou 2-oxohexahydropyrimidin-1-yle, dans lequel l'atome d'azote en position 3 peut être substitué chaque fois, par un groupe méthyle ou éthyle,
un groupe cyano, carboxy, (alkyloxy en C₁₋₃)carbonyle, aminocarbonyle, (alkyle en C₁₋₃)aminocarbonyle, di(alkyle en C₁₋₃)aminocarbonyle, pyrrolidin-1-ylcarbonyle, pipéridin-1-ylcarbonyle, morpholin-4-ylcarbonyle, pipérazin-1-ylcarbonyle ou 4-(alkyle en C₁₋₃)pipérazin-1-ylcarbonyle,
un groupe (alkyle en C₁₋₃)carbonyle ou arylcarbonyle,
un groupe carboxy(alkyle en C₁₋₃), (alkyloxy en C₁₋₃)carbonyl(alkyle en C₁₋₃), cyano(alkyle en C₁₋₃), aminocarbonyl(alkyle en C₁₋₃), (alkyle en C₁₋₃)aminocarbonyl(alkyle en C₁₋₃), di(alkyle en C₁₋₃)aminocarbonyl(alkyle en C₁₋₃), pyrrolidin-1-ylcarbonyl(alkyle en C₁₋₃), pipéridin-1-ylcarbonyl(alkyle en C₁₋₃), morpholin-4-ylcarbonyl(alkyle en C₁₋₃), pipérazin-1-ylcarbonyl(alkyle en C₁₋₃) ou 4-(alkyle en C₁₋₃)pipérazin-1-ylcarbonyl(alkyle en C₁₋₃),
un groupe carboxy(alkyloxy en C₁₋₃), (alkyloxy en C₁₋₃)carbonyl(alkyloxy en C₁₋₃), cyano(alkyloxy en C₁₋₃), aminocarbonyl(alkyloxy en C₁₋₃), (alkyle en C₁₋₃)aminocarbonyl(alkyloxy en C₁₋₃), di(alkyle en C₁₋₃)aminocarbonyl(alkyloxy en C₁₋₃), pyrrolidin-1-ylcarbonyl(alkyloxy en C₁₋₃), pipéridin-1-ylcarbonyl(alkyloxy en C₁₋₃), morpholin-4-ylcarbonyl(alkyloxy en C₁₋₃), pipérazin-1-ylcarbonyl(alkyloxy en C₁₋₃) ou 4-(alkyle en C₁₋₃)pipérazin-1-ylcarbonyl(alkyloxy en C₁₋₃),
un groupe hydroxy(alkyle en C₁₋₃), (alkyloxy en C₁₋₃)(alkyle en C₁₋₃), amino(alkyle en C₁₋₃), (alkyle en C₁₋₃)amino(alkyle en C₁₋₃), di(alkyle en C₁₋₃)amino(alkyle en C₁₋₃), pyrrolidin-1-yl(alkyle en C₁₋₃), pipéridin-1-yl(alkyle en C₁₋₃), morpholin-4-yl(alkyle en C₁₋₃), pipérazin-1-yl(alkyle en C₁₋₃), 4-(alkyle en C₁₋₃)pipérazin-1-yl(alkyle en C₁₋₃), un groupe hydroxy(alkyloxy en C₁₋₃), (alkyloxy en C₁₋₃)(alkyloxy en C₁₋₃), (alkyle en C₁₋₃) sulfanyl (alkyloxy en C₁₋₃), (alkyle en C₁₋₃) sulfinyl(alkyloxy en C₁₋₃), (alkyle en C₁₋₃) sulfonyl(alkyloxy en C₁₋₃), amino(alkyloxy en C₁₋₃), (alkyle en C₁₋₃)amino(alkyloxy en C₁₋₃), di(alkyle en C₁₋₃)amino(alkyloxy en C₁₋₃), pyrrolidin-1-yl(alkyloxy en C₁₋₃), pipéridin-1-yl(alkyloxy en C₁₋₃), morpholin-4-yl(alkyloxy en C₁₋₃), pipérazin-1-yl(alkyloxy en C₁₋₃), 4-(alkyle en C₁₋₃)pipérazin-1-yl(alkyloxy en C₁₋₃),
un groupe mercapto, (alkyle en C₁₋₃)sulfanyle, (alkyle en C₁₋₃)sulfinyle, (alkyle en C₁₋₃)sulfonyle, (alkyle en C₁₋₃)sulfonyloxy, arylsulfonyloxy, trifluorométhylsulfanyle, trifluorométhylsulfinyle ou trifluorométhylsulfonyle,
un groupe sulfo, aminosulfonyle, (alkyle en C₁₋₃)aminosulfonyle, di(alkyle en C₁₋₃) aminosulfonyle, pyrrolidin-1-ylsulfonyle, pipéridin-1-ylsulfonyle, morpholin-4-ylsulfonyle, pipérazin-1-ylsulfonyle ou 4-(alkyle en C₁₋₃)pipérazin-1-ylsulfonyle,
un groupe méthyle ou méthoxy substitué par 1 à 3 atomes de fluor,
un groupe éthyle ou éthoxy substitué par 1 à 5 atomes de fluor,
un groupe alcényle en C₂₋₄ ou alcynyle en C₂₋₄,
un groupe alcényloxy en C₃₋₄ ou alcynyloxy en C₃₋₄,
un groupe cycloalkyle en C₃₋₆ ou cycloalkyloxy en C₃₋₆,
un groupe (cycloalkyle en C₃₋₆)-(alkyle en C₁₋₃) ou (cycloalkyle en C₃₋₆)-(alkyloxy en C₁₋₃),
un groupe aryle, aryloxy, aryl(alkyle en C₁₋₃) ou aryl(alkyloxy en C₁₋₃),
R¹¹ et R¹², qui peuvent être identiques ou différents, représentent un atome d'hydrogène, un atome de fluor, de chlore, de brome ou d'iode, un groupe alkyle en C₁₋₃, trifluorométhyle, hydroxy, alkyloxy en C₁₋₃ ou cyano, ou
R¹¹ avec R¹², dans la mesure ou ceux-ci sont liés à des atomes de carbone voisins, représentent également un groupe méthylènedioxy, difluorométhylènedioxy ou alkylène en C₃₋₅ linéaire, et
R¹³ et R¹⁴, qui peuvent être identiques ou différents, représentent un atome d'hydrogène, un atome de fluor, de chlore ou de brome, un groupe trifluorométhyle, alkyle en C₁₋₃, ou alkyloxy en C₁₋₃,
un groupe phényl(alkyle en C₁₋₄), dans lequel la partie alkyle est substituée par un groupe cyano, carboxy, (alkyloxy en C₁₋₃)carbonyle, aminocarbonyle, (alkyle en C₁₋₃)aminocarbonyle, di(alkyle en C₁₋₃)aminocarbonyle, pyrrolidin-1-ylcarbonyle, pipéridin-1-ylcarbonyle, morpholin-4-ylcarbonyle, et la partie phényle est substituée par les groupes R¹⁰ à R¹⁴, où R¹⁰ à R¹⁴ sont définis comme indiqué ci-dessus,
un groupe phényle substituée par les groupes R¹⁰ à R¹⁴, où R¹⁰ à R¹⁴ sont définis comme indiqué ci-dessus,
un groupe phényl(alcényle en C₂₋₃), dans lequel la partie phényle est substituée par les groupes R¹⁰ R¹⁴ où R¹⁰ R¹⁴ sont définis comme indiqué ci-dessus,
un groupe phényl-(CH₂)ₘ-A-(CH₂)ₙ, dans lequel la partie phényle est substituée par les groupes R¹⁰ à R¹⁴**,** où R¹⁰ à R¹⁴ sont définis comme indiqué ci-dessus, et
A représente un groupe carbonyle, m le nombre 0, 1 ou 2 et n le nombre 1, 2 ou 3,
un groupe phénylcarbonylméthyle, dans lequel la partie phényle est substituée par les groupes R¹⁰ à R¹⁴, où R¹⁰ à R¹⁴ sont définis comme indiqué ci-dessus, et la partie méthyle est substituée par un groupe alkyle en C₁₋₃,
un groupe phényl-(CH₂)ₘ-B-(CH₂)ₙ, dans lequel la partie phényle est substituée par les groupes R¹⁰ à R¹⁴, où R¹⁰ à R¹⁴, m et n sont définis comme indiqué ci-dessus, et
B représente un groupe méthylène, qui est substitué par un groupe hydroxy, alkyloxy en C₁₋₃, amino, (alkyle en C₁₋₃)amino, di(alkyle en C₁₋₃)amino, mercapto, (alkyle en C₁₋₃)sulfanyle, (alkyle en C₁₋₃)sulfinyle ou (alkyle en C₁₋₃)sulfonyle, et le cas échéant, est substitué en outre, par un groupe méthyle ou éthyle,
un groupe naphtyl(alkyle en C₁₋₃), dans lequel la partie naphtyle est substituée par les groupes R¹⁰ à R¹⁴, où R¹⁰ à R¹⁴ sont définis comme indiqué ci-dessus,
un groupe naphtyl-(CH₂)ₘ A-(CH₂)ₙ, dans lequel la partie phényle est substituée par les groupes R¹⁰ à R¹⁴, où R¹⁰ à R¹⁴, A, m et n sont définis comme indiqué ci-dessus,
un groupe naphtyl-(CH₂)ₘ-B-(CH₂)ₙ, dans lequel la partie phényle est substituée par les groupes R¹⁰ à R¹⁴, où R¹⁰ à R¹⁴, B, m et n sont définis comme indiqué ci-dessus,
un groupe [1,4]naphtoquinon-2-yle, chromèn-4-on-3-yle, 1-oxoindan-2-yle, 1,3-dioxoindan-2-yle ou 2,3-dihydro-3-oxobenzofuran-2-yle,
un groupe hétéroaryl-(CH₂)ₘ-A-(CH₂)ₙ, dans lequel A, m et n sont définis comme indiqué ci-dessus,
un groupe hétéroaryl-(CH₂)ₘ-B-(CH₂)ₙ, dans lequel B, m et n sont définis comme indiqué ci-dessus,
un groupe (alkyle en C₁₋₆)-A-(CH₂)ₙ, dans lequel A et n sont définis comme indiqué ci-dessus,
un groupe (cycloalkyle en C₃₋₇)-(CH₂)ₘ-A-(CH₂)ₙ, dans lequel A, m et n sont définis comme indiqué ci-dessus,
un groupe (cycloalkyle en C₃₋₇)-(CH₂)ₘ-B-(CH₂)ₙ, dans lequel B, m et n sont définis comme indiqué ci-dessus,
un groupe R²¹-A-(CH₂)ₙ, dans lequel R²¹ représente un groupe (alkyloxy en C₁₋₃)carbonyle, aminocarbonyle, (alkyle en C₁₋₃)aminocarbonyle, di(alkyle en C₁₋₃)aminocarbonyle, pyrrolidin-1-ylcarbonyle, pipéridin-1-ylcarbonyle ou morpholin-4-ylcarbonyle, pipérazin-1-ylcarbonyle, 4-méthylpipérazin-1-ylcarbonyle ou 4-éthylpipérazin-1-ylcarbonyle et A et n sont définis comme indiqué ci-dessus,
un groupe phényl-(CH₂)ₘ-D-(alkyle en C₁₋₃), dans lequel la partie phényle est substituée par les groupes R¹⁰ à R¹⁴, où R¹⁰ à R¹⁴ et m sont définis comme indiqué ci-dessus, et D représente un atome d'oxygène ou de soufre, un groupe imino, (alkyle en C₁₋₃)imino, sulfinyle ou sulfonyle,
un groupe naphtyl-(CH₂)ₘ-D-(alkyle en C₁₋₃), dans lequel la partie naphtyle est substituée par les groupes R¹⁰ à R¹⁴, où R¹⁰ à R¹⁴, D et m sont définis comme indiqué ci-dessus,
un groupe alkyle en C₂₋₆ substitué par un groupe R_{b}, où
R_{b} est isolé par au moins deux atomes de carbone de l'atome d'azote cyclique en position 1 du squelette purine, et
R_{b} représente un groupe hydroxy, alkyloxy en C₁₋₃, mercapto, (alkyle en C₁₋₃) sulfanyle, (alkyle en C₁₋₃) sulfinyle, (alkyle en C₁₋₃) sulfonyle, amino, (alkyle en C₁₋₃)carbonylamino, (cycloalkyle en C₃₋₆)carbonylamino, arylcarbonylamino, (alkyle en C₁₋₃)amino, di(alkyle en C₁₋₃)amino, pyrrolidin-1-yle, pipéridin-1-yle, morpholin-4-yle, pipérazin-1-yle ou 4-(alkyle en C₁₋₃)pipérazin-1-yle,
un groupe cycloalkyle en C₃₋₆, ou
un groupe amino ou arylcarbonylamino,
R² représente un atome d'hydrogène,
un groupe alkyle en C₁₋₈,
un groupe alcényle en C₃₋₈,
un groupe alcényle en C₃₋₄, qui est substitué par un groupe (alkyloxy en C₁₋₂)-carbonyle, aminocarbonyle, (alkyle en C₁₋₃)aminocarbonyle, di(alkyle en C₁₋₃)-aminocarbonyle, pyrrolidin-1-ylcarbonyle, pipéridin-1-ylcarbonyle ou morpholin-4-ylcarbonyle,
un groupe alcynyle en C₃₋₈,
un groupe cycloalkyle en C₃₋₆,
un groupe alkyle en C₁₋₆, substitué par un groupe Rₐ, où Rₐ est défini comme indiqué ci-dessus,
un groupe phényle, qui est substitué par R¹⁰ à R¹⁴, où R¹⁰ à R¹⁴ sont définis comme indiqué ci-dessus,
un groupe alkyle en C₁₋₆ substitué par un groupe phényle, où le cycle phényle est substitué par les groupes R¹⁰ à R¹⁴, où R¹⁰ à R¹⁴ sont définis comme indiqué ci-dessus,
un groupe phényl(alkyle en C₁₋₄), dans lequel la partie alkyle est substituée par un groupe cyano, carboxy, (alkyloxy en C₁₋₃)carbonyle, aminocarbonyle, (alkyle en C₁₋₃)aminocarbonyle, di(alkyle en C₁₋₃)aminocarbonyle, pyrrolidin-1-ylcarbonyle, pipéridin-1-ylcarbonyle, morpholin-4-ylcarbonyle, et la partie phényle est substituée par les groupes R¹⁰ à R¹⁴, où R¹⁰ R¹⁴ sont définis comme indiqué ci-dessus,
un groupe phényl-(alcényl en C₂₋₃), où la partie phényle est substituée par R¹⁰ à R¹⁴, où R¹⁰ à R¹⁴ sont définis comme indiqué ci-dessus,
un groupe hétéroaryle,
un groupe phényl-(CH₂)ₘ-A- ou phényl-(CH₂)ₘ-A-(CH₂)ₙ, dans lequel la partie phényle est respectivement substituée par les groupes R¹⁰ à R¹⁴ où A, R¹⁰, à R¹⁴, m et n sont définis comme indiqué ci-dessus,
un groupe phénylcarbonylméthyle dans lequel la partie phényle est respectivement substituée par R¹⁰ à R¹⁴, où R¹⁰ à R¹⁴ sont définis comme indiqué ci-dessus, et la partie méthyle est substituée par un groupe alkyle en C₁₋₃,
un groupe phényl-(CH₂)ₘ-B- ou phényl-(CH₂)ₘ-B-(CH₂)ₙ, dans lequel la partie phényle est respectivement substituée par R¹⁰ à R¹⁴ où B, R¹⁰ à R¹⁴ m et n sont définis comme indiqué ci-dessus,
un groupe naphtyl(alkyle en C₁₋₃), dans lequel la partie naphtyle est substituée par les groupes R¹⁰ à R¹⁴, où R¹⁰ à R¹⁴ sont définis comme indiqué ci-dessus,
un groupe naphtyl-(CH₂)ₘ-A- ou naphtyl-(CH₂)ₘ-A-(CH₂)ₙ, dans lequel la partie naphtyle est respectivement substituée par R¹⁰ à R¹⁴, où A, R¹⁰ à R¹⁴, m et n sont définis comme indiqué ci-dessus,
un groupe naphtyl-(CH₂)ₘ-B- ou naphtyl-(CH₂)ₘ-B-(CH₂)ₙ, dans lequel la partie naphtyle est respectivement substituée par R¹⁰ à R¹⁴, où R¹⁰ à R¹⁴, B, m et n sont définis comme indiqué ci-dessus,
un groupe hétéroaryl-(CH₂)ₘ-A- ou hétéroaryl-(CH₂)ₘ-A-(CH₂)ₙ, où A, m et n sont définis comme indiqué ci-dessus,
un groupe hétéroaryl-(CH₂)ₘ-B- ou hétéroaryl-(CH₂)ₘ-B-(CH₂)ₙ, où B, m et n sont définis comme indiqué ci-dessus,
un groupe (alkyle en C₁₋₆)-A- ou (alkyle en C₁₋₆)-A-(CH₂)ₙ, où A et n sont définis comme indiqué ci-dessus,
un groupe (cycloalkyle en C₃₋₇)-(CH₂)ₘ-A- ou (cycloalkyle en C₃₋₇)-(CH₂)ₘ-A-(CH₂)ₙ, où A, m et n sont définis comme indiqué ci-dessus,
un groupe (cycloalkyle en C₃₋₇)-(CH₂)ₘ-B- ou (cycloalkyle en C₃₋₇)-(CH₂)ₘ-B-(CH₂)ₙ, où B, m et n sont définis comme indiqué ci-dessus,
un groupe R²¹-A-(CH₂)ₙ, dans lequel R²¹, A et n sont définis comme indiqué ci-dessus,
un groupe phényl-(CH₂)ₘ-D-(alkyle en C₁₋₃), dans lequel la partie phényle est substituée par les groupes R¹⁰ à R¹⁴, où R¹⁰ à R¹⁴, D et m sont définis comme indiqué ci-dessus,
un groupe naphtyl-(CH₂)ₘ-D-(alkyle en C₁₋₃), dans lequel la partie naphtyle est substituée par les groupes R¹⁰ à R¹⁴, où R¹⁰ à R¹⁴ D et m sont définis comme indiqué ci-dessus,
un groupe alkyle en C₁₋₆ substitué par un groupe R_{b}, où R_{b} est défini comme indiqué ci-dessus,
un groupe cyano, carboxy, (alkyloxy en C₁₋₃)carbonyle, aminocarbonyle, (alkyle en C₁₋₃)aminocarbonyle, di(alkyle en C₁₋₃)aminocarbonyle, pyrrolidin-1-ylcarbonyle, pipéridin-1-ylcarbonyle, morpholin-4-ylcarbonyle, pipérazin-1-ylcarbonyle, 4-méthylpipérazin-1-ylcarbonyle ou 4-éthylpipérazin-1-ylcarbonyle,
un groupe amino, (alkyle en C₁₋₆)amino ou di(alkyle en C₁₋₆)amino,
un groupe amino substitué par les radicaux R¹⁵ et R¹⁶, dans lequel
R¹⁵ représente un atome d'hydrogène ou un groupe alkyle en C₁₋₆, et
R¹⁶ représente un groupe alkyle en C₁₋₆, qui est substitué par Rₐ, où Rₐ est défini comme indiqué ci-dessus,
un groupe amino substitué par les radicaux R¹⁵ et R¹⁷, dans lequel
R¹⁵ est défini comme indiqué ci-dessus, et
R¹⁷ représente un groupe alkyle en C₂₋₆, qui est substitué par un groupe hydroxy, alkyloxy en C₁₋₃, aryloxy, mercapto, (alkyle en C₁₋₃)sulfanyle, (alkyle en C₁₋₃)sulfinyle, (alkyle en C₁₋₃)sulfonyle, (alkyle en C₁₋₃)sulfonylamino, arylsulfanyle, arylsulfinyle, arylsulfonyle, arylsulfonylamino, (alkyle en C₁₋₃)carbonylamino, (cycloalkyle en C₃₋₆)carbonylamino, arylcarbonylamino, (alkyle en C₁₋₃) oxycarbonylamino, aminocarbonylamino, (alkyle en C₁₋₃)aminocarbonylamino, di(alkyle en C₁₋₃)aminocarbonylamino, amino, (alkyle en C₁₋₃)amino, di(alkyle en C₁₋₃)amino, pyrrolidin-1-yle, pipéridin-1-yle, morpholin-4-yle, pipérazin-1-yle, 4-(alkyle en C₁₋₃)pipérazin-1-yle,
un groupe (cycloalkyle en C₃₋₆)amino ou N-(cycloalkyle en C₃₋₆)-N-(alkyle en C₁₋₃)amino,
un groupe phénylamino ou N-(phényl)-N-(alkyle en C₁₋₃)amino, dans lequel la partie phényle est respectivement substituée par R¹⁰ à R¹⁴, où R¹⁰ à R¹⁴ sont définis comme indiqué ci-dessus,
un groupe phényl(alkyle en C₁₋₆)amino ou N-(phényl-(alkyle en C₁₋₆))-N-(alkyle en C₁₋₃)amino, dans lequel la partie phényle est respectivement substituée par R¹⁰ à R¹⁴, où R¹⁰ à R¹⁴ sont définis comme indiqué ci-dessus,
un groupe naphtylamino ou N-(naphtyl)-N-(alkyle en C₁₋₃)amino,
un groupe naphtyl(alkyle en C₁₋₆)amino ou N-(naphtyl-(alkyle en C₁₋₆))-N-(alkyle en C₁₋₃)amino,
un groupe hétéroarylamino ou N-(hétéroaryl)-N-(alkyle en C₁₋₃)amino,
un groupe pyrrolidin-1-yle, pipéridin-1-yle, homopipéridin-1-yle, morpholin-4-yle, homomorpholin-4-yle, pipérazin-1-yle, 4-(alkyle en C₁₋₃)pipérazin-1-yle, homopipérazin-1-yle ou 4-(alkyle en C₁₋₃)homopipérazin-1-yle, ou
un groupe alkyloxy en C₁₋₆, cycloalkyloxy en C₃₋₆ ou (cycloalkyle en C₃₋₆)-(alkyloxy en C₁₋₆),
un groupe (alkyle en C₁₋₆)sulfanyle, (cycloalkyle en C₃₋₆)sulfanyle ou (cycloalkyle en C₃₋₆)-(alkyle en C₁₋₆)sulfanyle,
un groupe phényloxy ou phénylsulfanyle, dans lequel la partie phényle est respectivement substituée par R¹⁰ à R¹⁴, où R¹⁰ à R¹⁴ sont définis comme indiqué ci-dessus,
un groupe phényl(alkyloxy en C₁₋₆) ou phényl-(alkyle en C₁₋₆)sulfanyle, dans lequel la partie phényle est respectivement substituée par R¹⁰ à R¹⁴, où R¹⁰ à R¹⁴ sont définis comme indiqué ci-dessus,
un groupe naphtyloxy ou naphtylsulfanyle, dans lequel la partie naphtyle est respectivement substituée par R¹⁰ à R¹⁴, où R¹⁰ à R¹⁴ sont définis comme indiqué ci-dessus,
un groupe naphtyl-(alkyloxy en C₁₋₆) ou naphtyl-(alkyle en C₁₋₆)sulfanyle, dans lequel la partie naphtyle est respectivement substituée par R¹⁰ à R¹⁴, où R¹⁰ à R¹⁴ sont définis comme indiqué ci-dessus,
un groupe hétéroaryloxy ou hétéroarylsulfanyle, ou
un groupe hétéroaryl-(alkyloxy en C₁₋₆) ou hétéroaryl-(alkyle en C₁₋₆)sulfanyle,
R³ représente un groupe alkyle en C₁₋₈,
un groupe alkyle en C₁₋₄ substitué par un groupe R_{c}, où
R_{c} représente un groupe cycloalkyle en C₃₋₇ le cas échéant substitué par un ou deux groupes alkyle en C₁₋₃,
un groupe cycloalcényle en C₅₋₇ le cas échéant substitué par un ou deux groupes alkyle en C₁₋₃,
un groupe aryle, ou
un groupe furanyle, thiényle, oxazolyle, isoxazolyle, thiazolyle, isothiazolyle, pyridyle, pyridazinyle, pyrimidyle ou pyrazinyle, où les radicaux hétérocycliques indiqués ci-dessus peuvent être substitués chaque fois, par un ou deux groupes alkyle en C₁₋₃, ou par un atome de fluor, de chlore, de brome ou d'iode ou par un groupe trifluorométhyle, cyano ou alcoxy en C₁₋₃,
un groupe alcényle en C₃₋₈,
un groupe alcényle en C₃₋₆, substitué par un atome de fluor, de chlore ou de brome ou un groupe trifluorométhyle,
un groupe alcynyle en C₃₋₈,
un groupe aryle, ou
un groupe aryl-(alcényle en C₂₋₄),
et
R⁴ représente un groupe azétidin-1-yle ou pyrrolidin-1-yle, qui est substitué en position 3 par un groupe amino, (alkyle en C₁₋₃)amino ou di(alkyle en C₁₋₃)amino, et peut être substitué en outre, par un ou deux groupes alkyle en C₁₋₃,
un groupe pipéridin-1-yle ou hexahydroazépin-1-yle, qui est substitué en position 3 ou 4 par un groupe amino, (alkyle en C₁₋₃)amino ou di(alkyle en C₁₋₃)amino, et peut être substitué en outre, par un ou deux groupes alkyle en C₁₋₃,
un groupe 3-aminopipéridin-1-yle, dans lequel la partie pipéridin-1-yle est substituée en outre, par un groupe aminocarbonyle, (alkyle en C₁₋₂)aminocarbonyle, di(alkyle en C₁₋₂)aminocarbonyle, pyrrolidin-1-ylcarbonyle, (2-cyanopyrrolidin-1-yl)carbonyle, thiazolidin-3-ylcarbonyle, (4-cyanothiazolidin-3-yl)carbonyle, pipéridin-1-ylcarbonyle ou morpholin-4-ylcarbonyle,
un groupe 3-aminopipéridin-1-yle, dans lequel la partie pipéridin-1-yle est substituée en outre, en position 4 ou 5, par un groupe hydroxy ou méthoxy,
un groupe 3-aminopipéridin-1-yle, dans lequel le groupe méthylène en position 2 ou 6 est remplacé par un groupe carbonyle,
un groupe pipéridin-1-yle ou hexahydroazépin-1-yle, substitué en position 3 par un groupe amino, (alkyle en C₁₋₃)amino ou di(alkyle en C₁₋₃)amino, dans lesquels chaque fois deux atomes d'hydrogène sur le squelette carboné du groupe pipéridin-1-yle ou hexahydroazépin-1-yle, sont remplacés par un pont alkylène linéaire, où ce pont contient 2 à 5 atomes de carbone, lorsque les deux atomes d'hydrogène se trouvent sur le même atome de carbone, ou contient 1 à 4 atomes de carbone lorsque les atomes d'hydrogène se trouvent sur des atomes de carbone voisins, ou contient 1 à 4 atomes de carbone lorsque les atomes d'hydrogène se trouvent sur des atomes de carbone, qui sont séparés par un atome, ou contient 1 à 3 atomes de carbone lorsque les deux atomes d'hydrogène se trouvent sur des atomes de carbone, qui sont séparés par deux atomes,
un groupe azétidin-1-yle, pyrrolidin-1-yle, pipéridin-1-yle ou hexahydroazépin-1-yle, qui est substitué par un groupe amino(alkyle en C₁₋₃), (alkyle en C₁₋₃)amino(alkyle en C₁₋₃) ou di(alkyle en C₁₋₃)amino(alkyle en C₁₋₃),
un groupe pipérazin-1-yle ou [1,4]diazépan-1-yle, le cas échéant substitué sur le squelette carboné, par un ou deux groupes alkyle en C₁₋₃,
un groupe 3-iminopipérazin-1-yle, 3-imino-[1,4]diazépan-1-yle ou 5-imino-[1,4]diazépan-1-yle, le cas échéant substitué sur le squelette carboné, par un ou deux groupes alkyle en C₁₋₃,
un groupe [1,4]diazépan-1-yle, le cas échéant substitué par un ou deux groupes alkyle en C₁₋₃, qui est substitué en position 6 par un groupe amino,
un groupe cycloalkyle en C₃₋₇, qui est substitué par un groupe amino, (alkyle en C₁₋₃)amino ou di(alkyle en C₁₋₃)amino,
un groupe cycloalkyle en C₃₋₇, qui est substitué par un groupe amino(alkyle en C₁₋₃), (alkyle en C₁₋₃)amino(alkyle en C₁₋₃) ou di(alkyle en C₁₋₃)amino(alkyle en C₁₋₃),
un groupe (cycloalkyle en C₃₋₇)-(alkyle en C₁₋₂), qui est substitué dans la partie cycloalkyle, par un groupe amino, (alkyle en C₁₋₃)amino ou di(alkyle en C₁₋₃)amino,
un groupe (cycloalkyle en C₃₋₇)-(alkyle en C₁₋₂), qui est substitué dans la partie cycloalkyle, par un groupe amino(alkyle en C₁₋₃), (alkyle en C₁₋₃)amino(alkyle en C₁₋₃) ou di(alkyle en C₁₋₃)amino(alkyle en C₁₋₃),
un groupe (cycloalkyle en C₃₋₇)amino, qui est substitué dans la partie cycloalkyle, par un groupe amino, (alkyle en C₁₋₃)amino ou di(alkyle en C₁₋₃)amino, où les deux atomes d'azote dans la partie cycloalkyle sont séparés l'un de l'autre, par au moins deux atomes de carbone,
un groupe N-(cycloalkyle en C₃₋₇)-N-(alkyle en C₁₋₃)amino, dans lequel la partie cycloalkyle est substituée par un groupe amino, (alkyle en C₁₋₃)amino ou di(alkyle en C₁₋₃)amino, où les deux atomes d'azote dans la partie cycloalkyle sont séparés l'un de l'autre, par au moins deux atomes de carbone,
un groupe cyclo(alkyle en C₃₋₇)amino, dans lequel la partie cycloalkyle est substituée par un groupe amino(alkyle en C₁₋₃), (alkyle en C₁₋₃)amino(alkyle en C₁₋₃) ou di(alkyle en C₁₋₃)amino(alkyle en C₁₋₃),
un groupe N-(cycloalkyle en C₃₋₇)-N-(alkyle en C₁₋₃)amino, dans lequel la partie cycloalkyle est substituée par un groupe amino(alkyle en C₁₋₃), (alkyle en C₁₋₃)-amino(alkyle en C₁₋₃) ou di(alkyle en C₁₋₃)amino(alkyle en C₁₋₃),
un groupe (cycloalkyle en C₃₋₇)(alkyle en C₁₋₂)amino, dans lequel la partie cycloalkyle est substituée par un groupe amino, (alkyle en C₁₋₃)amino ou di(alkyle en C₁₋₃)amino,
un groupe N-((cycloalkyle en C₃₋₇)-(alkyle en C₁₋₂))-N-(alkyle en C₁₋₂)amino, dans lequel la partie cycloalkyle est substituée par un groupe amino, (alkyle en C₁₋₃)amino ou di(alkyle en C₁₋₃)amino,
un groupe (cycloalkyle en C₃₋₇)(alkyle en C₁₋₂)amino, dans lequel la partie cycloalkyle est substituée par un groupe amino(alkyle en C₁₋₃), (alkyle en C₁₋₃)-amino(alkyle en C₁₋₃) ou di(alkyle en C₁₋₃)amino(alkyle en C₁₋₃),
un groupe N-((cycloalkyle en C₃₋₇)-(alkyle en C₁₋₂))-N-(alkyle en C₁₋₂)amino, dans lequel la partie cycloalkyle est substituée par un groupe amino(alkyle en C₁₋₃), (alkyle en C₁₋₃)amino(alkyle en C₁₋₃) ou di(alkyle en C₁₋₃)amino(alkyle en C₁₋₃),
un groupe R¹⁹-(alkyle en C₂₋₄)amino, dans lequel R¹⁹ est séparé de l'atome d'azote de la partie (alkyle en C₂₋₄)amino par au moins deux atomes de carbone, et
R¹⁹ représente un groupe amino, (alkyle en C₁₋₃)amino ou di(alkyle en C₁₋₃)amino,
un groupe R¹⁹-(alkyle en C₂₋₄)amino, dans lequel l'atome d'azote de la partie (alkyle en C₂₋₄)amino, est substitué par un groupe alkyle en C₁₋₃ et R¹⁹ est séparé de l'atome d'azote de la partie (alkyle en C₂₋₄)amino par au moins deux atomes de carbone, où R¹⁹ est défini comme indiqué ci-dessus,
un groupe amino substitué par le radical R²⁰, dans lequel
R²⁰ représente un groupe azétidin-3-yle, azétidin-2-ylméthyle, azétidin-3-ylméthyle, pyrrolidin-3-yle, pyrrolidin-2-ylméthyle, pyrrolidin-3-ylméthyle, pipéridin-3-yle, pipéridin-4-yle, pipéridin-2-ylméthyle, pipéridin-3-ylméthyle ou pipéridin-4-ylméthyle, où les radicaux mentionnés pour R²⁰ peuvent être substitués chaque fois, par un ou deux groupes alkyle en C₁₋₃,
un groupe amino substitué par le radical R²⁰ et un groupe alkyle en C₁₋₃, dans lequel R²⁰ est défini comme indiqué ci-dessus, où les radicaux mentionnés pour R²⁰ peuvent être substitués chaque fois, par un ou deux groupes alkyle en C₁₋₃,
un groupe R¹⁹-(alkyle en C₃₋₄), dans lequel la partie alkyle en C₃₋₄ est linéaire et peut être en outre, substitué par un ou deux groupes alkyle en C₁₋₃, où R¹⁹ est défini comme indiqué ci-dessus,
un groupe 3-amino-2-oxopipéridin-5-yle ou 3-amino-2-oxo-1-méthylpipéridin-5-yle, un groupe pyrrolidin-3-yle, pipéridin-3-yle, pipéridin-4-yle, hexahydroazépin-3-yle ou hexahydroazépin-4-yle, qui est substitué en position 1 par un groupe amino, (alkyle en C₁₋₃)amino ou di(alkyle en C₁₋₃)amino,
ou un groupe azétidin-2-yl-(alkyle en C₁₋₂), azétidin-3-yl-(alkyle en C₁₋₂), pyrrolidin-2-yl-(alkyle en C₁₋₂), pyrrolidin-3-yle, pyrrolidin-3-yl-(alkyle en C₁₋₂), pipéridin-2-yl-(alkyle en C₁₋₂), pipéridin-3-yle, pipéridin-3-yl-(alkyle en C₁₋₂), pipéridin-4-yle ou pipéridin-4-yl-(alkyle en C₁₋₂), où les groupes cités précédemment peuvent être substitués chaque fois, par un ou deux groupes alkyle en C₁₋₃,
où par groupes aryle mentionnés lors de la définition des radicaux cités ci-dessus, on entend des groupes phényle ou naphtyle, qui peuvent être mono- ou disubstitués indépendamment l'un de l'autre, par Rₕ, où les substituants peuvent être identiques ou différents, et Rₕ représente un atome de fluor, de chlore, de brome ou d'iode, un groupe trifluorométhyle, cyano, nitro, amino, aminocarbonyl, aminosulfonyle, méthylsulfonyle, acétylamino, méthylsulfonylamino, alkyle en C₁₋₃, cyclopropyle, éthényle, éthynyle, hydroxy, alcoxy en C₁₋₃, difluorométhoxy ou trifluorométhoxy, par groupes hétéroaryle mentionnés lors de la définition des radicaux cités ci-dessus, on entend un groupe pyrrolyle, furannyle, thiényle, pyridyle, indolyle, benzofurannyle, benzothiophényle, quinoléinyle ou isoquinoléinyle,
ou un groupe pyrrolyle, furannyle, thiényle ou pyridyle, dans lequel un ou deux groupes méthine sont remplacés par un atome d'azote,
ou un groupe indolyle, benzofurannyle, benzothiophényle, quinoléinyle ou isoquinoléinyle, dans lequel un à trois groupes méthine sont remplacés par un atome d'azote,
ou un groupe 1,2-dihydro-2-oxopyridinyle, 1,4-dihydro-4-oxopyridinyle, 2,3-dihydro-3-oxopyridazinyle, 1,2,3,6-tétrahydro-3,6-dioxopyridazinyle, 1,2-dihydro-2-oxopyrimidinyle, 3,4-dihydro-4-oxopyrimidinyle, 1,2,3,4-tétrahydro-2,4-dioxopyrimidinyle, 1,2-dihydro-2-oxopyrazinyle, 1,2,3,4-tétrahydro-2,3-dioxopyrazinyle, 2,3-dihydro-2-oxoindolyle, 2,3-dihydrobenzofurannyle, 2,3-dihydro-2-oxo-1H-benzimidazolyle, 2,3-dihydro-2-oxo-benzoxazolyle, 1,2-dihydro-2-oxoquinoléinyle, 1,4-dihydro-4-oxoquinoléinyle, 1,2-dihydro-1-oxoisoquinoléinyle, 1,4-dihydro-4-oxocinnolinyle, 1,2-dihydro-2-oxoquinazolinyle, 3,4-dihydro-4-oxoquinazolinyle, 1,2,3,4-tétrahydro-2,4-dioxoquinazolinyle, 1,2-dihydro-2-oxoquinoxalinyle, 1,2,3,4-tétrahydro-2,3-dioxoquinoxalinyle, 1,2-dihydro-1-oxophtalazinyle, 1,2,3,4-tétrahydro-1,4-dioxophtalazinyle, chromanyle, coumarinyle, 2,3-dihydrobenzo-[1,4]dioxinyle ou 3,4-dihydro-3-oxo-2H-benzo[1,4]oxazinyle,
où les groupes hétéroaryle indiqués ci-dessus peuvent être substitués par R¹⁰ à R¹⁴, où R¹⁰ à R¹⁴ sont définis comme indiqué ci-dessus,
où sauf indication contraire, les groupes alkyle, alcényle et alcynyle indiqués ci-dessus peuvent être linéaires ou ramifiés,
ainsi que les dérivés N-oxydés ou méthylés ou éthylés sur l'atome d'azote cyclique en position 3 ou 9 du squelette hypoxanthine,
ainsi que les dérivés, dans lesquels le groupe 6-oxo du squelette hypoxanthine est remplacé par un groupe thioxo,
avec la condition que les composés
8-(pipéridin-4-ylméthyl)-7-(4-fluorobenzyl)-1,7-dihydropurin-6-one,
8-(1-méthylpipéridin-4-ylméthyl)-7-(4-fluorobenzyl)-1,7-dihydropurin-6-one,
8-(pipéridin-4-ylamino)-7-(4-fluorobenzyl)-1,7-dihydropurin-6-one, et
8-(1-méthylpipéridin-4-ylamino)-7-(4-fluorobenzyl)-1,7-dihydropurin-6-one, sont exclus,
leurs tautomères, énantiomères, diastéréoisomères, leurs mélanges et leurs sels.

2. Dérivés de purine de la formule générale I selon la revendication 1,
dans laquelle R¹, R² et R³ sont définis comme indiqué à la revendication 1, et
R⁴ représente un groupe pyrrolidin-1-yle, qui est substitué en position 3 par un groupe amino,
un groupe pipéridin-1-yle, qui est substitué en position 3 par un groupe amino,
un groupe pipéridin-3-yle ou pipéridin-4-yle,
un groupe hexahydroazépin-1-yle, qui est substitué en position 3 ou 4 par un groupe amino,
un groupe pipérazin-1-yle ou [1,4]diazépan-1-yle,
un groupe (2-aminocyclohexyl)amino,
un groupe cyclohexyle, qui est substitué en position 3 par un groupe amino, ou
un groupe N-(2-aminoéthyl)méthylamino ou N-(2-aminoéthyl)éthylamino,
leurs tautomères, énantiomères, diastéréoisomères, leurs mélanges et leurs sels.

3. Dérivés de purine de la formule générale I selon la revendication 1, dans laquelle
R¹ représente un atome d'hydrogène,
un groupe alkyle en C₁₋₆,
un groupe alcényle en C₃₋₆,
un groupe alcynyle en C₃₋₄,
un groupe (cycloalkyle en C₃₋₆)méthyle,
un groupe phényl-(alkyle en C₁₋₃), dans lequel la partie phényle est substituée par R¹⁰ et R¹¹, où
R¹⁰ représente un atome d'hydrogène, un atome de fluor, de chlore ou de brome, un groupe méthyle ou trifluorométhyle,
un groupe cyano, aminocarbonyle, diméthylaminocarbonyle ou méthylsulfonyle,
un groupe amino, acétylamino ou méthylsulfonylamino,
un groupe hydroxy, méthoxy, difluorométhoxy, trifluorométhoxy, carboxyméthoxy, méthoxycarbonylméthoxy, éthoxycarbonylméthoxy, aminocarbonylméthoxy, méthylaminocarbonylméthoxy, éthylaminocarbonylméthoxy ou diméthylaminocarbonylméthoxy, et
R¹¹ représente un atome d'hydrogène, un atome de fluor ou de chlore, ou
un groupe méthyle ou méthoxy,
un groupe naphtylméthyle, dans lequel la partie naphtyle est substituée par R¹⁰ et R¹¹, où R¹⁰ et R¹¹ sont définis comme indiqué ci-dessus,
un groupe hétéroarylméthyle, où par le terme
hétéroaryle, on entend un groupe furannyle, thiényle, oxazolyle, isoxazolyle, thiazolyle, pyridyle, pyrimidinyle, pyrazinyle, quinoléinyle, isoquinoléinyle ou quinazolinyle et les groupes hétéroaryle indiqués ci-dessus sont substitués par R¹⁰ et R¹¹, où R¹⁰ et R¹¹ sont définis comme indiqué ci-dessus,
un groupe phénylcarbonylméthyle, dans lequel la partie phényle est substituée par R¹⁰ et R¹¹, où R¹⁰ et R¹¹ sont définis comme indiqué ci-dessus,
un groupe furanylcarbonylméthyle, thiénylcarbonylméthyle ou pyridylcarbonylméthyle,
ou un groupe 2-oxopropyle ou cyclohexylcarbonylméthyle,
R² représente un atome d'hydrogène,
un groupe alkyle en C₁₋₆,
un groupe alcényle en C₃₋₆,
un groupe alcynyle en C₃₋₄,
un groupe cycloalkyle en C₃₋₆ ou (cycloalkyle en C₃₋₆)alkyle en C₁₋₃,
un groupe phényle, qui est substitué par R¹⁰ et R¹¹, où R¹⁰ et R¹¹ sont définis comme indiqué ci-dessus,
un groupe phényl-(alkyle en C₁₋₃), dans lequel la partie phényle est substituée par R¹⁰ et R¹¹, où R¹⁰ et R¹¹ sont définis comme indiqué ci-dessus,
un groupe phényl-(alcényle en C₂₋₃), dans lequel la partie phényle est substituée par R¹⁰ et R¹¹, où R¹⁰ et R¹¹ sont définis comme indiqué ci-dessus,
un groupe phénylcarbonylméthyle, dans lequel la partie phényle est substituée par R¹⁰ et R¹¹, où R¹⁰ et R¹¹ sont définis comme indiqué ci-dessus,
un groupe furannyle, thiényle ou pyridyle,
un groupe furannyl-(alkyle en C₁₋₃), thiényl-(alkyle en C₁₋₃), ou pyridyl-(alkyle en C₁₋₃),
un groupe cyano,
un groupe amino, (alkyle en C₁₋₄)amino ou di(alkyle en C₁₋₄)amino,
un groupe amino substitué par les radicaux R¹⁵ et R¹⁶, dans lequel
R¹⁵ représente un atome d'hydrogène ou un groupe méthyle ou éthyle, et
R¹⁶ représente un groupe alkyle en C₁₋₄, qui est substitué par un groupe cyano, carboxy, méthoxycarbonyle, éthyloxycarbonyle, aminocarbonyle, méthylaminocarbonyle, diméthylaminocarbonyle, éthylaminocarbonyle, diéthylaminocarbonyle, pyrrolidin-1-ylcarbonyle ou morpholin-4-ylcarbonyle,
un groupe amino substitué par les radicaux R¹⁵ et R¹⁷, dans lequel
R¹⁵ est défini comme indiqué ci-dessus, et
R¹⁷ représente un groupe alkyle en C₂₋₄ linéaire, qui est substitué chaque fois à son extrémité, par un groupe amino, méthylamino, diméthylamino, acétylamino, éthyloxycarbonylamino, phénylcarbonylamino, méthylsulfonylamino, phénylsulfonylamino, hydroxy, méthoxy, phényloxy, méthylsulfanyle ou phénylsulfanyle,
un groupe pyrrolidin-1-yle, pipéridin-1-yle, morpholin-4-yle, pipérazin-1-yle ou 4-méthylpipérazin-1-yle,
un groupe (cycloalkyle en C₃₋₆)amino ou (cycloalkyle en C₃₋₆)(alkyle en C₁₋₃)amino,
un groupe phénylamino,
un groupe phényl(alkyle en C₁₋₃)amino, dans lequel la partie phényle est substituée par R¹⁰ et R¹¹, où R¹⁰ et R¹¹ sont définis comme indiqué ci-dessus,
un groupe naphtylméthylamino,
un groupe (hétéroaryl)(alkyle en C₁₋₂)amino, où le terme hétéroaryle est défini comme indiqué ci-dessus, ou
un groupe méthylsulfanyle, benzylsulfanyle ou (2-phényléthyl)sulfanyle,
R³ représente un groupe alcényle en C₄₋₆,
un groupe alcényle en C₃₋₄, substitué par un atome de fluor, de chlore ou de brome ou un groupe trifluorométhyle,
un groupe 2-butyn-1-yle, ou
un groupe méthyle substitué par le groupe R_{c}, où
R_{c} représente un groupe 1-cyclopentèn-1-yle ou 1-cyclohexèn-1-yle,
un groupe phényle le cas échéant substitué par un atome de fluor, de chlore, de brome ou d'iode, par un groupe méthyle, trifluorométhyle, cyano, méthoxy, difluorométhoxy ou trifluorométhoxy,
un groupe phényle, qui est substitué par deux atomes de fluor,
un groupe naphtyle, ou
un groupe furanyle, thiényle ou pyridyle,
et
R⁴ représente un groupe pipéridin-1-yle, qui est substitué en position 3 par un groupe amino,
un groupe hexahydroazépin-1-yle, qui est substitué en position 3 ou 4 par un groupe amino,
un groupe (2-aminocyclohexyl)amino,
un groupe cyclohexyle, qui est substitué en position 3 par un groupe amino, ou
un groupe N-(2-aminoéthyl)méthylamino ou N-(2-aminoéthyl)éthylamino,
où sauf indication contraire, les groupes alkyle, alcényle et alcynyle mentionnés précédemment peuvent être linéaires ou ramifiés,
leurs tautomères, énantiomères, diastéréoisomères, leurs mélanges et leurs sels.

4. Dérivés de purine de la formule générale I selon la revendication 3, dans laquelle
R¹ représente un atome d'hydrogène,
un groupe méthyle, benzyle ou 2-phényléthyle,
un groupe naphtylméthyle ou méthoxynaphtylméthyle, ou
un groupe phénylcarbonylméthyle,
R² représente un atome d'hydrogène,
un groupe méthyle ou 2-phényléthyle,
un groupe phénylcarbonylméthyle,
un groupe cyano,
un groupe amino, méthylamino, diméthylamino, isopropylamino, cyclohexylamino, ou (cyclohexylméthyl)amino,
un groupe benzylamino, fluorobenzylamino ou (2-phényléthyl)amino, ou
un groupe pipéridin-1-yle,
R³ représente un groupe benzyle ou 3-méthylbut-2-èn-1-yle,
et
R⁴ représente un groupe 3-aminopipéridin-1-yle,
leurs tautomères, énantiomères, diastéréoisomères, leurs mélanges et leurs sels.

5. Composés suivants de la formule générale I selon la revendication 1 :
(1) 8-(3-aminopipéridin-1-yl)-7-benzyl-2-benzylamino-1-méthyl-1,7-dihydropurin-6-one,
(2) 8-(3-aminopipéridin-1-yl)-7-benzyl-2-(4-fluorobenzylamino)-1-méthyl-1,7-dihydropurin-6-one,
(3) 8-(3-aminopipéridin-1-yl)-7-benzyl-1-méthyl-2[(2-phényléthyl)amino]-1,7-dihydropurin-6-one,
(4) 8-(3-aminopipéridin-1-yl)-7-benzyl-2-isopropylamino-1-méthyl-1,7-dihydro-purin-6-one,
(5) 8-(3-aminopipéridin-1-yl)-7-benzyl-1-méthyl-2-méthylamino-1,7-dihydropurin-6-one,
(6) 8-(3-aminopipéridin-1-yl)-7-benzyl-2-cyclohexylamino-1-méthyl-1,7-dihydro-purin-6-one,
(7) 8-(3-aminopipéridin-1-yl)-7-benzyl-2-[(cyclohexylméthyl)amino]-1-méthyl-1,7-dihydropurin-6-one,
(8) 8-(3-aminopipéridin-1-yl)-7-benzyl-1-méthyl-2(pipéridin-1-yl)-1,7-dihydro-purin-6-one,
(9) 8-(3-aminopipéridin-1-yl)-7-benzyl-2-diméthylamino-1-méthyl-1,7-dihydro-purin-6-one,
(10) 2-amino-8-(3-aminopipéridin-1-yl)-7-benzyl-1-méthyl-1,7-dihydropurin-6-one,
(11) 8-(3-aminopipéridin-1-yl)-2-benzylamino-1-méthyl-7-(3-méthylbut-2-èn-1-yl)-1,7-dihydropurin-6-one,
(12) 8-(3-aminopipéridin-1-yl)-7-benzyl-1-méthyl-2-méthyl-1,7-dihydropurin-6-one,
(13) 8-(3-aminopipéridin-1-yl)-1-méthyl-7-(3-méthylbut-2-èn-1-yl)-2-(2-phényléthyl)-1,7-dihydropurin-6-one,
(14) 8-(3-aminopipéridin-1-yl)-7-benzyl-1-méthyl-1,7-dihydropurin-6-one,
(15) 8-(3-aminopipéridin-1-yl)-7-benzyl-1-(2-oxo-2-phényléthyl)-1,7-dihydropurin-6-one,
(16) 8-(3-aminopipéridin-1-yl)-2-méthyl-7-(3-méthylbut-2-èn-1-yl)-1-[(naphtalèn-1-yl)méthyl]-1,7-dihydropurin-6-one,
(17) 8-(3-aminopipéridin-1-yl)-7-(3-méthylbut-2-èn-1-yl)-1-[(naphtalèn-1-yl) méthyl]-1,7-dihydropurin-6-one,
(18) 8-(3-aminopipéridin-1-yl)-7-(3-méthylbut-2-èn-1-yl)-1-[(4-méthoxynaphtalèn-1-yl)méthyl]-1,7-dihydropurin-6-one,
ainsi que leurs tautomères, énantiomères, diastéréoisomères, leurs mélanges et leurs sels.

6. Sels physiologiquement compatibles des composés selon au moins une des revendications 1 à 5 avec des acides ou bases inorganiques ou organiques.

7. Médicament, contenant un composé selon au moins une des revendications 1 à 5 ou un sel physiologiquement compatible selon la revendication 6, avec le cas échéant, un ou plusieurs supports et/ou agents de dilution inertes.

8. Utilisation d'un composé selon au moins une des revendications 1 à 6, pour la préparation d'un médicament adapté au traitement du diabète de type I et de type II, l'arthrite, d'adipose, la transplantation d'allogreffe et l'ostéoporose engendrée par la calcitonine.

9. Procédé de préparation d'un médicament selon la revendication 7, **caractérisé en ce que** l'on incorpore par une voie non chimique, un composé selon au moins une des revendications 1 à 6 dans un ou plusieurs supports et/ou agents de dilution inertes.

10. Procédé de préparation de composés de la formule générale I selon les revendications 1 à 6, **caractérisé en ce que**
a) pour la préparation de composés de la formule générale I, dans laquelle R⁴ représente un des groupes indiqués à la revendication 1, qui contiennent un groupe imino, amino, alkylamino ou dialkylamino,
on déprotège un composé de la formule générale : 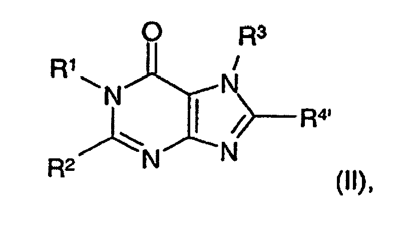
dans laquelle R¹, R² et R³ sont définis comme indiqué à la revendication 1 et
R^{4'} représente un des groupes indiqués pour R⁴ à la revendication 1, qui contiennent un groupe imino, amino ou alkylamino, où le groupe imino, amino et respectivement, alkylamino est substitué par un groupe protecteur, comme par exemple t-butyloxycarbonyle, 2,2,2-trichloroéthoxycarbonyle ou carbobenzyloxycarbonyle, et ensuite, on alkyle le cas échéant,
et si souhaité, ensuite, on clive un groupe protecteur utilisé pendant les réactions pour la protection de groupes réactifs, et/ou
un composé ainsi obtenu de la formule générale I est séparé de ses stéréoisomères et/ou
un composé ainsi obtenu de la formule générale I est transformé en ses sels, en particulier pour l'utilisation pharmaceutique, en ses sels physiologiquement compatibles avec un acide inorganique ou organique.
